# EUROPÄISCHE PATENTSCHRIFT

(11) **EP 1 144 685 B1**
(45) Veröffentlichungstag und Bekanntmachung des Hinweises auf die Patenterteilung: **23.04.2003**
(21) Anmeldenummer: 00904884.4
(22) Anmeldetag: 07.01.2000
(51) Int. Cl.: C12Q 1/68, C07H 21/00

(54) **VERFAHREN ZUR ELEKTROCHEMISCHEN DETEKTION VON NUKLEINSÄURE -OLIGOMER- HYBRIDISIERUNGSEREIGNISSEN**
METHOD FOR ELECTROCHEMICALLY DETECTING NUCLEIC ACID-OLIGOMER HYBRIDISATION EVENTS
PROCEDE DE DETECTION ELECTROCHIMIQUE D'HYBRIDATION D'ACIDE NUCLEIQUE-OLIGOMERE

(30) Priorität: 18.01.1999 DE 19901761; 29.04.1999 DE 19926457
(43) Veröffentlichungstag der Anmeldung: 17.10.2001
(73) Patentinhaber: Friz Biochem GmbH, 80639 München (DE)
(72) Erfinder: HARTWICH, Gerhard, D-80639 München (DE)
(74) Vertreter: Kritzenberger, Jürgen Hermann
(86) Internationale Anmeldenummer: EP0000084
(87) Internationale Veröffentlichungsnummer: WO00042217

(56) Entgegenhaltungen:
- EP-A- 0 831 327
- WO-A-00/31101
- WO-A-98/20162
- WO-A-98/31839
- WO-A-99/51778
- DE-A- 4 216 696
- US-A- 5 312 527
- US-A- 5 622 946
- US-A- 5 770 369
- KATZ E ET AL: "ELECTROCHEMICAL STUDY OF PYRROLOQUINOLINE QUINONE COVALENTLY IMMOBILIZED AS A MONOLAYER ONTO A CYSTAMINE-MODIFIED GOLD ELECTRODE" JOURNAL OF ELECTROANALYTICAL CHEMISTRY AND INTERFACIAL ELECTROCHEMISTRY,NL,ELSEVIER, AMSTERDAM, Bd. 367, 1994, Seiten 59-70, XP000672483 ISSN: 0022-0728
- SCHRöTER ET AL.: "Taschenbuch der Chemie", 1991, FACHBUCHVERLAG, LEIPZIG

## Beschreibung

### Technisches Gebiet

Die vorliegende Erfindung betrifft ein modifiziertes Nukleinsäure-Oligomer, sowie ein Verfahren zur elektrochemischen Detektion von sequenzspezifischen Nukleinsäure-Oligomer-Hybridisierungsereignissen.

### Stand der Technik

Zur Sequenzanalyse von DNA und RNA, z. B. in der Krankheitsdiagnose, bei toxikologischen Testverfahren, in der genetischen Forschung und Entwicklung, sowie auf dem Agrar- und pharmazeutischen Sektor, werden im allgemeinen gel-elektrophoretische Verfahren mit autoradiographischer oder optischer Detektion verwendet.

Beim wichtigsten gel-elektrophoretischen Verfahren mit optischer Detektion, dem Sanger-Verfahren wird eine DNA enthaltende Lösung in vier Ansätze aufgeteilt. Zur Unterscheidung der vier Ansätze ist der Primer (komplementäre Startsequenz zur Replikation) jedes Ansatzes mit je einem bei verschiedener Wellenlänge emitierenden Fluoreszenzfarbstoff kovalent modifiziert. Ausgehend vom Primer wird jeder Ansatz durch DNA-Polymerase I enzymatisch repliziert. Neben den dazu nötigen Desoxyribonucleosid-Triphosphaten der Basen A (Adenin), T (Thymin), C (Cytosin), und G (Guanin) enthält jedes Reaktionsgemisch noch genügend 2',3'-Didesoxyanalogon eines dieser Nukleosidtriphosphate als Stopbase (je eine der 4 möglichen Stopbasen pro Ansatz), um die Replikation an allen möglichen Bindungsstellen zu stoppen. Nach Vereinigung der vier Ansätze entstehen replizierte DNA-Fragmente aller Längen mit stopbasenspezifischer Fluoreszenz, die gel-elektrophoretisch der Länge nach sortiert und durch Fluoreszenz-Spektroskopie charakterisiert werden können.

Ein anderes optisches Detektionsverfahren basiert auf der Anlagerung von Fluoreszenzfarbstoffen wie z. B. Ethidiumbromid an Oligonukleotide. Im Vergleich zur freien Lösung des Farbstoffs ändert sich die Fluoreszenz solcher Farbstoffe bei Assoziation mit doppelsträngiger DNA oder RNA drastisch und kann deshalb zum Nachweis hybridisierter DNA oder RNA verwendet werden.

Bei der radioaktiven Markierung wird ³²P in das Phosphatgerüst der Oligonukleotide eingebaut, wobei ³²P gewöhnlich am 5'-Hydroxylende durch Polynukleotid-Kinase addiert wird. Die markierte DNA wird anschließend an jeweils einem der vier Nukleotidtypen bevorzugt gespalten und zwar unter definierten Bedingungen, so daß pro Kette durchschnittlich eine Spaltung erfolgt. Damit liegen im Reaktionsgemisch für einen bestimmten Basentyp Ketten vor, die sich von der ³²P-Markierung bis zur Position dieser Base erstrecken (bei mehrfachem Auftreten der Base erhält man entsprechend Ketten unterschiedlicher Länge). Die vier Fragmentgemische werden anschließend auf vier Bahnen gel-elektrophoretisch aufgetrennt. Danach wird vom Gel ein Autoradiogramm angefertigt, an dem die Sequenz unmittelbar abgelesen werden kann.

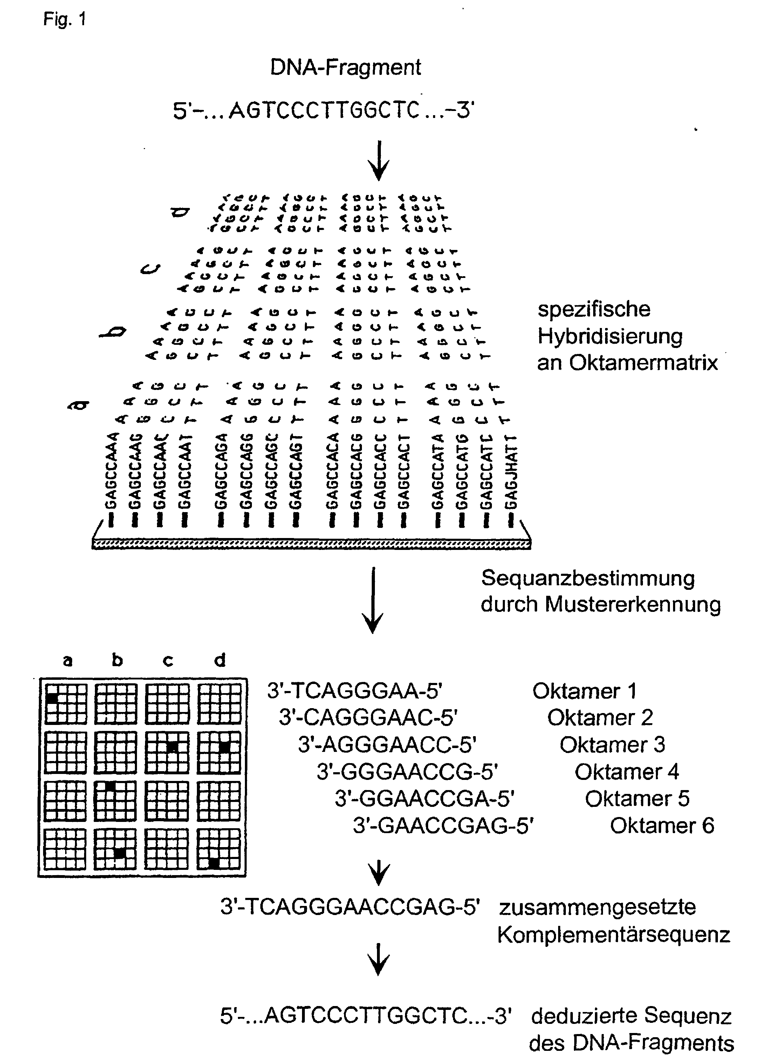

Vor einigen Jahren wurde ein weiteres, auf optischer (oder autoradiographischer) Detektion beruhendes Verfahren zur DNA-Sequenzierung entwickelt, nämlich die Sequenzierung durch Oligomer-Hybridisierung (vgl. z. B. Drmanac et al., Genomics 4, (1989), S. 114-128 oder Bains et al., Theor. Biol. 135, (1988), S. 303-307). Bei diesem Verfahren wird ein vollständiger Satz kurzer Oligonukleotide bzw. Nukleinsäure-Oligomere (Sonden-Oligonukleotide), z. B. alle 65536 möglichen Kombinationen der Basen A, T, C und G eines Oligonukleotid-Oktamers auf ein Trägermaterial gebunden. Die Anbindung geschieht in einem geordneten Raster aus 65536 Test-Sites, wobei jeweils eine größere Menge einer Oligonukleotid-Kombination ein Test-Site definieren und die Position jeder einzelnen Test-Site (Oligonukleotid-Kombination) bekannt ist. Auf solch einer Hybridisierungsmatrix, dem Oligomer-Chip, wird ein DNA-Fragment, dessen Sequenz man ermitteln will (das Target), mit Fluoreszenzfarbstoff (oder ³²P) markiert und unter Bedingungen, die nur eine spezifische Doppelstrangbildung erlauben, hybridisiert. Dadurch bindet das Target DNA-Fragment nur an die Nukleinsäure-Oligomere (im Beispiel an die Oktamere), deren komplementäre Sequenz exakt einem Teil (einem Oktamer) seiner eigenen Sequenz entspricht. Durch optische (oder autoradiographische) Detektion der Bindungsposition des hybridisierten DNA-Fragments werden damit alle im Fragment vorhandenen Nukleinsäure-Oligomersequenzen (Oktamersequenzen) bestimmt. Aufgrund der Überlappung benachbarter Nukleinsäure-Oligomersequenzen kann durch geeignete mathematische Algorithmen die fortlaufende Sequenz des DNA-Fragments bestimmt werden. Die Vorteile dieses Verfahrens liegen unter anderem in der Miniaturisierung der Sequenzierung und damit in der enormen Datenmenge, die gleichzeitig in einem Arbeitsgang erfaßt wird.
Daneben kann auf Primer und auf das gel-elektrophoretische Auftrennen der DNA-Fragmente verzichtet werden. Beispielhaft ist dieses Prinzip in Figur 1 für ein 13 Basen langes DNA-Fragment gezeigt.

Die Verwendung radioaktiver Markierungen bei der DNA-/RNA-Sequenzierung ist mit mehreren Nachteilen verbunden, wie z. B. aufwendige, gesetzlich vorgeschriebene Sicherheitsvorkehrungen beim Umgang mit radioaktiven Materialien, die Strahlenbelastung, das begrenzte räumliche Auflösungsvermögen (maximal 1mm²) und eine Sensitivität, die nur dann hoch ist, wenn die Strahlung der radioaktiven Fragmente entsprechend lange (Stunden bis Tage) auf einen Röntgenfilm einwirkt. Es kann zwar die räumliche Auflösung durch zusätzliche Hard- und Software erhöht und die Detektionszeit durch die Verwendung von β-Scannern verkürzt werden, beides ist jedoch mit erheblichen zusätzlichen Kosten verbunden.

Die Fluoreszenzfarbstoffe, die üblicherweise zur Markierung der DNA verwendet werden, sind zum Teil (z. B. Ethidiumbromid) mutagen und erfordern, ebenso wie die Anwendung der Autoradiographie, entsprechende Sicherheitsvorkehrungen. In fast allen Fällen erfordert die Verwendung optischer Detektion den Gebrauch von einem oder mehreren Lasersystemen und somit geschultes Personal und entsprechende Sicherheitsvorkehrungen. Die eigentliche Detektion der Fluoreszenz erfordert zusätzliche Hardware, wie z. B. optische Bauelemente zur Verstärkung und, bei verschiedenen Anregungs- und Abfragewellenlängen wie im Sanger-Verfahren, ein Kontrollsystem. Abhängig von den benötigten Anregungswellenlängen und der gewünschten Detektionsleistung können somit erhebliche Investitionskosten entstehen. Bei der Sequenzierung durch Hybridisierung auf dem Oligomer-Chip ist die Detektion noch (kosten)aufwendiger, da, neben dem Anregungssystem, zur 2-dimensionalen Detektion der Fluoreszenzspots hochauflösende CCD-Kameras (Charge Coupled Device Kameras) benötigt werden.

Die US-A-5 770 369 offenbart Nukleinsäure-Oligomere, die durch kovalente Anbindung von redoxaktiven Verbindungen modifiziert sind. Diese Nukleinsäure-Oligomere werden an eine Elektrode gebunden, wodurch ein Elektronentransport zwischen der Elektrode und den Nukleinsäure-Oligomeren erfolgen kann. Das Vorliegen einer doppelsträngigen DNA kann durch Vergleich der Elektronentransferraten von unhybridisierter Probe und hybridisierter Probe erfolgen. Zur Detektion des Elektronentransfers wird unter anderem die Amperometrie vorgeschlagen.

Die WO-A-98 20162 offenbart Nukleinsäuren, die durch kovalente Anbindung von redoxaktiven Verbindungen modifiziert sind. Diese modifizierten Nukleinsäuren werden über ein leitfähiges Polymer an eine Elektrode gebunden, wodurch ein Elektronentransport zwischen der Elektrode und der Nukleinsäure erfolgen kann. Das Vorliegen einer doppelsträngigen DNA kann durch erhöhten Elektronentransfer von der DNA auf die Elektrode detektiert werden. Zur Detektion des Elektronentransfers wird unter anderem die Amperometrie vorgeschlagen.

Aus der DE-A-42 16 696 ist ein Verfahren zur Durchführung von Assays bekannt, die unter anderem auf Wechselwirkungen zwischen DNA-Strängen beruhen. Die Detektion basiert zum Beispiel auf einer Messung der Stromstärke, wobei stabile redox-markierte Analytmoleküle Verwendung finden. Dazu wird ein DNA-Strang mit einem stabilen Redox-System kovalent verbunden, wobei als Redox-Systeme Ferrocenderivate, Rutheniumkomplexe, Hydrochinone, Hexacyanoferrat (II/III), Jod/Jodid oder ähnliche vorgeschlagen werden.

In der nachveröffentlichten WO-A-00 31101 wird ein Verfahren zur elektrochemischen Detektion von sequenzspezifischen Nukleinsäure-Oligomer-Hybridisierungsereignissen beschrieben. Dabei werden durch Anbindung einer redoxaktiven Substanz modifizierte Oligonukleotid-Einzelstränge an eine Trägeroberfläche gebunden. Durch Behandlung mit der zu untersuchenden Oligonukleotid-Lösung wird im Falle der Hybridisierung mit dem komplementären Oligonukleotidstrang die elektrische Kommunikation zwischen der leitfähigen Trägeroberfläche und der redoxaktiven Substanz verstärkt. Die Detektion eines Hybridisierungsereignisses erfolgt durch elektrochemische Verfahren wie cyclische Voltametrie, Amperometrie oder Leitfähigkeitsmessung.

Obwohl es also quantitative und extrem sensitive Methoden zur DNA-/RNA-Sequenzierung gibt, sind diese Methoden zeitaufwendig, bedingen aufwendige Probenpräparation und teure Ausstattung und sind im allgemeinen nicht als transportable Systeme verfügbar.

### Darstellung der Erfindung

Aufgabe der vorliegenden Erfindung ist es deshalb, eine Vorrichtung und ein Verfahren zur Detektion von Nukleinsäure-Oligomer-Hybriden zu schaffen, welche die Nachteile des Standes der Technik nicht ausweisen.

Diese Aufgabe wird erfindungsgemäß durch das modifizierte Nukleinsäure-Oligomer gemäß unabhängigem Patentanspruch 1, durch das Verfahren zur Herstellung eines modifizierten Nukleinsäure-Oligomers gemäß unabhängigem Anspruch 21, durch die modifizierte leitfähige Oberfläche gemäß unabhängigem Patentanspruch 29, das Verfahren zur Herstellung einer modifizierten leitfähigen Oberfläche gemäß unabhängigem Patentanspruch 43 und ein Verfahren zur elektrochemischen Detektion von Nukleinsäure-Oligomer-Hybridisierungsereignissen gemäß unabhängigen Patentanspruch 48 gelöst.

Im Rahmen der vorliegenden Erfindung werden die folgenden Abkürzungen und Begriffe benutzt:

| ***Genetik*** | |
|---|---|
| DNA | Desoxyribonukleinsäure |
| RNA | Ribonukleinsäure |
| PNA | Peptidnukleinsäure (synthetische DNA oder RNA, bei der die Zucker-Phosphat Einheit durch eine Aminosäure ersetzt ist. Bei Ersatz der Zucker-Phosphat Einheit durch die -NH-(CH₂)₂-N(COCH₂-Base)-CH₂CO- Einheit hybridisiert PNA mit DNA.) |
| A | Adenin |
| G | Guanin |
| C | Cytosin |
| T | Thymin |
| U | Uracil |
| Base | A, G, T, C oder U |
| Bp | Basenpaar |
| Nukleinsäure | wenigstens zwei kovalent verbundene Nukleotide oder wenigstens zwei kovalent verbundene Pyrimidin- (z. B. Cytosin, Thymin oder Uracil) oder Purin-Basen (z. B. Adenin oder Guanin). Der Begriff Nukleinsäure bezieht sich auf ein beliebiges "Rückgrat" der kovalent verbundenen Pyrimidin-oder Purin-Basen, wie z. B. auf das Zucker-Phosphat Rückgrat der DNA, cDNA oder RNA, auf ein Peptid-Rückgrat der PNA oder auf analoge Strukturen (z. B. Phosphoramid-, Thio-Phosphat- oder Dithio-Phosphat-Rückgrat). |
| | Wesentliches Merkmal einer Nukleinsäure im Sinne der vorliegenden Erfindung ist, daß sie natürlich vorkommende cDNA oder RNA sequenzspezifisch binden kann. |
| Nukleinsäure-Oligomer | Nukleinsäure nicht näher spezifizierter Basenlänge (z. B. Nukleinsäure-Oktamer: eine Nukleinsäure mit beliebigem Rückgrat, bei dem 8 Pyrimidin- oder Purin-Basen kovalent aneinander gebunden sind). |
| Oligomer | Äquivalent zu Nukleinsäure-Oligomer. |
| Oligonukleotid | Äquivalent zu Oligomer oder Nukleinsäure-Oligomer, also z. B. ein DNA, PNA oder RNA Fragment nicht näher spezifizierter Basenlänge. |
| Oligo | Abkürzung für Oligonukleotid. |
| Primer | Start-Komplementär-Fragment eines Oligonukleotids, wobei die Basenlänge des Primers nur ca. 4-8 Basen beträgt. Dient als Ansatzpunkt für die enzymatische Replikation des Oligonukleotids. |
| Mismatch | Zur Ausbildung der Watson Crick Struktur doppelsträngiger Oligonukleotide hybridisieren die beiden Einzelstränge derart, daß die Base A (bzw. C) des einen Strangs mit der Base T (bzw. G) des anderen Strangs Wasserstoffbrücken ausbildet (bei RNA ist T durch Uracil ersetzt). Jede andere Basenpaarung bildet keine Wasserstoffbrücken aus, verzerrt die Struktur und wird als "Mismatch" bezeichnet. |
| ss | single strand (Einzelstrang) |
| ds | double strand (Doppelstrang) |

| ***Photoinduzierbar und chemisch induzierbar redoxaktive Einheiten*** | |
|---|---|
| redoxaktive Einheit | photoinduzierbar redoxaktive Einheit oder chemisch induzierbar redoxaktive Einheit |
| Elektron-Donor | Der Begriff Elektron-Donor bezeichnet im Rahmen der vorliegenden Erfindung einen Bestandteil einer photoinduzierbar redoxaktiven Einheit bzw. einer chemisch induzierbar redoxaktiven Einheit. Bei einem Elektron-Donor handelt es sich um ein Molekül, das unmittelbar oder nach Einwirkung bestimmter äußerer Umstände ein Elektron an einen Elektron-Akzeptor transferieren kann. Ein solcher äußerer Umstand ist z. B. die Lichtabsorption durch den Elektron-Donor oder -Akzeptor einer photoinduzierbar redoxaktiven Einheit. Durch Einstrahlung von Licht bestimmter oder beliebiger Wellenlänge gibt der Elektron-Donor "D" an den/einen Elektron-Akzeptor "A" ein Elektron ab und es bildet sich, zumindest temporär, ein ladungsgetrennter Zustand D⁺A⁻ aus oxidiertem Donor und reduziertem Akzeptor. Ein weiterer solcher äußerer Umstand kann z. B. die Oxidation oder Reduktion des Elektron-Donors oder -Akzeptors der chemisch induzierbar redoxaktiven Einheit durch ein externes Oxidations- oder Reduktionsmittel sein, also z. B. die Übertragung eines Elektrons auf den Elektron-Donor durch ein Reduktionsmittel bzw. die Abgabe eines Elektrons durch den Elektron-Akzeptor an ein Oxidationsmittel sein. Diese Oxidations- bzw. Reduktionsmittel können sowohl externe redoxaktive Substanzen sein, d. h. sie sind nicht kovalent mit der redoxaktiven Einheit, dem Nukleinsäure-Oligomer oder der leitfähigen Oberfläche verbunden, stehen aber mit diesen, z. B. über die der modifizierten leitfähigen Oberfläche zugefügte Lösung, in Kontakt oder sie sind kovalent mit dem Nukleinsäure- Oligomer verbunden, wobei das Oxidations- bzw. Reduktionsmittel an einer Stelle des Nukleinsäure-Oligomers kovalent angebunden ist, die mindestens zwei kovalent verbundene Nukleotide oder wenigstens zwei kovalent verbundene Pyrimidin- oder Purin-Basen von der kovalenten Anbindungstelle der redoxaktiven Einheit entfernt ist, bevorzugt an dem der Modifikation mit redoxaktiver Einheit entgegengesetzten Ende des Oligonukleotids in der Nähe der leitfähigen Oberfläche. Die Fähigkeit als Elektron-Donor oder -Akzeptor zu wirken ist relativ, d. h. ein Molekül, das unmittelbar oder nach Einwirkung bestimmter äußerer Umstände gegenüber einem anderen Molekül als Elektron-Donor wirkt, kann gegenüber diesem Molekül unter abweichenden experimentellen Bedingungen oder gegenüber einem dritten Molekül unter gleichen oder abweichenden experimentellen Bedingungen auch als Elektron-Akzeptor wirken. |
| Elekron-Akzeptor | Der Begriff Elektron-Akzeptor bezeichnet im Rahmen der vorliegenden Erfindung einen Bestandteil einer photoinduzierbar redoxaktiven Einheit bzw. einer chemisch induzierbar |
| | redoxaktiven Einheit. Bei einem Elektron-Akzeptor handelt es sich um ein Molekül, das unmittelbar oder nach Einwirkung bestimmter äußerer Umstände ein Elektron von einem Elektron-Donor aufnehmen kann. Ein solcher äußerer Umstand ist z. B. die Lichtabsorption durch den Elektron-Donor oder -Akzeptor einer photoinduzierbar redoxaktiven Einheit. Durch Einstrahlung von Licht bestimmter oder beliebiger Wellenlänge gibt der Elektron-Donor "D" an den/einen der Elektron-Akzeptor "A" ein Elektron ab und es bildet sich, zumindest temporär, ein ladungsgetrennter Zustand D⁺A⁻ aus oxidiertem Donor und reduziertem Akzeptor. Ein weiterer solcher äußerer Umstand kann z. B. die Oxidation oder Reduktion des Elektron-Donors oder -Akzeptors der chemisch induzierbar redoxaktiven Einheit durch ein extemes Oxidations- oder Reduktionsmittel sein, also z. B. die Übertragung eines Elektrons auf den Elektron-Donor durch ein Reduktionsmittel bzw. die Abgabe eines Elektrons durch den Elektron-Akzeptor an ein Oxidationsmittel sein. Diese Oxidations- bzw. Reduktionsmittel können sowohl externe redoxaktive Substanzen sein, d. h. sie sind nicht kovalent mit der redoxaktiven Einheit, dem Nukleinsäure-Oligomer oder der leitfähigen Oberfläche verbunden, stehen aber mit diesen, z. B. über die der modifizierten leitfähigen Oberfläche zugefügte Lösung, in Kontakt oder sie sind kovalent mit dem Nukleinsäure- Oliqomer verbunden, wobei das Oxidations- bzw. Reduktionsmittel an einer Stelle des Nukleinsäure-Oligomers kovalent angebunden ist, die mindestens zwei kovalent verbundene Nukleotide oder wenigstens zwei kovalent verbundene Pyrimidin- oder Purin-Basen von der kovalenten Anbindungstelle der photoinduzierbar redoxaktiven Einheit entfernt ist, bevorzugt an dem der Modifikation mit redoxaktiver Einheit entgegengesetzten Ende des Oligonukleotids in der Nähe der leitfähigen Oberfläche. Die Fähigkeit als Elektron-Akzeptor oder -Donor zu wirken ist relativ, d. h. ein Molekül, das unmittelbar oder nach Einwirkung bestimmter äußerer Umstände gegenüber einem anderen Molekül als Elektron-Akzeptor wirkt, kann gegenüber diesem Molekül unter abweichenden experimentellen Bedingungen oder gegenüber einem dritten Molekül unter gleichen oder abweichenden experimentellen Bedingungen auch als Elektron-Donor wirken. |
| Elektron-Donor-Molekül | entspricht einem Elektron-Donor. |
| Elekron-Akzeptor-Molekül | entspricht einem Elektron-Akzeptor. |
| Oxidationsmittel | chemische Verbindung (chemische Substanz), die durch Aufnahme von Elektronen aus einer anderen chemischen Verbindung (chemischen Substanz, Elektron-Donor, Elektron-Akzeptor) diese andere chemische Verbindung (chemischen Substanz, Elektron-Donor, Elektron-Akzeptor) oxidiert. Ein Oxidationsmittel sich analog zu einem Elektron-Akzeptor, wird aber im Rahmen der vorliegenden Erfindung als Begriff für einen externen, nicht unmittelbar zur photoinduzierbar redoxaktiven Einheit bzw. chemisch induzierbar redoxaktiven Einheit gehörigen Elektron-Akzeptor verwendet. Nicht unmittelbar bedeutet in diesem Zusammenhang, daß das Oxidationsmittel entweder eine freie redoxaktive Substanz ist, die nicht an das Nukleinsäure-Oligomer gebunden ist, aber mit diesem in Kontakt steht oder daß das Oxidationsmittel kovalent an das Nukleinsäure-Oligomer angebunden ist, jedoch an einer Stelle des Nukleinsäure-Oligomers, die mindestens zwei kovalent verbundene Nukleotide oder wenigstens zwei kovalent verbundene Pyrimidin- oder Purin-Basen von der kovalenten Anbindungstelle der (photoinduzierbar) redoxaktiven Einheit entfernt ist. Insbesondere kann die Elektrode das Oxidationsmittel darstellen. |
| Reduktionsmittel | chemische Verbindung (chemische Substanz), die durch Abgabe von Elektronen an eine andere chemische Verbindung (chemische Substanz, Elektron-Donor, Elektron-Akzeptor) diese andere chemische Verbindung (chemischen Substanz, Elektron-Donor, Elektron-Akzeptor) reduziert. Ein Reduktionsmittel verhält sich analog zu einem Elektron-Donor, wird aber im Rahmen der vorliegenden Erfindung als Begriff für einen externen, nicht unmittelbar zur photoinduzierbar redoxaktiven Einheit bzw. zur chemisch induzierbar redoxaktiven Einheit gehörigen Elektron-Donor verwendet. Nicht unmittelbar bedeutet in diesem Zusammenhang, daß das Reduktionsmittel entweder eine freie redoxaktive Substanz ist, die nicht an das Nukleinsäure-Oligomer gebunden ist, aber mit diesem in Kontakt steht oder daß das |
| | Reduktionsmittel kovalent an das Nukleinsäure-Oligomer angebunden ist, jedoch an einer Stelle des Nukleinsäure-Oligomers, die mindestens zwei kovalent verbundene Nukleotide oder wenigstens zwei kovalent verbundene Pyrimidin- oder Purin-Basen von der kovalenten Anbindungstelle der edoxaktiven Einheit entfernt ist. Insbesondere kann die Elektrode das Reduktionsmittel darstellen. |
| photoinduzierbar | photoinduzierbar bedeutet, daß eine gewisse Eigenschaft erst durch Einstrahlen von Licht bestimmter oder beliebiger Wellenlänge entfaltet wird. So entfaltet z. B. eine photoinduzierbar redoxaktive Einheit ihre Redoxaktivität, also ihre Eigenschaft, unter bestimmten äußeren Umständen innerhalb der photoinduzierbar redoxaktiven Einheit eine Ladungstrennung durchzuführen, also z. B. den Zustand D⁺A⁻ auszubilden, und an ein anderes geeignetes Oxidationsmittel Elektronen abzugeben oder von einem anderen geeigneten Reduktionsmittel Elektronen aufzunehmen, erst durch Einstrahlen von Licht bestimmter oder beliebiger Wellenlänge. Ein weiteres Beispiel ist die photoinduzierbar reaktive Gruppe, d. h. eine Gruppe, die erst durch Einstrahlen von Licht bestimmter oder beliebiger Wellenlänge reaktiv wird. |
| redoxaktiv | redoxaktiv bezeichnet die Eigenschaft einer redoxaktiven Einheit unter bestimmten äußeren Umständen an ein geeignetes Oxidationsmittel Elektronen abzugeben oder von einem geeigneten Reduktionsmittel Elektronen aufzunehmen bzw. die Eigenschaft einer redoxaktiven Substanz unter bestimmten äußeren Umständen an einen geeigneten Elektron-Akzeptor Elektronen abzugeben oder von einem geeigneten Elektron-Donor Elektronen aufzunehmen. |
| freie, redoxaktive Substanz | freies, nicht kovalent mit der redoxaktiven Einheit dem Nukleinsäure-Oligomer oder der leitfähigen Oberfläche verbundes, aber mit diesen, z. B. über die der modifizierten leitfähigen Oberfläche zugefügte Lösung, in Kontakt stehendes Oxidations-oder Reduktionsmittel, wobei die freie redoxaktive Substanz z. B. ein ungeladenes Molekül, eine beliebiges Salz oder ein redoxaktives Protein oder Enzym (Oxydoreductase) sein kann. Die freie redoxaktive Substanz ist dadurch gekennzeichnet, daß sie den oxidierten Donor (bzw. den |
| | reduzierten Akzeptor) der photoinduzierbar redoxaktive Einheit re-reduzieren (bzw. re-oxidieren) kann bzw. daß die freie, redoxaktive Substanz den Donor (bzw. den Akzeptor) der chemisch induzierbar redoxaktiven Einheit reduzieren (bzw. oxidieren) kann. |
| photoinduzierbar redoxaktive Einheit | Oberbegriff für eine Einheit, die ein oder mehrere Elektron-Donor-Moleküle und ein oder mehrere Elektron-Akzeptor-Moleküle enthält, wobei dieses (diese) Elektron-Donor-Molekül(e) und/oder dieses (diese) Elektron-Akzeptor-Molekül(e) in ein oder mehrere Makromoleküle eingebettet sein können. Elektron-Donor(en) und Elektron-Akzeptor(en) können untereinander durch eine oder mehrere kovalente oder ionische Bindungen, durch Wasserstoff-Brücken-Bindungen, van-der-Waals-Brücken, durch π-π-Wechselwirkung oder durch Koordination mittels Elektronenpaar-Donation und -Akzeptation miteinander verbunden sein, wobei kovalente Verbindungen direkte oder indirekte (z. B. über einen Spacer, nicht aber über ein Nukleinsäure-Oligomer) Verbindungen sein können. Daneben können die Elektron-Donor(en) und/oder Elektron-Akzeptor(en), falls sie in ein oder mehrere Makromolekül(e) eingebettet sind, mit dem (den) Makromolekül(en) durch kovalente Anbindung an das (die) Makromolekül(e), durch Einkapseln in passende molekulare Kavitäten (Bindungstaschen) des Makromoleküls (der Makromoleküle), durch ionische Bindungen, Wasserstoff-Brücken-Bindungen, van-der-Waals-Brücken, π-π-Wechselwirkung oder durch Koordination mittels Elektronenpaar-Donation und -Akzeptation zwischen dem(n) Makromolekül(en) und dem(n) Elektron-Donor-Molekül(en) und/oder dem(n) Elektron-Akzeptor-Molekül(en) verbunden sein. Sind mehrere Makromoleküle Bestandteil der photoinduzierbar redoxaktiven Einheit kann die Bindung der Makromoleküle untereinander ebenfalls kovalent, ionisch, durch Wasserstoff-Brücken-Bindungen, van-der-Waals-Brücken, π-π-Wechselwirkung oder durch Koordination mittels Elektronenpaar-Donation und -Akzeptation erfolgen. Wesentliche Merkmale der photoinduzierbar redoxaktiven Einheit sind neben der Zusammensetzung aus Elektron-Donor(en) und Elektron- |
| | Akzeptor(en) oder aus Elektron-Donor(en) und Elektron-Akzeptor(en) und Makromolekül(en): (i) die Einheit ist in den erfindungsrelevanten Erscheinungsformen (Elektron-Donor(en) und Elektron-Akzeptor(en) im ursprünglichen bzw. oxidierten oder reduzierten Zustand) stabil und dissoziiert nicht in ihre Bestandteile, (ii) die Einheit enthält keine Nukleinsäure, (iii) die Zusammensetzung der Einheit aus Elektron-Donor(en) und Elektron-Akzeptor(en) oder aus Elektron-Donor(en) und Elektron-Akzeptor(en) und Makromolekül(en) kann - unabhängig von der Bindung zwischen den Bestandteilen - vom Fachmann erkannt werden, da sie prinzipiell auch als Einzelmoleküle vorkommen können und (iv) Elektron-Donor(en) und Elektron-Akzeptor(en) der photoinduzierbar redoxaktiven Einheit wirken unter gleichen oder ähnlichen äußeren Umständen in Form von Einzelmolekülen in Lösung als Elektron-Donor(en) und Elektron-Akzeptor(en), d. h. auch bei freien gelösten Elektron-Donor(en) und Elektron-Akzeptor(en) kann unmittelbar oder nach Einwirkung bestimmter äußerer Umstände, entsprechend den Umständen die innerhalb der photoinduzierbar redoxaktiven Einheit zu einem Elektrontransfer führen, ein Elektron vom (von den) gelösten Elektron-Donor(en) auf den (die) gelösten Elektron-Akzeptor(en) übertragen werden. Die photoinduzierbar redoxaktive Einheit kann z. B. jedes beliebige photoinduzierbar redoxaktive Protein/Enzym oder jeder beliebige photoinduzierbar redoxaktive, verknüpfte, wenigstens bimolekulare Elekton-Donor-/Elektron-Akzeptor-Komplex sein. Durch Einstrahlung von Licht bestimmter oder beliebiger Wellenlänge gibt der/ein Elektron-Donor an einen der Elektron-Akzeptoren ein Elektron ab und es bildet sich, zumindest temporär, ein ladungsgetrennter Zustand D⁺A⁻ aus einem oxidierten Donor und einem reduzierten Akzeptor. Dieser Vorgang innerhalb der photoinduzierbar redoxaktiven Einheit wird als photoinduzierte Ladungstrennung bezeichnet. Bei entsprechend gewählten äußeren Umständen entfaltet die photoinduzierbar redoxaktive Einheit ihre Redoxaktivität, also ihre Eigenschaft, an einen geeignetes Oxidationsmittel Elektronen abzugeben oder von einem geeigneten Reduktionsmittel Elektronen aufzunehmen, erst im |
| | ladungsgetrennten Zustand, da das Reduktionsmittel (bzw. Oxidationsmittel) nur auf den oxidierten Donor (bzw. vom reduzierten Akzeptor) der photoinduzierbar redoxaktiven Einheit Elektronen überträgt (bzw. aufnimmt), z. B. in Gegenwart eines Reduktionsmittels, das D⁺, jedoch nicht D, reduzieren kann (bzw. in Gegenwart eines Oxidationsmittels das A⁻, jedoch nicht A, oxidieren kann). Insbesondere kann dieses Oxidations- bzw. Reduktionsmittel auch eine Elektrode sein, wobei die photoinduzierbar redoxaktive Einheit erst nach der photoinduzierten Ladungstrennung ein Elektron an eine Elektrode abgeben (bzw. von dieser aufnehmen) kann, z. B. wenn die Elektrode auf ein Potential gesetzt wird, bei dem A⁻, jedoch nicht A, oxidiert (bzw. D⁺, jedoch nicht D, reduziert) wird. |
| chemisch induzierbar redoxaktive Einheit | entspricht in Zusammensetzung und Funktionsweise einer photoinduzierbar redoxaktive Einheit, wobei aber im Unterschied zur Funktionsweise einer photoinduzierbar redoxaktiven Einheit Photoaktivierung als äußerer Umstand zur Entfaltung der Redoxaktivität der redoxaktive Einheit ausgeschlossen ist. Die redoxaktive Einheit kann also z. B. jedes beliebige redoxaktive Protein/Enzym oder jeder beliebige redoxaktive, verknüpfte, wenigstens bimolekulare Elekton-Donor-/Elektron-Akzeptor-Komplex sein. Bei entsprechend gewählten äußeren Umständen entfaltet die redoxaktive Einheit ihre Redoxaktivität, also ihre Eigenschaft, z. B. an ein geeignetes Oxidationsmittel Elektronen abzugeben, erst nach Übertragung eines Elektrons von einem Reduktionsmittel auf den/einen Elektron-Donor "D", der nur im reduzierten Zustand "D⁻ " ein Elektron auf den Akzeptor "A" übertragen kann und das Oxidationsmittel nur von diesem reduzierten Akzeptor "A⁻" der redoxaktiven Einheit Elektronen aufnimmt, also in Gegenwart eines Oxidationsmittels, das A⁻, jedoch nicht A, oxidieren kann (sukzessive Ladungsübertragung). Insbesondere kann dieses Oxidationsmittel auch eine Elektrode sein, z. B. wenn die Elektrode auf ein Potential gesetzt wird, bei dem A⁻, jedoch nicht A, oxidiert wird. Umgekehrt kann bei abweichend gewählten äußeren Umständen die redoxaktive Einheit ihre Redoxaktivität, also z. B. ihre Eigenschaft von einem geeigneten Reduktionsmittel |
| | Elektronen aufzunehmen, erst nach Übertragung eines Elektrons von einem Elektron-Akzeptor "A" auf ein Oxidationsmittel entfalten, wenn nur der oxidierte Akzeptor "A⁺ " ein Elektron vom Donor D aufnehmen kann und das Reduktionsmittel nur auf den oxidierten Donor "D⁺" der redoxaktiven Einheit Elektronen übertragen kann, z. B. in Gegenwart eines Reduktionsmittels, das D⁺, jedoch nicht D, reduziert kann (sukzessive Ladungsübertragung). Insbesondere kann dieses Reduktionsmittel auch eine Elektrode sein, z. B. wenn die Elektrode auf ein Potential gesetzt wird, bei dem D⁺, jedoch nicht D, reduziert wird. |
| photoinduzierbar redoxaktives Protein/Enzym | besteht in der Regel aus sogenanntem Apoprotein, dem (den) bevorzugten Makromolekül(en) der vorliegenden Erfindung, und Cofaktoren, den Elektron-Donor(en) und Elektron-Akzeptor(en) im Sinne der vorliegenden Erfindung. Die photoinduzierte Ladungstrennung innerhalb des photoaktivierbar redoxaktiven Proteins/Enzyms wird durch Licht bestimmter oder beliebiger Wellenlänge ausgelöst. So sind zum Beispiel im photosynthetischen Reaktionszentrum (reaction center, RC) als Cofaktoren ein primärer Elektron-Donor P und mehrere verschiedene Elektron-Akzeptoren A, darunter auch Quinon-Cofaktor(en) Q, in eine Proteinmatrix eingebettet und bilden so eine "polymolekulare" Einheit (vgl. Struktur 1). Die Einbettung erfolgt in diesem Fall durch Einkapselung der Cofaktoren in passende Kavitäten, sogennante Bindungstaschen der Proteinmatrix aus mehreren Protein-Untereinheiten. Sowohl die Protein-Untereinheiten als auch die Einkapselung der Cofaktoren in die Proteinmatrix ist im Fall einiger natürlich vorkommender RC durch nicht-kovalente Bindungen realisiert. Bei Lichteinstrahlung geeigneter Wellenlänge gibt der primäre Donor ein Elektron an einen der Elektron-Akzeptoren ab und es bildet sich, zumindest temporär, aus den anfänglich neutralen Cofaktoren ein ladungsgetrennter RC-Zustand P⁺A⁻, insbesondere auch der Zustand P⁺Q⁻. |
| chemisch induzierbar redoxaktives Protein/ Enzym | entspricht in Zusammensetzung und Funktionsweise einem photoinduzierbar redoxaktiven Protein/Enzym, wobei aber im Unterschied zur Funktionsweise einer photoinduzierbar |
| | redoxaktiven Einheit Photoaktivierung als äußerer Umstand zur Entfaltung der Redoxaktivität der redoxaktive Einheit ausgeschlossen ist; das chemisch induzierbar redoxaktive Protein/Enzym besteht in der Regel aus sogenanntem Apoprotein, dem (den) bevorzugten Makromolekül(en) der vorliegenden Erfindung, und Cofaktoren, den Elektron-Donor(en) und Elektron-Akzeptor(en) im Sinne der vorliegenden Erfindung. Die Eigenschaft des redoxaktiven Proteins/Enzyms zur sukzessiven Ladungsübertragung wird durch eine freie redoxaktive Substanz (Substrat) ausgelöst. |
| photoinduzierbar redoxaktiver, verknüpfter, wenigstens bimolekularer Elekton-Donor-/Elektron-Akzeptor-Komplex | Verbindung aus einem oder mehreren Elektron-Donor Molekülen D1, D2, D3 etc. und mindestens einem oder mehreren geeigneten Elektron-Akzeptor Molekülen A1, A2, A3 etc., wobei die Elektron-Donor(en) und Elektron-Akzeptor(en) untereinander durch eine oder mehrere kovalente oder ionische Bindungen, durch Wasserstoff-Brücken-Bindungen, van-der-Waals-Brücken, durch π-π-Wechselwirkung oder durch Koordination mittels Elektronenpaar-Donation und -Akzeptation miteinander verbunden sind. Kovalente Verbindungen in diesem Sinne können direkte oder indirekte (z. B. über einen Spacer, nicht aber über ein Nukleinsäure-Oligomer) Verbindungen sein. Wesentliche Merkmale des photoinduzierbar redoxaktiven, verknüpften, wenigstens bimolekularen Elekton-Donor-/Elektron-Akzeptor-Komplexes sind neben der Zusammensetzung aus Elektron-Donor(en) und Elektron-Akzeptor(en): (i) der Elekton-Donor-/Elektron-Akzeptor-Komplex ist in den erfindungsrelevanten Erscheinungsformen (Elektron-Donor(en) und Elektron-Akzeptor(en) im ursprünglichen bzw. oxidierten oder reduzierten Zustand) stabil und dissoziiert nicht in seine Bestandteile, (ii) die Einheit enthält keine Nukleinsäure, (iii) die Zusammensetzung des wenigstens bimolekularen Elekton-Donor-/Elektron-Akzeptor-Komplexes aus Elektron-Donor(en) und Elektron-Akzeptor(en) kann - unabhängig von der Bindung zwischen den Bestandteilen - vom Fachmann erkannt werden und (iv) Elektron-Donor(en) und Elektron-Akzeptor(en) des photoinduzierbar redoxaktiven, verknüpften, wenigstens bimolekularen Elekton-Donor-/Elektron-Akzeptor-Komplex |
| | wirken unter gleichen oder ähnlichen äußeren Umständen auch in Form von Einzelmolekülen in Lösung als Elektron-Donor(en) und Elektron-Akzeptor(en), d. h. auch bei freien gelösten Elektron-Donor(en) und Elektron-Akzeptor(en) kann unmittelbar oder nach Einwirkung bestimmter äußerer Umstände, entsprechend den Umständen die innerhalb der photoinduzierbar redoxaktiven Einheit zu einem Elektrontransfer führen, ein Elektron vom (von den) gelösten Elektron-Donor(en) auf den (die) gelösten Elektron-Akzeptor(en) übertragen werden. Der photoinduzierbar redoxaktive, verknüpfte, wenigstens bimolekulare Elekton-Donor-/Elektron-Akzeptor-Komplex entspricht in seiner erfindungsrelevanten Funktionsweise einem photoinduzierbar redoxaktiven Protein/Enzym, d. h. auch hier kommt es durch Lichteinstrahlung geeigneter Wellenlänge zur photoinduzierten Ladungstrennung und es wird, zumindest temporär, ein ladungsgetrennter Zustand D⁺A⁻ gebildet (wobei D für ein beliebiges D1, D2, D3 etc. und A für ein beliebiges A1, A2, A3 etc. steht). Im Ausdruck "photoinduzierbar redoxaktiver, verknüpfter, wenigstens bimolekularer Elekton-Donor-/ Elektron-Akzeptor-Komplex" steht der Begriff "wenigstens bimolekular" dafür, daß der Komplex aus wenigstens einem Elektron-Donor und wenigstens einem Elektron-Akzeptor aufgebaut ist, auch wenn der Donor mit dem Akzeptor direkt (oder indirekt über einen Spacer) kovalent verbunden ist. |
| chemisch induzierbar redoxaktiver, verknüpfter, wenigstens bimolekularer Elekton-Donor-/Elektron-Akzeptor-Komplex | entspricht in Zusammensetzung und Funktionsweise einem photoinduzierbar redoxaktiven, verknüpften, wenigstens bimolekularen Elektron-Donor/Elektron-Akzeptor-Komplex, wobei aber im Unterschied zur Funktionsweise eines photoinduzierbar redoxaktiven, verknüpften, wenigstens bimolekularen Elekton-Donor-/Elektron-Akzeptor-Komplexes Photoaktivierung als äußerer Umstand zur Entfaltung der Redoxaktivität der redoxaktiven Einheit ausgeschlossen ist. Bei entsprechend gewählten äußeren Umständen entfaltet die redoxaktive Einheit ihre Redoxaktivität, also ihre Eigenschaft, z. B. an ein geeignetes Oxidationsmittel Elektronen abzugeben, erst nach Übertragung eines Elektrons von einem Reduktionsmittel auf den/einen Elektron-Donor "D", der nur im reduzierten Zustand "D⁻" ein Elektron auf den Akzeptor "A" |
| | übertragen kann und das Oxidationsmittel nur von diesem reduzierten Akzeptor "A⁻" der redoxaktiven Einheit Elektronen aufnimmt, also in Gegenwart eines Oxidationsmittels das A⁻, jedoch nicht A, oxidieren kann (sukzessive Ladungsübertragung). Insbesondere kann dieses Oxidationsmittel auch eine Elektrode sein, z. B. wenn die Elektrode auf ein Potential gesetzt wird, bei dem A⁻, jedoch nicht A, oxidiert wird. Umgekehrt kann bei abweichend gewählten äußeren Umständen die redoxaktive Einheit ihre Redoxaktivität, also z. B. ihre Eigenschaft von einem geeigneten Reduktionsmittel Elektronen aufzunehmen, erst nach Übertragung eines Elektrons von einem Elektron-Akzeptor "A" auf ein Oxidationsmittel entfalten, wenn nur der oxidierte Akzeptor "A⁺" ein Elektron vom Donor D aufnehmen kann und das Reduktionsmittel nur auf den oxidierten Donor "D⁺" der redoxaktiven Einheit Elektronen übertragen kann, z. B. in Gegenwart eines Reduktionsmittels, das D⁺, jedoch nicht D, reduzieren kann (sukzessive Ladungsübertragung). Insbesondere kann dieses Reduktionsmittel auch eine Elektrode sein, z. B. wenn die Elektrode auf ein Potential gesetzt wird, bei dem D⁺, jedoch nicht D, reduziert wird. Im Ausdruck "redoxaktiver, verknüpfter, wenigstens bimolekularer Elekton-Donor-/Elektron-Akzeptor-Komplex" steht der Begriff "wenigstens bimolekular" dafür, daß der Komplex aus wenigstens einem Elektron-Donor und wenigstens einem Elektron-Akzeptor aufgebaut ist, auch wenn der Donor mit dem Akzeptor direkt (oder indirekt über einen Spacer) kovalent verbunden ist. |
| RC | Reaktionszentrum. Beispiel eines photoinduzierbar redoxaktiven Proteins/Enzyms. Bei dem Protein/Enzym handelt es sich um einen sogenannten Pigment/Protein-Komplex der aus Apoprotein mit mehreren Proteinuntereinheiten und mehreren Cofaktoren (im Beispiel RC sogenannte Pigmente) handelt. In solchen Pigment/Protein-Komplexen spielen sich die ersten Schritte der lichtgetriebenen Ladungstrennung der bakteriellen oder pflanzlichen Photosynthese ab. Das RC der Photosynthese betreibenden Bakterien des Stammes *Rhodobacter sphaeroides z.* B. (vgl. Struktur 1) besteht aus drei Protein- |
| | Untereinheiten und acht Cofaktoren (Pigmenten). Die Cofaktoren, ein Bakteriochlorophyll-Dimer P, zwei Bakteriochlorophyll-Monomere B_{A} und B_{B}, zwei Bakteriopheophytin-Monomere H_{A} und H_{B} und zwei Ubichinon-50 (UQ) Moleküle Q_{A} und Q_{B}, sind in den jeweiligen Protein-Bindungstaschen (also der P-, B_{A}- etc. Bindungstasche) lokalisiert. |
| Q_{A}-Protein-Bindungstasche | Proteinbindungstasche bzw. Proteinumgebung, in der sich der Chinon-Cofaktor Q_{A} befindet. In RC von z.B. *Rhodobacter sphaeroides* ist der Chinon-Cofaktor Q_{A} ein Ubichinon-50 (vgl. Struktur 1). |
| Q_{A}-Bindungstasche | Q_{A}-Protein-Bindungstasche |

| ***Chemische Substanzen*/*Gruppen*** | |
|---|---|
| ZnBChl | Zn-Bakteriochlorophyll (Formel 11 mit M = Zn) |
| Q | allgemein für Chinon (engl. Quinone), im Beispiel 3 und den sich darauf beziehenden Textpassagen ist Q ein modifiziertes Anthrachinon oder Pyrrollochinolinochinon (PQQ). |
| UQ | Ubichinon-50, RC-Cofaktor und temporärer Elektron-Akzeptor z. B. im RC der Photosynthese betreibenden Bakterien aus z. B. *Rhodobacter sphaeroides* oder *Rhodopseudomonas viridis.* |
| (cyt c₂)²⁺ | reduzierte Form des Cytochrom c₂, ein frei bewegliches Häm-Protein, das in der bakteriellen Photosynthese in *Rhodobacter sphaeroides* den oxidierten primären Donor P⁺ zu P reduziert; Beispiel einer redoxaktiven Substanz. |
| PQQ | Pyrrolo-Chinolino-Chinon, entspricht: 4,5-Dihydro-4,5-dioxo-1H-pyrrolo-[2,3-f]-chinolin-2,7,9-tricarboxylsäure) |
| EDTA | Ethylendiamin-Tetraacetat (Natriumsalz) |
| sulfo-NHS | N-Hydroxysulfosuccinimid |
| EDC | (3-Dimethylaminopropyl)-carbodiimid |
| HEPES | N-[2-Hydroxyethyl]piperazin-N'-[2-ethansulfonsäure] |
| Tris | Tris-(hydroxymethyl)-aminomethan |
| Alkyl | Der Begriff "Alkyl" bezeichnet eine gesättigte Kohlenwasserstoffgruppe, die geradkettig oder verzweigt ist (z.B. Ethyl, 2,5-Dimethylhexyl oder Isopropyl etc.). Wenn "Alkyl" benutzt wird, um auf einen Linker oder Spacer zu verweisen, bezeichnet der Begriff eine Gruppe mit zwei verfügbaren Valenzen für die kovalente Verknüpfung (z. B. -CH₂CH₂-, -CH₂C(CH₃)₂CH₂CH₂C(CH₃)₂CH₂- oder -CH₂CH₂CH₂-etc.). Bevorzugte Alkylgruppen als Substituenten oder Seitenketten R sind solche der Kettenlänge 1 - 30 (längste durchgehende Kette von kovalent aneinander gebundenen Atomen). Bevorzugte Alkylgruppen als Linker oder Spacer sind solche der Kettenlänge 1 - 20, insbesondere der Kettenlänge 1 - 14, wobei die Kettenlänge hier die kürzeste durchgehende Verbindung zwischen den durch den Linker oder Spacer verbundenen Strukturen, also zwischen den zwei Molekülen bzw. zwischen einem Oberflächenatom, Oberflächenmolekül oder einer Oberflächenmolekülgruppe und einem anderen Molekül, darstellt. |
| Alkenyl | Alkylgruppen, bei denen eine oder mehrere der C-C Einfachbindungen durch C=C Doppelbindungen ersetzt sind. |
| Alkinyl | Alkyl- oder Alkenylgruppen, bei denen eine oder mehrere der C-C Einfach- oder C=C Doppelbindungen durch C≡C Dreifachbindungen ersetzt sind. |
| Hetero-Alkyl | Alkylgruppen, bei denen eine oder mehrere der C-H Bindungen oder C-C Einfachbindungen durch C-N, C=N, C-P, C=P, C-O, C=O, C-S oder C=S Bindungen ersetzt sind. |
| Hetero-Alkenyl | Alkenylgruppen, bei denen eine oder mehrere C-H Bindungen, C-C Einfach- oder C=C Doppelbindungen durch C-N, C=N, C-P, C=P, C-O, C=O, C-S oder C=S Bindungen ersetzt sind. |
| Hetero-Alkinyl | Alkinylgruppen, bei denen eine oder mehrere der C-H Bindungen, C-C Einfach-, C=C Doppel- oder C≡C Dreifachbindung durch C-N, C=N, C-P, C=P, C-O, C=O, C-S oder C=S Bindungen ersetzt sind. |
| Linker | molekulare Verbindung zwischen zwei Molekülen bzw. zwischen einem Oberflächenatom, Oberflächenmolekül oder einer Oberflächenmolekülgruppe und einem anderen Molekül. |
| | In der Regel sind Linker als Alkyl-, Alkenyl-, Alkinyl-, Hetero-Alkyl-, Hetero-Alkenyl- oder Heteroalkinylkette käuflich zu erwerben, wobei die Kette an zwei Stellen mit (gleichen oder verschiedenen) reaktiven Gruppen derivatisiert ist. Diese Gruppen bilden in einfachen/bekannten chemischen Reaktionen mit den entsprechenden Reaktionspartner eine kovalente chemische Bindung aus. Die reaktiven Gruppen können auch photoaktivierbar sein, d. h. die reaktiven Gruppen werden erst durch Licht bestimmter oder beliebiger Wellenlänge aktiviert. Bevorzugte Linker sind solche der Kettenlänge 1 - 20, insbesondere der Kettenlänge 1 - 14, wobei die Kettenlänge hier die kürzeste durchgehende Verbindung zwischen den zu verbindenden Strukturen, also zwischen den zwei Molekülen bzw. zwischen einem Oberflächenatom, Oberflächenmolekül oder einer Oberflächenmolekülgruppe und einem anderen Molekül, darstellt. |
| Spacer | Linker, der über die reaktiven Gruppen an eine oder beide der zu verbindenden Strukturen (siehe Linker) kovalent angebunden ist. Bevorzugte Spacer sind solche der Kettenlänge 1 - 20, insbesondere der Kettenlänge 1 - 14, wobei die Kettenlänge die kürzeste durchgehende Verbindung zwischen den zu verbindenden Strukturen darstellt. |
| (n x HS-Spacer)-oligo | Nukleinsäure-Oligomer, an das n Thiolfunktionen über jeweils einen Spacer anqebunden sind, wobei die Spacer jeweils eine unterschiedliche Kettenlänqe (kürzeste durchgehende Verbindung zwischen Thiolfunktion und Nukleinsäure- Oligomer) aufweisen können, insbesondere jeweils eine beliebige Kettenlänqe zwischen 1 und 14. Diese Spacer können wiederum an verschiedene natürlich am Nukleinsäure-Oligomer vorhandene oder an diesem durch Modifikation angebrachte reaktive Gruppen gebunden sein und "n" ist eine beliebige qanze Zahl, insbesondere eine Zahl zwischen 1 und 20. |
| (n x R-S-S-Spacer)-oligo | Nukleinsäure-Oliqomer, an das n Disulfidfunktionen über jeweils einen Spacer anqebunden sind, wobei ein beliebiger Rest R die Disulfidfunktion absättigt. Der Spacer zur Anbindung der Disulfidfunktion an das Nukleinsäure-Oligomer |
| | kann jeweils eine unterschiedliche Kettenlänge (kürzeste durchgehende Verbindung zwischen Disulfidfunktion und Nukleinsäure-Oligomer) aufweisen, insbesondere jeweils eine beliebiqe Kettenlänge zwischen 1 und 14. Diese Spacer können wiederum kann an verschiedene natürlich am Nukleinsäure-Oligomer vorhandene oder an diesem durch Modifikation angebrachte reaktive Gruppen gebunden sein. Der Platzhalter n ist eine beliebige ganze Zahl, insbesondere eine Zahl zwischen 1 und 20. |
| oligo-Spacer-S-S-Spacer-oligo | zwei gleiche oder verschiedene Nukleinsäure-Oligomere, die über eine Disulfid-Brücke miteinander verbunden sind, wobei die Disulfidbrücke über zwei beliebiqe Spacer an die Nukleinsäure-Oligomere angebunden ist und die beiden Spacer eine unterschiedliche Kettenlänge (kürzeste durchgehende Verbindung zwischen Disulfidbrücke und dem ieweiligen Nukleinsäure-Oligomer) aufweisen können, insbesondere jeweils eine beliebiqe Kettenlänge zwischen 1 und 14 und diese Spacer wiederum an verschiedene natürlich am Nukleinsäure-Oligomer vorhandene oder an diese durch Modifikation angebrachte reaktive Gruppen gebunden sein können. |

| ***Modifizierte Oberflächen*/*Elektroden*** | |
|---|---|
| Mica | Muskovit-Plättchen, Trägermaterial zum Aufbringen dünner Schichten. |
| *Au-S-(CH*_{*2*}*)*_{*2*}*-ss-oligo- Spacer-UQ(RC)* | Gold-Film auf Mica mit kovalent aufgebrachter Monolayer aus derivatisiertem 12Bp Einzelstrang DNA-Oligonukleotid (Sequenz: TAGTCGGAAGCA). Hierbei ist die endständige Phosphatgruppe des Oligonukleotids am 3' Ende mit (HO-(CH₂)₂-S)₂ zum P-O-(CH₂)₂-S-S-(CH₂)₂-OH verestert, wobei die S-S Bindung homolytisch gespalten wird und je eine Au-S-R Bindung bewirkt. Die endständige Base Thymin am 5'- Ende des Oligonukleotids ist am C-5 Kohlenstoff mit -CH=CH-CO-NH-CH₂-CH₂-NH₂ modifiziert, wobei dieser Rest wiederum über seine freie Aminogruppe durch Amidbildung mit der Carbonsäuregruppe des modifizierten Ubichinon-50 verbunden ist. Anschließend wird das UQ mit dem restlichen RC rekonstituiert. |
| *Au-S-(CH*_{*2*}*)*_{*2*}*-ds-oligo-* | *Au-S-(CH*_{*2*}*)*_{*2*}*-ss-oligo-Spacer-UQ(RC)* hybridisiert mit dem zu ss- |
| *Spacer-UQ(RC)* | oligo (Sequenz: TAGTCGGMGCA) komplementären Oligonukleotid. |
| *Au-S-(CH*_{*2*}*)*_{*2*}*-ss-oligo*-*Spacer-Q-ZnBChl* | identisch zu *Au-S-(CH*_{*2*}*)*_{*2*}*-ss-oligo-Spacer-UQ(RC)* mit der Ausnahme, daß, statt des über UQ angebundenen RCs, Q-ZnBChl als photoinduzierbar redoxaktive Einheit angebunden ist. |
| *Au-S-(CH*_{*2*}*)*_{*2*}*-ds-oligo- Spacer-Q-ZnBChl* | *Au-S-(CH*_{*2*}*)*_{*2*}*-ss-oligo-Spacer-Q-ZnBChl* hybridisiert mit dem zu ss-oligo (Sequenz: TAGTCGGAAGCA) komplementären Oligonukleotid. |

| ***Elektrochemie*** | |
|---|---|
| *E* | Elektrodenpotential, das an der Arbeitselektrode anliegt. |
| *E*_{Ox} | Potential beim Strom-Maximum der Oxidation einer reversiblen Elektrooxidation oder-reduktion. |
| *i* | Stromdichte (Strom pro cm² Elektrodenoberfläche) |
| Cyclovoltametrie | Aufzeichnung einer Strom/Spannungskurve. Hierbei wird das Potential einer stationären Arbeitselektrode zeitabhängig linear verändert, ausgehend von einem Potential, bei dem keine Elektrooxidation oder -reduktion stattfindet bis zu einem Potential, bei dem eine gelöste oder an die Elektrode adsorbierte Spezies oxidiert oder reduziert wird (also Strom fließt). Nach Durchlaufen des Oxidations- bzw. Reduktionsvorgangs, der in der Strom/ Spannungskurve einen zunächst ansteigenden Strom und nach Erreichen eines Maximums einen allmählich abfallenden Strom erzeugt, wird die Richtung des Potentialvorschubs umgekehrt. Im Rücklauf wird dann das Verhalten der Produkte der Elektrooxidation oder-reduktion aufgezeichnet. |
| Amperometrie | Aufzeichnung einer Strom/Zeitkurve. Hierbei wird das Potential einer stationären Arbeitselektrode z. B. durch einen Potentialsprung auf ein Potential gesetzt, bei dem die Elektrooxidation oder -reduktion einer gelösten oder adsorbierten Spezies stattfindet und der fließende Strom wird in Abhängigkeit von der Zeit aufgezeichnet. |

Die vorliegende Erfindung betrifft ein Nukleinsäure-Oligomer, das durch chemische Bindung einer redoxaktiven Einheit modifiziert ist. Die redoxaktive Einheit ist entweder eine photoinduzierbar redoxaktive Einheit oder eine chemisch induzierbar redoxaktive Einheit. Die photoinduzierbar redoxaktive Einheit kann nach photoinduzierter Abgabe eines Elektrons an ein externes Oxidationsmittels, z. B. einer Elektrode, oder Aufnahme eines Elektrons von einem externen Reduktionsmittel, z. B. einer Elektrode, durch eine freie redoxaktive Substanz re-reduziert bzw. re-oxidiert, also in seinen ursprünglichen Zustand zurückversetzt werden. Die chemisch induziert redoxaktive Einheit kann nach Abgabe eines Elektrons an ein externes Oxidationsmittels von einem externen Reduktionsmittel, z. B. einer Elektrode, reduziert oder nach Aufnahme eines Elektrons von einem externen Reduktionsmittel durch ein externes Oxidationsmittel, z. B. einer Elektrode, oxidiert werden.

Als Nukleinsäure-Oligomer wird im Rahmen der vorliegenden Erfindung eine Verbindung aus wenigstens zwei kovalent verbundenen Nukleotiden oder aus wenigstens zwei kovalent verbundenen Pyrimidin- (z. B. Cytosin, Thymin oder Uracil) oder Purin-Basen (z. B. Adenin oder Guanin), bevorzugt ein DNA-, RNA- oder PNA-Fragment, verwendet. In der vorliegenden Erfindung bezieht sich der Begriff Nukleinsäure auf ein beliebiges "Rückgrat" der kovalent verbundenen Pyrimidin- oder Purin-Basen, wie z. B. auf das Zucker-Phosphat Rückgrat der DNA, cDNA oder RNA, auf ein Peptid-Rückgrat der PNA oder auf analoge Rückgrat-Strukturen, wie z. B. ein Thio-Phosphat-, ein Dithio-Phosphat- oder ein Phosphoramid-Rückgrat. Wesentliches Merkmal einer Nukleinsäure im Sinne der vorliegenden Erfindung ist, daß sie natürlich vorkommende cDNA oder RNA sequenzspezifisch binden kann. Alternativ zu dem Begriff "Nukleinsäure-Oligomer" werden die Begriffe "(Sonden-) Oligonukleotid", "Nukleinsäure" oder "Oligomer" verwendet.

Der Begriff "Elektron-Akzeptor" bzw. "Elektron-Akzeptor-Molekül" und der Begriff "Elektron-Donor" bzw. "Elektron-Donor-Molekül" bezeichnet im Rahmen der vorliegenden Erfindung einen Bestandteil einer redoxaktiven Einheit.

Unter einer "redoxaktiven Einheit" wird im Rahmen der vorliegenden Erfindung jede Einheit verstanden, die einen oder mehrere Elektron-Donor-Moleküle und einen oder mehrere Elektron-Akzeptor-Moleküle enthält. Die Elektron-Donor-Molekül(e) oder-Molekülteil(e) und Elektron-Akzeptor-Molekül(e) oder -Molekülteil(e) dieser redoxaktiven Einheit können untereinander durch eine oder mehrere kovalente oder ionische Bindungen, durch Wasserstoff-Brücken-Bindungen, van-der-Waals-Brücken, durch π-π-Wechselwirkung oder durch Koordination mittels Elektronenpaar-Donation und -Akzeptation miteinander verbunden sein, wobei kovalente Bindungen direkte oder indirekte (z. B. über einen Spacer, nicht aber über ein Nukleinsäure-Oligomer) Bindungen sein können. Außerdem können die Elektron-Donor-Molekül(e) und/oder Elektron-Akzeptor-Molekül(e) in ein oder mehrere Makromolekül(e) eingebunden sein, wobei diese Einbindung durch Einkapseln in passende molekulare Kavitäten (Bindungstaschen) des Makromoleküls (der Makromoleküle), durch Wasserstoff-Brücken-Bindungen, van-der-Waals-Brücken, π-π-Wechselwirkung oder durch Koordination mittels Elektronenpaar-Donation und - Akzeptation zwischen dem(n) Makromolekül(en) und dem(n) Elektron-Donor-Molekül(en) und/oder dem(n) Elektron-Akzeptor-Molekül(en) erfolgt. In diesem Fall bilden also die Makromolekül(e) und die Elektron-Donor-Molekül(e) und die Elektron-Akzeptor-Molekül(e) die redoxaktive Einheit. Sind mehrere Makromoleküle Bestandteil der redoxaktiven Einheit kann die Bindung der Makromoleküle untereinander ebenfalls kovalent, ionisch, durch Wasserstoff-Brücken-Bindungen, van-der-Waals-Brücken, π-π-Wechselwirkung oder durch Koordination mittels Elektronenpaar-Donation und -Akzeptation erfolgen.

Die angesprochenen Donor- und Akzeptor-Moleküle bilden erfindungsgemäß eine redoxaktive Einheit, d. h. sie sind direkt oder über weitere Molekülteile aneinander gebunden. Einzige erfindungsgemäße Einschränkung der die Bestandteile der redoxaktiven Einheit verbindenden Moleküle oder Molekülteile ist der Ausschluß von Nukleinsäure-Oligomeren. Gemäß der vorliegenden Erfindung ist die redoxaktive Einheit als eine komplette Einheit an das Sonden-Oligonukleotid gebunden, wobei natürlich mehrere chemische Bindungen zwischen Oligonukleotid und der redoxaktiven Einheit ausgebildet werden können. Durch den Ausschluß von Nukleinsäure-Oligomeren als die die Bestandteile der redoxaktiven Einheit verbindenden Moleküle oder Molekülteile soll verdeutlicht werden, daß nicht einzelne Teile der redoxaktiven Einheit an verschiedenen Stellen des Sonden-Oligonukleotids angebunden sind. Das Sonden-Oligonukleotid stellt also explizit nicht die Verbindung zwischen den Elektron-Donor-Molekül(en) oder -Molekülteil(en) und den Elektron-Akzeptor-Molekül(en) oder-Molekülteil(en) der redoxaktiven Einheit dar.

Die redoxaktive Einheit ist entweder eine photoinduzierbar redoxaktive Einheit oder eine chemisch induzierbar redoxaktive Einheit.

"Photoinduzierbar" heißt im Rahmen der vorliegenden Erfindung, daß die Redoxaktivität der photoinduzierbar redoxaktiven Einheit, also deren Eigenschaft unter bestimmten äußeren Umständen an ein geeignetes Oxidationsmittel Elektronen abzugeben oder von einem geeigneten Reduktionsmittel Elektronen aufzunehmen, erst durch Einstrahlen von Licht bestimmter oder beliebiger Wellenlänge entfaltet wird. Durch Einstrahlung von Licht bestimmter oder beliebiger Wellenlänge gibt der Elektron-Donor "D" an einen der Elektron-Akzeptoren "A" ein Elektron ab und es bildet sich, zumindest temporär, ein ladungsgetrennter Zustand D⁺A⁻ aus oxidiertem Donor und reduziertem Akzeptor. Dieser Vorgang innerhalb der photoinduzierbar redoxaktiven Einheit wird als photoinduzierte Ladungstrennung bezeichnet. Bei entsprechend gewählten äußeren Umständen entfaltet die photoinduzierbar redoxaktive Einheit ihre Redoxaktivität erst im ladungsgetrennten Zustand, da das Reduktionsmittel (bzw. das Oxidationsmittel) nur auf den oxidierten Donor (bzw. vom reduzierten Akzeptor) der photoinduzierbar redoxaktiven Einheit Elektronen übertragen kann (bzw. aufnehmen kann), z. B. in Gegenwart eines Oxidationsmittels, das A⁻, jedoch nicht A, oxidieren kann (bzw. in Gegenwart eines Reduktionsmittels, das D⁺, jedoch nicht D, reduzieren kann).

Insbesondere kann das angesprochene Oxidations- bzw. Reduktionsmittel eine Elektrode sein, wobei die photoinduzierbar redoxaktive Einheit erst nach der photoinduzierten Ladungstrennung ein Elektron an die Elektrode abgeben (bzw. von dieser aufnehmen) kann, z. B. wenn die Elektrode auf ein Potential gesetzt wird, bei dem A⁻, jedoch nicht A, oxidiert (bzw. D⁺, jedoch nicht D, reduziert) wird. Daneben kann das Oxidations- bzw. Reduktionsmittel eine freie, redoxaktive Substanz sein, wobei die photoinduzierbar redoxaktive Einheit erst nach der photoinduzierten Ladungstrennung ein Elektron an die freie, redoxaktive Substanz abgeben (bzw. von dieser aufnehmen) kann, z. B. wenn die freie redoxaktive Substanz A⁻, jedoch nicht A, oxidiert (bzw. D⁺, jedoch nicht D, reduziert).

"Chemisch induzierbar" heißt im Rahmen der vorliegenden Erfindung, daß die Redoxaktivität der chemisch induzierbar redoxaktiven Einheit eine redoxaktive Einheit verstanden, also deren Eigenschaft unter bestimmten äußeren Umständen an ein geeignetes Oxidationsmittel Elektronen abzugeben (bzw. von einem geeigneten Reduktionsmittel Elektronen aufzunehmen), erst nach Reduktion (bzw. nach Oxidation) durch ein externes Reduktionsmittel (bzw. Oxidatinsmittel) entfaltet wird. Die chemisch induzierbar redoxaktiven Einheit entspricht in Zusammensetzung und Funktionsweise einer photoinduzierbar redoxaktive Einheit, wobei aber im Unterschied zur Funktionsweise einer photoinduzierbar redoxaktiven Einheit Photoaktivierung als äußerer Umstand zur Entfaltung der Redoxaktivität der redoxaktive Einheit ausgeschlossen ist. Bei entsprechend gewählten äußeren Umständen entfaltet die chemisch induzierbar redoxaktive Einheit ihre Redoxaktivität, also ihre Eigenschaft, z. B. an ein geeignetes Oxidationsmittel Elektronen abzugeben, erst nach Übertragung eines Elektrons von einem Reduktionsmittel auf den/einen Elektron-Donor "D": Nur im reduzierten Zustand "D⁻ " kann der Elektron-Donor ein Elektron auf den Akzeptor "A" übertragen und das Oxidationsmittel kann nur von diesem reduzierten Akzeptor "A⁻" der redoxaktiven Einheit Elektronen aufnehmen, z.B. in Gegenwart eines Oxidationsmittels das A⁻, jedoch nicht A, oxidieren kann (sukzessive Ladungsübertragung). Insbesondere kann das besagte Oxidationsmittel auch eine Elektrode sein, z. B. wenn die Elektrode auf ein Potential gesetzt wird, bei dem A⁻, jedoch nicht A, oxidiert wird. Umgekehrt kann - bei abweichend gewählten äußeren Umständen - die chemisch induzierbar redoxaktive Einheit ihre Redoxaktivität, also z. B. ihre Eigenschaft von einem geeigneten Reduktionsmittel Elektronen aufzunehmen, erst nach Übertragung eines Elektrons von einem Elektron-Akzeptor "A" auf ein Oxidationsmittel entfalten: Nur im oxidierten Zustand "A⁺" kann der Elektron-Akzeptor ein Elektron vom Donor D aufnehmen und das Reduktionsmittel kann nur auf den oxidierten Donor "D⁺" der redoxaktiven Einheit Elektronen übertragen, z. B. in Gegenwart eines Reduktionsmittels, das D⁺, jedoch nicht D, reduzieren kann (sukzessive Ladungsübertragung). Insbesondere kann das besagte Reduktionsmittel auch eine Elektrode sein, z. B. wenn die Elektrode auf ein Potential gesetzt wird, bei dem D⁺, jedoch nicht D, reduziert wird.

Wesentliche Merkmale der photoinduzierbar oder chemisch induzierbar redoxaktiven Einheit sind neben der Zusammensetzung aus Elektron-Donor(en) und Elektron-Akzeptor(en) oder aus Elektron-Donor(en) und Elektron-Akzeptor(en) und Makromolekül(en): (i) die Einheit ist in den erfindungsrelevanten Erscheinungsformen (Elektron-Donor(en) und Elektron-Akzeptor(en) im ursprünglichen bzw. oxidierten oder reduzierten Zustand) stabil und dissoziiert nicht in ihre Bestandteile, (ii) die Einheit enthält keine Nukleinsäure, (iii) die Zusammensetzung der Einheit aus Elektron-Donor(en) und Elektron-Akzeptor(en) oder aus Elektron-Donor(en) und Elektron-Akzeptor(en) und Makromolekül(en) kann - unabhängig von der Bindung zwischen den Bestandteilen - vom Fachmann erkannt werden, da Elektron-Donor(en) und Akzeptor(en) prinzipiell auch als Einzelmoleküle vorkommen können und (iv) Elektron-Donor(en) und Elektron-Akzeptor(en) der redoxaktiven Einheit wirken unter gleichen oder ähnlichen äußeren Umständen wie in ihrer erfindungsrelevanten Erscheinung als Bestandteile der redoxaktiven Einheit auch als Einzelmoleküle in Lösung als Elektron-Donor(en) und Elektron-Akzeptor(en), d. h. auch bei freien gelösten Elektron-Donor(en) und Elektron-Akzeptor(en) kann unmittelbar oder nach Einwirkung bestimmter äußerer Umstände, entsprechend den Umständen die innerhalb der redoxaktiven Einheit zu einem Elektrontransfer führen, ein Elektron vom (von den) gelösten Elektron-Donor(en) auf den (die) gelösten Elektron-Akzeptor(en) übertragen werden. Wie für die Elekron-Donor(en) und Elektron-Akzeptor(en) der photoinduzierbar redoxaktiven Einheit kann ein solcher äußerer Umstand für die freien, gelösten Elekron-Donor(en) und Elektron-Akzeptor(en) die Lichtabsorption durch den (die) freien, gelösten Elektron-Donor(en) oder -Akzeptor(en) sein, wobei der (ein) Elektron-Donor "D" an den (einen) Elektron-Akzeptor "A" ein Elektron abgibt und, zumindest temporär, ein ladungsgetrennter Zustand D⁺A⁻ aus einem freien, gelösten oxidierten Donor und einem freien gelösten, reduzierten Akzeptor gebildet wird. Ein weiterer solcher äußerer Umstand kann - wie für die Elekron-Donor(en) und Elektron-Akzeptor(en) der chemisch induzierbar redoxaktiven Einheit - die Übertragung eines Elektrons auf den freien, gelösten Elektron-Donor durch ein Reduktionsmittel bzw. die Abgabe eines Elektrons durch den freien, gelösten Elektron-Akzeptor an ein Oxidationsmittel sein.

Die photoinduzierbar redoxaktive Einheit kann z. B. jedes beliebige photoinduzierbar redoxaktive Protein/Enzym oder jeder beliebige photoinduzierbar redoxaktive, verknüpfte, wenigstens bimolekulare Elekton-Donor-/Elektron-Akzeptor-Komplex sein. Im Ausdruck "photoinduzierbar redoxaktiver, verknüpfter, wenigstens bimolekularer Elekton-Donor-/Elektron-Akzeptor-Komplex" steht der Begriff "wenigstens bimolekular" dafür, daß der Komplex aus wenigstens einem Elektron-Donor und wenigstens einem Elektron-Akzeptor aufgebaut ist, auch wenn dieser Donor und dieser Akzeptor direkt (oder indirekt über einen Spacer) kovalent verbunden sind. Durch Einstrahlung von Licht bestimmter oder beliebiger Wellenlänge gibt der/ein Elektron-Donor an einen der Elektron-Akzeptoren ein Elektron ab und es bildet sich, zumindest temporär, ein ladungsgetrennter Zustand D⁺A⁻ aus einem oxidierten Donor und einem reduzierten Akzeptor. Dieser Vorgang innerhalb der photoinduzierbar redoxaktiven Einheit wird als photoinduzierte Ladungstrennung bezeichnet. Bei entsprechend gewählten äußeren Umständen entfaltet die photoinduzierbar redoxaktive Einheit ihre Redoxaktivität, also ihre Eigenschaft, an einen geeignetes Oxidationsmittel Elektronen abzugeben oder von einem geeigneten, Reduktionsmittel Elektronen aufzunehmen, erst im ladungsgetrennten Zustand, da das Reduktionsmittel (bzw. Oxidationsmittel) nur auf den oxidierten Donor (bzw. vom reduzierten Akzeptor) der photoinduzierbar redoxaktiven Einheit Elektronen überträgt (bzw. aufnimmt), z. B. in Gegenwart eines Reduktionsmittels, das D⁺, jedoch nicht D, reduziert kann (bzw. in Gegenwart eines Oxidationsmittels das A⁻, jedoch nicht A, oxidieren kann). Insbesondere kann dieses Oxidations- bzw. Reduktionsmittel auch eine Elektrode sein, wobei die photoinduzierbar redoxaktive Einheit erst nach der photoinduzierten Ladungstrennung ein Elektron an eine Elektrode abgeben (bzw. von dieser aufnehmen), z. B. wenn die Elektrode auf ein Potential gesetzt wird, bei dem A⁻, jedoch nicht A, oxidiert (bzw. D⁺, jedoch nicht D, reduziert) wird.

Die chemisch induzierbar redoxaktive Einheit kann z. B. jedes beliebige chemisch induzierbar redoxaktive Protein/Enzym oder jeder beliebige chemisch induzierbar redoxaktive, verknüpfte, wenigstens bimolekulare Elekton-Donor-/Elektron-Akzeptor-Komplex sein. Im Ausdruck "chemisch induzierbar redoxaktiver, verknüpfter, wenigstens bimolekularer Elekton-Donor-/Elektron-Akzeptor-Komplex" steht der Begriff "wenigstens bimolekular" dafür, daß der Komplex aus wenigstens einem Elektron-Donor und wenigstens einem Elektron-Akzeptor aufgebaut ist, auch wenn dieser Donor und dieser Akzeptor direkt (oder indirekt über einen Spacer) kovalent verbunden sind. Bei entsprechend gewählten äußeren Umständen entfaltet die chemisch induzierbar redoxaktive Einheit ihre Redoxaktivität, also deren Eigenschaft unter bestimmten äußeren Umständen an ein geeignetes Oxidationsmittel Elektronen abzugeben (bzw. von einem geeigneten Reduktionsmittel Elektronen aufzunehmen), erst nach Reduktion (bzw. nach Oxidation) durch ein externes Reduktionsmittel (bzw. Oxidationsmittel). Erst nach Übertragung eines Elektrons von einem Reduktionsmittel auf den/einen Elektron-Donor "D" kann der dann reduzierte Donor "D⁻ " ein Elektron auf den Akzeptor "A" übertragen und das Oxidationsmittel kann nur von diesem reduzierten Akzeptor "A⁻" der redoxaktiven Einheit Elektronen aufnehmen. Insbesondere kann das angesprochene Oxidationsmittel auch eine Elektrode sein, z. B. wenn die Elektrode auf ein Potential gesetzt wird, bei dem A⁻, jedoch nicht A, oxidiert wird. Umgekehrt kann - bei abweichend gewählten äußeren Umständen - der Elektron-Akzeptor der chemisch induzierbar redoxaktive Einheit erst nach Übertragung eines Elektrons vom Akzeptor "A" auf ein externes Oxidationsmittel in seinem dann oxidierten Zustand "A⁺" ein Elektron vom Donor D aufnehmen und das Reduktionsmittel nur auf den oxidierten Donor "D⁺ " der redoxaktiven Einheit Elektronen übertragen. Insbesondere kann das angesprochene Reduktionsmittel auch eine Elektrode sein, z. B. wenn die Elektrode auf ein Potential gesetzt wird, bei dem D⁺, jedoch nicht D, reduziert wird.

Mit dem Begriff "Oxidationsmittel" wird im Rahmen der vorliegenden Erfindung eine chemische Verbindung (chemische Substanz) bezeichnet, die durch Aufnahme von Elektronen aus einer anderen chemischen Verbindung (chemische Substanz, Elektron-Donor, Elektron-Akzeptor) diese andere chemische Verbindung (chemischen Substanz, Elektron-Donor, Elektron-Akzeptor) oxidiert. Das Oxidationsmittel verhält sich analog zu einem Elektron-Akzeptor, wird aber im Rahmen der vorliegenden Erfindung als Begriff für einen externen, nicht zur redoxaktiven Einheit gehörigen Elektron-Akzeptor verwendet. "Nicht unmittelbar" bedeutet in diesem Zusammenhang, daß das Oxidationsmittel entweder eine freie redoxaktive Substanz ist, die nicht an das Nukleinsäure-Oligomer gebunden ist, aber mit diesem in Kontakt steht oder daß das Oxidationsmittel kovalent an das Nukleinsäure-Oligomer angebunden ist, jedoch an einer Stelle des Nukleinsäure-Oligomers, die mindestens zwei kovalent verbundene Nukleotide oder wenigstens zwei kovalent verbundene Pyrimidin- oder Purin-Basen von der kovalenten Anbindungstelle der redoxaktiven Einheit entfernt ist. Insbesondere kann die Elektrode das Oxidationsmittel darstellen.

Mit dem Begriff "Reduktionsmittel" wird im Rahmen der vorliegenden Erfindung eine chemische Verbindung (chemische Substanz) bezeichnet, die durch Abgabe von Elektronen an eine andere chemische Verbindung (chemische Substanz, Elektron-Donor, Elektron-Akzeptor) diese andere chemische Verbindung (chemische Substanz, Elektron-Donor, Elektron-Akzeptor) reduziert. Das Reduktionsmittel verhält sich analog zu einem Elektron-Donor, wird aber im Rahmen der vorliegenden Erfindung als Begriff für einen extemen, nicht unmittelbar zur redoxaktiven Einheit gehörigen Elektron-Donor verwendet. "Nicht unmittelbar" bedeutet in diesem Zusammenhang, daß das Reduktionsmittel entweder eine freie redoxaktive Substanz ist, die nicht an das Nukleinsäure-Oligomer gebunden ist, aber mit diesem in Kontakt steht oder daß das Reduktionsmittel kovalent an das Nukleinsäure-Oligomer angebunden ist, jedoch an einer Stelle des Nukleinsäure-Oligomers, die mindestens zwei kovalent verbundene Nukleotide oder wenigstens zwei kovalent verbundene Pyrimidin- oder Purin-Basen von der kovalenten Anbindungstelle der redoxaktiven Einheit entfernt ist. Insbesondere kann die Elektrode das Reduktionsmittel darstellen.

Mit dem Begriff "freie redoxaktive Substanz" wird im Rahmen der vorliegenden Erfindung ein freies, nicht kovalent mit der redoxaktiven Einheit, dem Nukleinsäure-Oligomer oder der leitfähigen Oberfläche verbundenes, aber mit diesen, z. B. über die der modifizierten leitfähigen Oberfläche zugefügte Lösung, in Kontakt stehendes Oxidationsoder Reduktionsmittel bezeichnet, wobei die freie redoxaktive Substanz z. B. ein ungeladenes Molekül, eine beliebiges Salz oder ein redoxaktives Protein oder Enzym (Oxydoreductase) sein kann. Die freie redoxaktive Substanz ist dadurch gekennzeichnet, daß sie den oxidierten Donor (bzw. den reduzierten Akzeptor) der photoinduzierbar redoxaktive Einheit re-reduzieren (bzw. re-oxidieren) kann bzw. daß die freie, redoxaktive Substanz den Donor (bzw. den Akzeptor) der chemisch induzierbar redoxaktiven Einheit reduzieren (bzw. oxidieren) kann. Desweiteren ist die freie redoxaktive Substanz dadurch gekennzeichnet, daß sie bei einem Potential ϕ oxidierbar und reduzierbar ist, wobei ϕ der Bedingung 2,0 V ≥ ϕ ≥ - 2,0 V genügt. Das Potential bezieht sich hierbei auf das freie redoxaktive Molekül in einem geeigneten Lösungsmittel, gemessen gegen Normalwasserstoffelektrode. Im Rahmen der vorliegenden Erfindung ist der Potentialbereich 1,7 V ≥ ϕ ≥ - 1.7 V bevorzugt, wobei der Bereich 1,4 V ≥ ϕ ≥ - 1,2 V besonders bevorzugt ist und der Bereich 0,9 V ≥ ϕ ≥ - 0,7 V, in dem die redoxaktiven Substanzen der Anwendungsbeispiele oxidiert (bzw. reduziert) werden, ganz besonders bevorzugt ist.

Das modifizierte Nukleinsäure-Oligomer ist direkt oder indirekt (über einen Spacer) an eine leitfähige Oberfläche gebunden. Unter dem Begriff "leitfähige Oberfläche" wird jede elektrisch leitfähige Oberfläche beliebiger Dicke verstanden, insbesondere metallische Oberflächen, Oberflächen aus Metallegierungen oder dotierte oder nicht dotierte Halbleiteroberflächen, wobei sämtliche Halbleiter als Reinsubstanzen oder als Gemische Verwendung finden können. Die leitfähige Oberfläche kann im Rahmen der vorliegenden Erfindung alleine oder auf einem beliebigen Trägermaterial, wie z. B. Glas, aufgebracht vorliegen. Im Rahmen der vorliegenden Erfindung wird der Begriff "Elektrode" alternativ zu "leitfähige Oberfläche" gebraucht.

Unter dem Begriff "modifizierte leitfähige Oberfläche" wird eine leitfähige Oberfläche verstanden, die durch Anbindung eines mit einer redoxaktiven Einheit modifizierten Nukleinsäure-Oligomers modifiziert ist.

Gemäß eines weiteren Aspekts betrifft die vorliegende Erfindung ein Verfahren, das die elektrochemische Detektion molekularer Strukturen, insbesondere die elektrochemische Detektion von DNA-/RNA-/PNA-Fragmenten in einer Probenlösung durch sequenzspezifische Nukleinsäure-Oligomer-Hybridisierung ermöglicht. Die Detektion der Hybridisierungsereignisse durch elektrische Signale ist eine einfache und kostengünstige Methode und ermöglicht in einer batteriebetriebenen Variante den Einsatz vor Ort.

Außerdem stellt die vorliegende Erfindung ein photoadressierbares Ausleseverfahren zur Detektion molekularer Strukturen zur Verfügung, unter anderem zur Detektion von Hybridisierungsereignissen auf einem Oligomer-Chip durch z. B. elektrische Signale. Erfindungsgemäß wird unter photoadressierbarem (Oligomer-Chip-) Ausleseverfahren ein Verfahren verstanden, bei dem die Detektion molekularer Strukturen auf ein bestimmtes Test-Site oder eine bestimmte Test-Site-Gruppe innerhalb des Gesamtsystems (des kompletten Oligomer-Chips) begrenzt wird, indem Licht bestimmter oder beliebiger Wellenlänge zur Induktion der Redoxaktivität der photoinduzierbar redoxaktiven Einheit räumlich auf diese Test-Site (-Gruppe) fokussiert (begrenzt) wird.

### Bindung einer redoxaktiven Einheit an ein Nukleinsäure-Oligomer

Voraussetzung für das erfindungsgemäße Verfahren ist die Bindung einer photoinduzierbar redoxaktiven Einheit bzw. einer chemisch induzierbar redoxaktiven Einheit an ein Nukleinsäure-Oligomer.

Als Beispiele einer photoinduzierbar redoxaktiven Einheit seien genannt:
(i) das photosynthetische bakterielle Reaktionszentrum (RC), wie z. B. das RC von *Rhodobacter sphaeroides* mit der schematischen Struktur 1, das RC anderer photosynthetischer Bakterien, wie z. B. das Reaktionszentrum von *Rhodopseudomonas viridis* oder von *Rhodobacter capsulatus,* oder ein Reaktionszentrum der Photosynthese betreibenden Pflanzen, wie z. B das Photosystem 1 oder das Photosystem 2, als Beispiele für ein photoinduzierbar redoxaktives Protein/Enzym.
(ii) Cydophane, also verbrückte Porphyrin-Chinon-Systeme, der allgemeinen Struktur 2 als Beispiel für einen photoinduzierbar redoxaktiven, verknüpften, wenigstens bimolekularen Elektron-Donor/Elektron-Akzeptor-Komplex. Die beiden Spacer-verbrückten, kovalenten Verbindungen ("- - Spacer - -" in Struktur 2) zwischen dem Elektron-Akzeptor (1,4-Benzochinon in der Struktur 2) und dem Elekron-Donor (Metallo-Porphyrin in der Struktur 2) können an beliebigen Stellen des Elektron-Donors und/oder Elektron-Akzeptors angebracht sein. Neben den in der Struktur 2 gezeigten Elektron-Akzeptoren können auch Flavine der allgemeinen Formel 1, Nicotinsäureamide der allgemeinen Formel 2 oder andere Chinone, z. B. solche der allgemeinen Formeln 3 - 8 oder organische bzw. anorganische Elektron-Akzeptoren und außerdem neben den (Metallo-) Porphyrinen der allgemeinen Formel 9 andere Elektron-Donoren, wie z. B. (Metallo-)Chlorophylle der allgemeinen Formel 10 oder (Metallo-) Bakteriochlorophylle der allgemeinen Formel 11 oder andere organische bzw. anorganische Elektron-Donoren verwendet werden. Daneben können auch einfach kovalent (Spacer-)verbrückte Elektron-Donor/Elektron-Akzeptor-Komplexe wie z. B. kovalente Verbindungen einer Substanz gemäß Formel 9 und einer der Substanzen gemäß einer der Formeln 1 - 8, kovalente Verbindungen einer Substanz gemäß Formel 10 und einer der Substanzen gemäß einer der Formeln 1-8 oder kovalente Verbindungen einer Substanz gemäß Formel 11 und einer der Substanzen gemäß einer der Formeln 1 - 8 als photoinduzierbar redoxaktive, verknüpfte, wenigstens bimolekulare Elektron-Donor/Elektron-Akzeptor-Komplexe verwendet werden.
(iii) photoinduzierbar redoxaktive, verknüpfte, wenigstens bimolekularen Elektron-Donor/Elektron-Akzeptor-Komplexe bei denen (einer) der Elektron-Donor(en) und/oder (einer) der Elektron-Akzeptor(en) ein Charge-Transfer-Komplex oder Übergangsmetall-Komplex ist. Beispiele für Übergangsmetall-Komplexe sind [Ru(bipy)₂(py)(im)]²⁺, beliebige andere [Ru(II)(L1)(L2)(L3)(L4)(L5)(L6)]-komplexe, Cr(III)-, Fe(II)-, Os(II)-, oder Co(II)-Komplexe, wobei "bipy" für einen Bispyridyl-Liganden, "py" für einen Pyridyl-Liganden, "im" für einen Immidazol-Liganden und L1 bis L3 für einen beliebigen Liganden steht und auch mehr oder weniger als 6 Liganden an ein Übergangsmetall koordinieren können.

Beispiele einer chemisch induzierbar redoxaktiven Einheit sind der Cytochrom-bc Komplex oder der Cytochrom c₂ Komplex der Photosynthese betreibenden Bakterien (Komplex aus Proteinmatrix und vier eingebetteten Fe-Porphyrin Cofaktoren als Elektron-Donoren und/oder Elektron-Akzeptoren) als Beispiele für ein chemisch induzierbar redoxaktives Protein/Enzym oder, wie unter (ii) und (iii) aufgelistet, geeignet zusammengesetzte Cyclophane bzw. analoge Verbindungen als Beispiele für einen chemisch induzierbar redoxaktiven, verknüpften, wenigstens bimolekularen Elektron-Donor/Elektron-Akzeptor-Komplex.

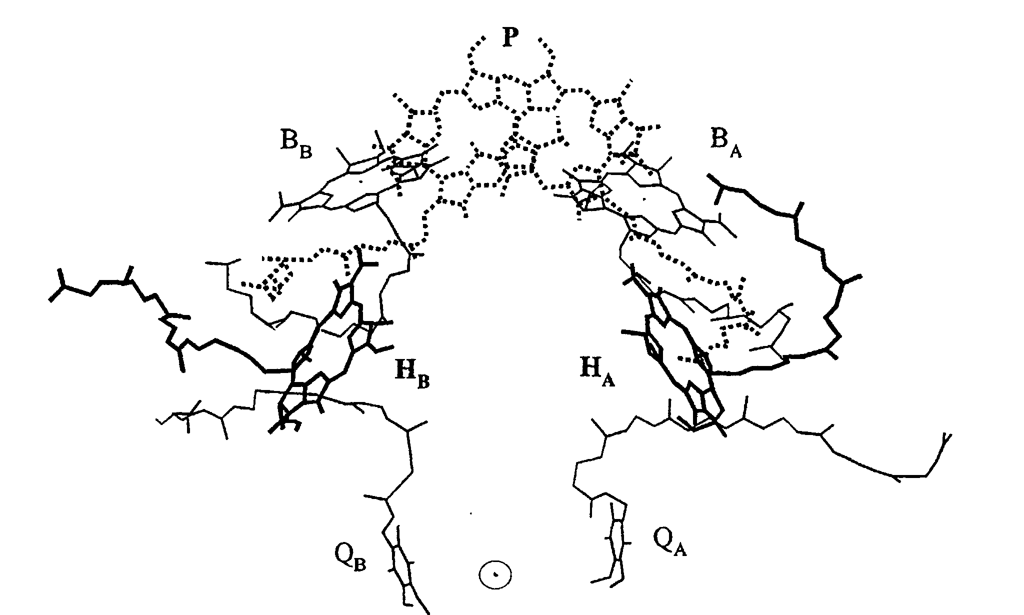

*Struktur 1:* Reaktionszentrum bestehend aus den Cofaktoren P (primärer Donor, ein Bakteriochlorophyll Dimer), B_{A} und B_{B} (Bakteriochlorophyll Monomere), H_{A} und H_{B} (Bakteriopheophytine), Q_{A} und Q_{B} (Ubichinon-50) und den Proteinuntereinheiten L, M, und H (nicht gezeigt), die die Cofaktoren einhüllen.

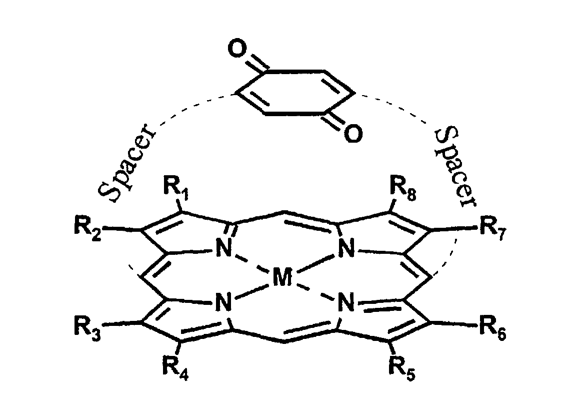

*Struktur* 2: ein Cyclophan; M = z. B. 2H, Mg, Zn, Cu, Ni, Pd, Co, Cd, Mn, Fe, Sn, Pt etc.; R₁ bis R₈, oder Spacer sind unabhängig voneinander beliebige Alkyl-, Alkenyl-, Alkinyl-, Heteroalkyl-, Heteroalkenyl- oder Heteroalkinyl-Substituenten.

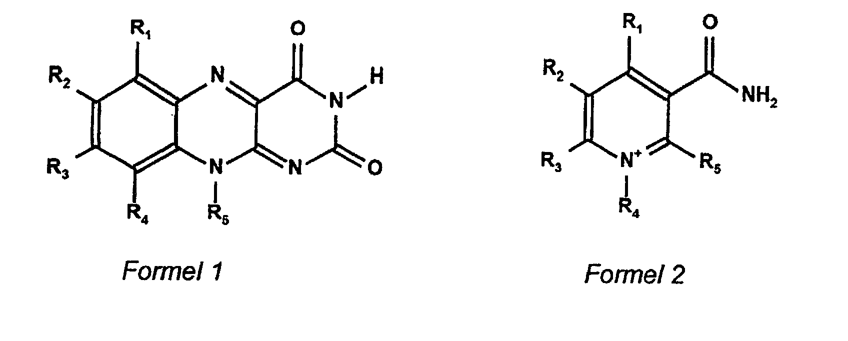

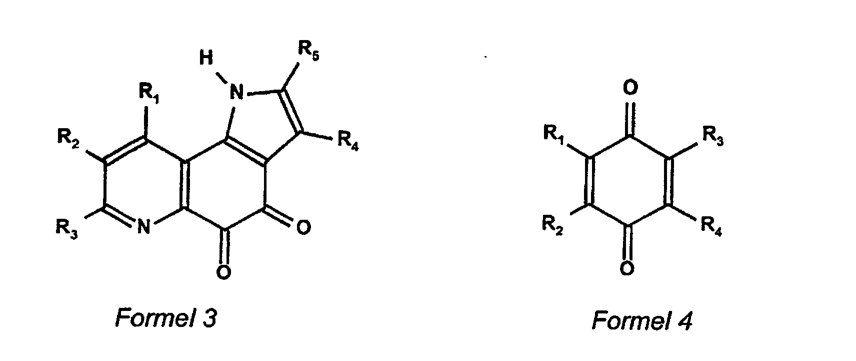

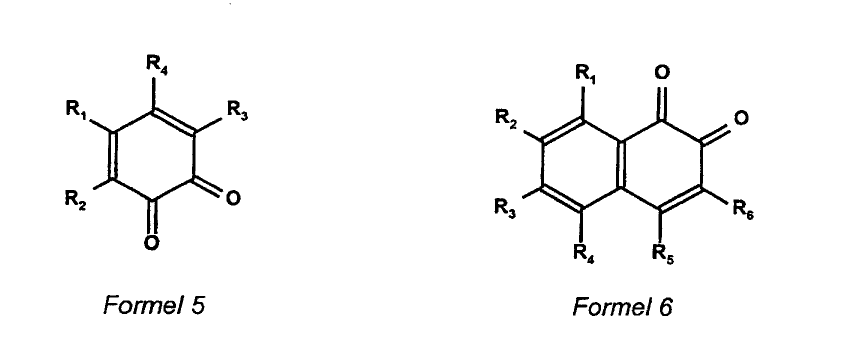

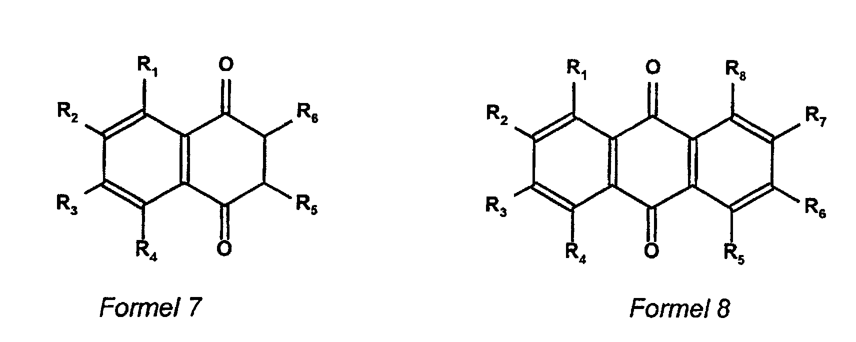

R₁ bis R₈ sind unabhängig voneinander H oder beliebige Alkyl-, Alkenyl-, Alkinyl-, Heteroalkyl-, Heteroalkenyl- oder Heteroalkinyl-Substituenten.

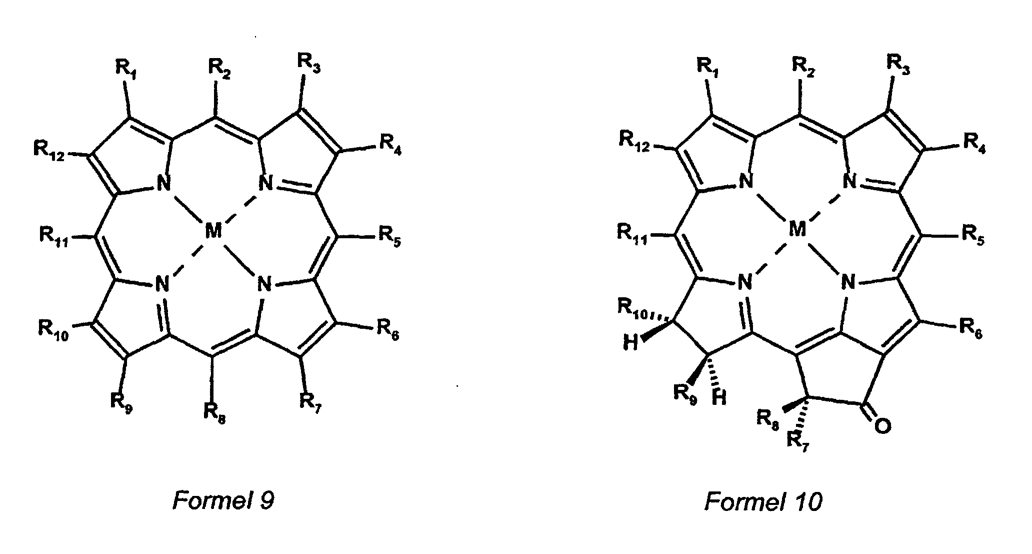

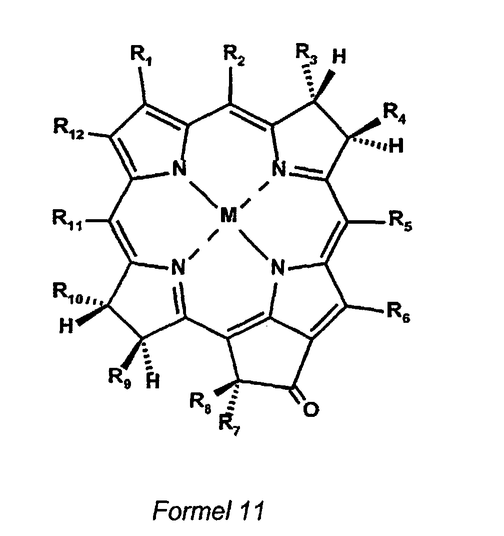

M = 2H, Mg, Zn, Cu, Ni, Pd, Co, Cd, Mn, Fe, Sn, Pt etc.; R₁ bis R₁₂ sind unabhängig voneinander H oder beliebige Alkyl-, Alkenyl-, Alkinyl-, Heteroalkyl-, Heteroalkenyl- oder Heteroalkinyl-Substituenten.

Daneben zeichnet sich die redoxaktive Einheit erfindungsgemäß dadurch aus, daß besagte Einheit an ein ebenfalls kovalent an das Nukleinsäure-Oligomer angebundenes Oxidationsmittel Elektronen abgibt bzw. von einen anderen ebenfalls kovalent an das Oligonukleotid angebundenen Reduktionsmittel Elektronen aufnimmt, wobei dieses Oxidations- oder Reduktionsmittel insbesondere eine elektrisch leitfähige Oberfläche (Elektrode) sein kann und die redoxaktive Einheit durch Anlegen einer äußeren Spannung an dieser Elektrode im elektrochemisch zugänglichen Potentialbereich der Elektrode elektrooxidiert/-reduziert werden kann.

Die redoxaktive Substanz zeichnet sich erfindungsgemäß dadurch aus, daß sie die photoinduzierbar redoxaktive Einheit, nach deren Elektron-Abgabe an ein anderes, von der redoxaktiven Substanz verschiedenes, kovalent an das Oligonukleotid angebundenes Oxidationsmittel re-reduzieren kann (bzw. nach deren Elektron-Aufnahme von einem anderen, von der redoxaktiven Substanz verschiedenen, kovalent an das Oligonukleotid angebundenen Reduktionsmittel re-oxidieren kann). bzw. daß die freie, redoxaktive Substanz den Donor (bzw. den Akzeptor) der chemisch induzierbar redoxaktiven Einheit reduzieren (bzw. oxidieren) kann. Erfindungsgemäß kann dazu jede redoxaktive Substanz verwendet werden, solange sie bei einem Potential ϕ, das der Bedingung 2,0 V ≥ ϕ ≥ - 2,0 V genügt, oxidierbar und reduzierbar ist und das Potential geeignet ist, besagte photoinduzierbar redoxaktive Einheit nach deren Elektron-Abgabe an ein anderes, ebenfalls kovalent an das Nukleinsäure-Oligomer angebundenes Oxidationsmittel zu re-reduzieren (bzw. nach deren Aufnahme eines Elektrons von einen anderen, ebenfalls kovalent an das Nukleinsäure-Oligomer angebundenen Reduktionsmittel zu re-oxidieren) oder besagte chemisch induzierbar redoxaktive Einheit zu reduzieren bzw. zu oxidieren. Das Potential bezieht sich hierbei auf die freie, unmodifizierte, redoxaktive Substanz in einem geeigneten Lösungsmittel, gemessen gegen Normalwasserstoffelektrode. Im Rahmen der vorliegenden Erfindung ist der Potentialbereich 1,7 V ≥ ϕ ≥ - 1.7 V bevorzugt, wobei der Bereich 1,4 V ≥ ϕ ≥ - 1,2 V besonders bevorzugt ist und der Bereich 0,9 V ≥ ϕ ≥ - 0,7 V, in dem die redoxaktiven Substanzen des Anwendungsbeispiels oxidiert (und rereduziert) werden, ganz besonders bevorzugt ist. Geeignet sind, neben den üblichen organischen und anorganischen redoxaktiven Molekülen wie z. B. Hexacyanoferraten, Ferrocenen, Cobaltocenen und Chinonen, vor allem die Ascorbinsäure (oder das Na⁺ Salz davon), [Ru(NH₃)₆]²⁺, oder Cytochrom c₂ (cyt c₂)²⁺, ein frei bewegliches eisenhaltiges Protein, das den oxidierten primären Donor P⁺ in RC von *Rhodobacter sphaeroides* zu P reduziert und dabei selbst zu (cyt c₂)³⁺ oxidiert wird.

In einer bevorzugten Ausführungsform der vorliegenden Erfindung ist der photo- oder chemisch induzierbar redoxaktive, verknüpfte, wenigstens bimolekularen Elektron-Donor/Elektron-Akzeptor-Komplex dergestalt in ein oder mehrere Makromoleküle eingebettet, daß das Makromolekül als elektrisch isolierende Einhüllende des redoxaktiven, verknüpften, wenigstens bimolekularen Elektron-Donor/Elektron-Akzeptor-Komplexes wirkt, indem er die direkte Elektrooxidation/-reduktion des redoxaktiven, verknüpften, wenigstens bimolekularen Elektron-Donor/Elektron-Akzeptor-Komplexes an der Elektrode , z. B. bei direktem Kontakt zwischen Elektrode und redoxaktivem, verknüpften, wenigstens bimolekularen Elektron-Donor/Elektron-Akzeptor-Komplex, verhindert, die indirekte, über doppelsträngiges Nukleinsäure-Oligomer vermittelte Elektrooxidation/-reduktion des Elektron-Donor/Elektron-Akzeptor-Komplexes aber erlaubt. Ein solchen Makromolekül kann z. B. ein maßgeschneidertes Cyclodextrin sein, das durch seine Form eines abgeschnittenen, innen hohlen Kegels ein Cyclophan oder ähnliche Elektron-Donor/Elektron-Akzeptor-Komplex ummantelt.

Erfindungsgemäß wird eine redoxaktive Einheit an ein Nukleinsäure-Oligomer kovalent durch die Reaktion des Nukleinsäure-Oligomers mit der redoxaktiven Einheit oder Teilen davon (siehe auch Abschnitt "Wege zur Ausführung der Erfindung") gebunden. Diese Bindung kann auf vier verschiedene Arten durchgeführt werden:
a) Als reaktive Gruppe zur Bindungsbildung am Nukleinsäure-Oligomer wird eine freie Phosphorsäure-, Zucker-C-3-Hydroxy-, Carbonsäure- oder Amin-Gruppe des Oligonukleotid-Rückgrats, insbesondere eine Gruppe an einem der beiden Enden des Oligonukleotid-Rückgrats, verwendet. Die freien, endständigen Phosphorsäure-, Zucker-C-3-Hydroxy-, Carbonsäure- oder Amin-Gruppen weisen eine erhöhte Reaktivität auf und gehen daher leicht typische Reaktionen wie z. B. Amidbildung mit (primären oder sekundären) Aminogruppen bzw. mit Säuregruppen, Esterbildung mit (primären, sekundären oder tertiären) Alkoholen bzw. mit Säuregruppen, Thioesterbildung mit (primären, sekundären oder tertiären) Thio-Alkoholen bzw. mit Säuregruppen oder die Kondensation von Amin und Aldehyd mit anschließender Reduktion der entstandenen CH=N Bindung zur CH₂-NH Bindung ein. Die zur kovalenten Anbindung der redoxaktiven Einheit nötige Kopplungsgruppe (Säure-, Amin-, Alkohol-, Thioalkohol- oder Aldehydfunktion) ist entweder natürlicherweise an der redoxaktiven Einheit vorhanden oder wird durch chemische Modifikation der redoxaktiven Einheit erhalten. Die Anbindung der redoxaktiven Einheit kann komplett oder in Teilen der Einheit mit anschließender Vervollständigung der redoxaktiven Einheit erfolgen (siehe unten).
b) Das Nukleinsäure-Oligomer ist über einen kovalent angebundenen Molekülteil (Spacer) beliebiger Zusammensetzung und Kettenlänge (längste durchgehende Kette von aneinander gebundenen Atomen), insbesondere der Kettenlänge 1 bis 14, am Oligonukleotid-Rückgrat bzw. an einer Base mit einer reaktiven Gruppe modifiziert. Die Modifikation erfolgt bevorzugt an einem der Enden des Oligonukleotid-Rückgrats bzw. an einer terminalen Base. Als Spacer kann z.B. ein Alkyl-, Alkenyl-, Alkinyl-, Heteroalkyl-, Heteroalkenyl- oder Heteroalkinylsubstituent verwendet werden. Mögliche einfache Reaktionen zur Ausbildung der kovalenten Bindung zwischen redoxaktiver Einheit und des so modifizierten Nukleinsäure-Oligomers sind wie unter a) beschrieben, die Amidbildung aus Säure- und Amino-Gruppe, die Esterbildung aus Säure- und Alkohol-Gruppe, die Thioesterbildung aus Säure- und Thio-Alkohol-Gruppe oder die Kondensation von Aldehyd und Amin mit anschließender Reduktion der entstandenen CH=N Bindung zur CH₂-NH Bindung. Die Anbindung der redoxaktiven Einheit kann komplett oder in Teilen der redoxaktiven Einheit mit anschließender Vervollständigung der Einheit erfolgen (siehe unten).
c) Bei der Synthese des Nukleinsäure-Oligomers wird eine terminale Base durch die redoxaktive Einheit ersetzt. Diese Anbindung der redoxaktiven Einheit kann komplett oder in Teilen der Einheit mit anschließender Vervollständigung der redoxaktiven Einheit erfolgen (siehe unten).
d) Bei der Verwendung eines verknüpften (wenigstens bimolekularen) Elekton-Donor-/Elektron-Akzeptor-Komplexes als redoxaktiver Einheit wird der Elektron-Akzeptor (oder -Donor) in einer ersten kovalenten Modifikation, wie unter b) oder c) in diesem Abschnitt beschrieben, an eine oder statt einer terminalen Base an das Nukleinsäure-Oligomer gebunden und anschließend in einer zweiten kovalenten Modifikation der Elektron-Donor (oder -Akzeptor), wie unter a) in diesem Abschnitt beschrieben, am selben Ende des Nukleinsäure-Oligomer-Rückgrats an eine reaktive Gruppe des Rückgrats oder an eine reaktive Gruppe des Akzeptors (bzw. des Donors) gebunden. Bei Verwendung eines kovalent verknüpften, tri- oder höhermolekularen Elekton-Donor-/Elektron-Akzeptor-Komplexes kann, statt des Elektron-Akzeptors (oder -Donors), auch ein beliebiger Teil des Elekton-Donor-/Elektron-Akzeptor-Komplexes in der ersten kovalenten Modifikation verwendet und in einer zweiten bzw. weiteren kovalenten Modifikation(en) komplettiert werden.

Erfindungsgemäß kann die Bindung der redoxaktiven Einheit an das Nukleinsäure-Oligomer ganz oder in Teilen vor oder nach der Bindung des Nukleinsäure-Oligomers an die leitfähige Oberfläche erfolgen. So kann im Falle eines redoxaktiven Proteins/Enzyms aus Apoprotein und Cofaktor(en) statt der kompletten redoxaktiven Einheit auch nur das Apoprotein, das Apoprotein und ein Teil der Cofaktoren oder ein oder mehrere Cofaktoren angebunden sein und die redoxaktive Einheit wird durch anschließende Rekonstitution mit den noch fehlenden Teilen komplettiert. Bei der Verwendung eines verknüpften (wenigstens bimolekularen) Elekton-Donor-/Elektron-Akzeptor-Komplexes als redoxaktiver Einheit wird der Elektron-Akzeptor (oder -Donor) in einer ersten kovalenten Modifikation, wie unter b) oder c) in diesem Abschnitt beschrieben, an oder statt einer terminalen Base an das Nukleinsäure-Oligomer gebunden und anschließend in einer zweiten kovalenten Modifikation der Elektron-Donor (oder -Akzeptor), wie unter a) in diesem Abschnitt beschrieben, am selben Ende des Nukleinsäure-Oligomer-Rückgrats an eine reaktive Gruppe des Rückgrats gebunden. Bei Verwendung eines kovalent verknüpften, tri- oder höhermolekularen Elekton-Donor-/Elektron-Akzeptor-Komplexes kann, statt des Elektron-Akzeptors (oder -Donors), auch ein beliebiger Teil des Elekton-Donor-/Elektron-Akzeptor-Komplexes in der ersten kovalenten Modifikation verwendet und in der zweiten kovalenten Modifikation komplettiert werden. Diese Modifikationen können vor oder nach der Bindung des Nukleinsäure-Oligomers an die leitfähige Oberfläche erfolgen.

Bei mehreren verschiedenen Nukleinsäure-Oligomer-Kombinationen (Test-Sites) auf einer gemeinsamen Oberfläche ist es vorteilhaft, die (kovalente) Anbindung der redoxaktiven Einheit an die Nukleinsäure-Oligomere durch geeignete Wahl der reaktiven Gruppe an den freien Nukleinsäure-Oligomerenden der verschiedenen Test-Sites für die gesamte Oberfläche zu vereinheitlichen, wenn die redoxaktive Einheit nach Immobilisierung des Nukleinsäure-Oligomers an der Oberfläche angebunden werden soll.

Bei Verwendung von redoxaktiven Proteinen/Enzymen als redoxaktiver Einheit, kann die kovalente Anbindung des Nukleinsäure-Oligomers an eine beliebige, natürlicherweise vorhandene oder durch Modifikation angebrachte, reaktive Gruppe des Proteins erfolgen oder - in dem Falle, daß das redoxaktive Protein/Enzym aus Apoprotein und Cofaktor(en) besteht - an eine beliebige, natürlicherweise vorhandene oder durch Modifikation angebrachte, reaktive Gruppe eines (beliebigen) Cofaktors. Im Rahmen der vorliegenden Erfindung ist die kovalente Anbindung an eine beliebige, natürlicherweise vorhandene oder durch Modifikation angebrachte, reaktive Gruppe eines (beliebigen) Cofaktors des Proteins bevorzugt. Ohne an mechanistische Details gebunden sein zu wollen, ist bei mehreren Cofaktoren derjenige besonders bevorzugt, der Elektronen an ein externes, ebenfalls kovalent an das Nukleinsäure-Oligomer angebundenes Oxidationsmittel abgeben oder von einem externen, ebenfalls kovalent an das Nukleinsäure-Oligomer angebundenen Reduktionsmittel aufnehmen kann (siehe auch Abschnitt "Verfahren zur amperometrischen Detektion von Nukleinsäure-Oligomer-Hybriden").

### Die leitfähige Oberfläche

Unter dem Begriff "leitfähige Oberfläche" wird erfindungsgemäß jeder Träger mit einer elektrisch leitfähigen Oberfläche beliebiger Dicke verstanden, insbesondere Oberflächen aus Platin, Palladium, Gold, Cadmium, Quecksilber, Nickel, Zink, Kohlenstoff, Silber, Kupfer, Eisen, Blei, Aluminium und Mangan.

Daneben können auch beliebige dotierte oder nicht dotierte Halbleiteroberflächen beliebiger Dicke verwendet werden. Sämtliche Halbleiter können als Reinsubstanzen oder als Gemische Verwendung finden. Als nicht einschränkend gemeinte Beispiele seien an dieser Stelle Kohlenstoff, Silizium, Germanium, α-Zinn, Cu(I)- und Ag(I)-Halogenide beliebiger Kristallstruktur genannt. Geeignet sind ebenfalls sämtliche binären Verbindungen beliebiger Zusammensetzung und beliebiger Struktur aus den Elementen der Gruppen 14 und 16, den Elementen der Gruppen 13 und 15, sowie den Elementen der Gruppen 15 und 16. Daneben können ternäre Verbindungen beliebiger Zusammensetzung und beliebiger Struktur aus den Elementen der Gruppen 11, 13 und 16 oder den Elementen der Gruppen 12, 13 und 16 verwendet werden. Die Bezeichnungen der Gruppen des Periodensystems der Elemente beziehen sich auf die IUPAC-Empfehlung von 1985.

### Bindung eines Nukleinsäure-Oligomers an die leitfähige Oberfläche

Erfindungsgemäß wird ein Nukleinsäure-Oligomer direkt oder über einen Linker/Spacer mit den Oberflächenatomen oder -molekülen einer leitfähigen Oberfläche der oben beschriebenen Art verknüpft. Diese Bindung kann auf drei verschiedene Arten durchgeführt werden:
a) Die Oberfläche wird so modifiziert, daß eine reaktive Molekül-Gruppe zugänglich ist. Dies kann durch direkte Derivatisierung der Oberflächenmoleküle, z. B. durch naßchemische oder elektrochemische Oxidation/Reduktion geschehen. So kann z. B. die Oberfläche von Graphitelektroden durch Oxidation naßchemisch mit Aldehydoder Carbonsäure-Gruppen versehen werden. Elektrochemisch besteht z. B. die Möglichkeit durch Reduktion in Gegenwart von Aryl-Diazoniumsalzen das entsprechende (funktionalisierte, also mit einer reaktiven Gruppe versehene) Aryl-Radikal oder durch Oxidation in Gegenwart von R'CO₂H das (funktionalisierte) R'-Radikal auf der Graphit-Elektrodenoberfläche anzukoppeln. Ein Beispiel der direkten Modifikation von Halbleiteroberflächen ist die Derivatisierung von Siliziumoberflächen zu reaktiven Silanolen, d. h. Silizium-Träger mit Si-OR" Gruppen an der Oberfläche, wobei R" ebenso wie R' einen beliebigen, funktionalisierten, organischen Rest darstellt (z.B. Alkyl-, Alkenyl-, Alkinyl-, Heteroalkyl-, Heteroalkenyl- oder Heteroalkinylsubstituent). Alternativ kann die gesamte Oberfläche durch die kovalente Anbindung einer reaktiven Gruppe eines bifunktionalen Linkers modifiziert werden, so daß auf der Oberfläche eine monomolekulare Schicht beliebiger Moleküle entsteht, die, bevorzugt endständig, eine reaktive Gruppe enthalten. Unter dem Begriff "bifunktionaler Linker" wird jedes Molekül beliebiger Kettenlänge, insbesondere der Kettenlängen 2-14, mit zwei gleichen (homo-bifunktional) oder zwei verschiedenen (hetero-bifunktional) reaktiven Molekül-Gruppen verstanden.
   Sollen mehrere verschiedene Test-Sites auf der Oberfläche durch Ausnutzen der Methodik der Photolithographie gebildet werden, so ist mindestens eine der reaktiven Gruppen des homo- oder hetereo-bifunktionalen Linkers eine photoinduzierbar reaktive Gruppe, d. h. eine erst durch Lichteinstrahlung bestimmter oder beliebiger Wellenlänge reaktiv werdende Gruppe. Dieser Linker wird so aufgebracht, daß die/eine photoaktivierbare reaktive Gruppe nach der kovalenten Anbindung des Linkers auf der Oberfläche zur Verfügung steht. An die so modifizierte Oberfläche werden die Nukleinsäure-Oligomere kovalent angebunden, wobei diese selbst über einen Spacer beliebiger Zusammensetzung und Kettenlänge, insbesondere der Kettenlänge 1 - 14, mit einer reaktiven Gruppe modifiziert sind, bevorzugt in der Nähe eines Endes des Nukleinsäure-Oligomers. Bei der reaktiven Gruppe des Oligonukleotids handelt es sich um Gruppen, die direkt (oder indirekt) mit der modifizierten Oberfläche unter Ausbildung einer kovalenten Bindung reagieren. Daneben kann an die Nukleinsäure-Oligomere in der Nähe ihres zweiten Endes eine weitere reaktive Gruppe gebunden sein, wobei diese reaktive Gruppe wiederum, wie oben beschrieben, direkt oder über einen Spacer beliebiger Zusammensetzung und Kettenlänge, insbesondere der Kettenlänge 1 - 14, angebunden ist. Desweiteren kann die redoxaktive Einheit (komplett oder Bestandteile davon), alternativ zu dieser weiteren reaktiven Gruppe, an diesem zweiten Ende des Nukleinsäure-Oligomers angebunden sein.
b) Das Nukleinsäure-Oligomer, das auf die leitfähige Oberfläche aufgebracht werden soll, ist über einen kovalent angebundenen Spacer beliebiger Zusammensetzung und Kettenlänge, insbesondere der Kettenlänge 1 - 14, mit einer oder mehreren reaktiven Gruppen modifiziert, wobei sich die reaktive Gruppen bevorzugt in der Nähe eines Endes des Nukleinsäure-Oligomers befindet. Bei den reaktiven Gruppen handelt es sich um Gruppen, die direkt mit der unmodifizierten Oberfläche reagieren können. Beispiele hierfür sind: (i) Thiol- (HS-) oder Disulfid- (S-S-) derivatisierte Nukleinsäure-Oligomere der allgemeinen Formel (n x HS-Spacer)-oligo, (n x R-S-S-Spacer)-oligo oder oligo-Spacer-S-S-Spacer-oligo, die mit einer Goldoberfläche unter Ausbildung von Gold-Schwefelbindungen reagieren oder (ii) Amine, die sich durch Chemi- oder Physikosorption an Platin- oder Silizium-Oberflächen anlagern. Daneben kann an die Nukleinsäure-Oligomere in der Nähe ihres zweiten Endes eine weitere reaktive Gruppe gebunden sein, wobei diese reaktive Gruppe wiederum, wie oben beschrieben, direkt oder über einen Spacer beliebiger Zusammensetzung und Kettenlänge, insbesondere der Kettenlänge 1 - 14, angebunden ist. Desweiteren kann die photinduzierbar redoxaktive Einheit (komplett oder Bestandteile davon) alternativ zu dieser weiteren reaktiven Gruppe, an diesem zweiten Ende des Oligonukleotids angebunden sein. Insbesondere Nukleinsäure-Oligomere die mit mehreren Spacer-verbrückten Thiol oder Disulfidbrücken modifiziert sind ((n x HS-Spacer)-oligo bzw. (n x R-S-S-Spacer)-oligo) haben den Vorteil, daß solche Nukleinsäure-Oligomere unter einem bestimmten Anstellwinkel gegen die leitfähige Oberfläche (Winkel zwischen der Oberflächennormalen und der Helixachse eines doppelsträngigen helikalen Nukleinsäure-Oligomers bzw. zwischen der Oberflächennormalen und der Achse senkrecht zu den Basenpaaren eines doppelsträngigen nicht-helikalen Nukleinsäure-Oligomers) aufgebracht werden können, wenn die die Thiol- bzw. Disulfid-Funktionen an das Nukleinsäure-Oligomer anbindenden Spacer, von einem Ende der Nukleinsäure her betrachtet, eine zunehmende bzw. abnehmende Kettenlänge besitzen.
c) Als reaktive Gruppe am Sonden-Nukleinsäure-Oligomer werden die Phosphorsäure-, Zucker-C-3-Hydroxy-, Carbonsäure- oder Amin-Gruppen des Oligonukleotid-Rückgrats, insbesondere endständige Gruppen, verwendet. Die Phosphorsäure-, Zucker-C-3-Hydroxy-, Carbonsäure- oder Amin-Gruppen weisen eine erhöhte Reaktivität auf und gehen daher leicht typische Reaktionen wie z. B. Amidbildung mit (primären oder sekundären) Amino- bzw. Säuregruppen, Esterbildung mit (primären, sekundären oder tertiären) Alkoholen bzw. Säuregruppen, Thioesterbildung mit (primären, sekundären oder tertiären) Thio-Alkoholen bzw. Säuregruppen oder die Kondensation von Amin und Aldehyd mit anschließender Reduktion der entstandenen CH=N Bindung zur CH₂-NH Bindung ein. Die nötige Kopplungs-Gruppe zur kovalenten Anbindung an die Phosphorsäure-, Zucker-C-3-Hydroxy-, Carbonsäure- oder Amin-Gruppe ist in diesem Fall ein Teil der Oberflächenderivatisierung mit einer (monomolekularen) Schicht beliebiger Moleküllänge, wie unter a) in diesem Abschnitt beschrieben, oder die Phosphorsäure-, Zucker-C-3-Hydroxy-, Carbonsäure- oder Amin-Gruppe kann direkt mit der unmodifizierten Oberfläche reagieren, wie unter b) in diesem Abschnitt beschrieben. Daneben kann an die Oligonukleotide in der Nähe ihres zweiten Endes eine weitere reaktive Gruppe gebunden sein, wobei diese reaktive Gruppe wiederum, wie oben beschrieben, direkt oder über einen Spacer beliebiger Zusammensetzung und Kettenlänge, insbesondere der Kettenlänge 1 - 14, angebunden ist. Desweiteren kann die redoxaktive Einheit (komplett oder Bestandteile davon), alternativ zu dieser weiteren reaktiven Gruppe, an diesem zweiten Ende des Nukleinsäure-Oligomers angebunden sein.

Die Bindung des Nukleinsäure-Oligomers an die leitfähige Oberfläche kann vor oder nach der Anbindung der redoxaktive Einheit an das Nukleinsäure-Oligomer erfolgen. Im Falle eines redoxaktiven Proteins/Enzyms aus Apoprotein und Cofaktor(en) kann statt der kompletten redoxaktiven Einheit auch nur das Apoprotein, das Apoprotein mit einem Teil der Cofaktoren oder ein oder mehrere der Cofaktor angebunden sein und die redoxaktive Einheit wird durch anschließende Rekonstitution mit den noch fehlenden Teilen komplettiert. Bei der Verwendung eines verknüpften (wenigstens bimolekularen) Elekton-Donor-/Elektron-Akzeptor-Komplexes als redoxaktive Einheit kann der Elektron-Akzeptor ( bzw. -Donor), wie unter b) oder c) im Abschnitt "Bindung einer redoxaktiven Einheit an ein Nukleinsäure-Oligomer" beschrieben, an eine oder statt einer terminalen Base an das Nukleinsäure-Oligomer gebunden sein und der Elektron-Donor (bzw. -Akzeptor) durch anschließende kovalente Anbindung an eine reaktive Gruppe des Elektron-Akzeptors (oder -Donors) angebunden werden oder, wie unter a) im Abschnitt "Bindung einer redoxaktiven Einheit an ein Nukleinsäure-Oligomer" beschrieben, durch anschließende Anbindung an eine terminale reaktive Gruppe des Nukleinsäure-Oligomer-Rückgrats am selben Ende (siehe auch den Abschnitt "Wege zur Ausführung der Erfindung"). Alternativ kann die Bindung des Nukleinsäure-Oligomers an die leitfähige Oberfläche vor oder nach Anbinden des mit einer reaktiven Gruppe versehenen Spacers zur Bindung der redoxaktiven Einheit erfolgen. Die Bindung des bereits modifizierten Nukleinsäure-Oligomers an die leitfähige Oberfläche, d. h. die Bindung an die Oberfläche nach der Anbindung der redoxaktiven Einheit an das Nukleinsäure-Oligomer bzw. nach der Anbindung von Teilen der redoxaktiven Einheit oder nach Anbinden des mit einer reaktiven Gruppe versehenen Spacers zur Bindung der redoxaktiven Einheit, erfolgt ebenfalls wie unter a) bis c) in diesem Abschnitt beschrieben.

Bei der Herstellung der Test-Sites muß bei der Anbindung der Einzelstrang-Nukleinsäure-Oligomere an die Oberfläche darauf geachtet werden, daß zwischen den einzelnen Nukleinsäure-Oligomeren ein genügend großer Abstand verbleibt, um zum einen den für eine Hybridisierung mit dem Target-Nukleinsäure-Oligomer nötigen Freiraum und zum anderen den für die Anbindung der redoxaktiven Einheit nötigen Freiraum zur Verfügung zu stellen. Dazu bieten sich insbesondere drei verschiedene Vorgehensweisen (und Kombinationen daraus) an:
1.) Herstellung einer modifizierten Oberfläche durch Anbindung eines hybridisierten Nukleinsäure-Oligomers, also eine Oberflächen-Derivatisierung mit hybridisiertem Sonden-Nukleinsäure-Oligomer statt mit Einzelstrang-Sonden-Oligonukleotid. Der zur Hybridisierung verwendete Nukleinsäure-Oligomer-Strang ist unmodifiziert (die Oberflächenanbindung wird durchgeführt wie unter a) - c) in diesem Abschnitt beschrieben). Anschließend wird der hybridisierte Nukleinsäure-Oligomer-Doppelstrang thermisch dehybridisiert, wodurch eine mit Einzelstrang- Nukleinsäure-Oligomer modifizierte Oberfläche mit größerem Abstand zwischen den Sonden-Nukleinsäure-Oligomeren hergestellt wird.
2.) Herstellung einer modifizierten Oberfläche durch Anbindung eines Einzelstrangoder Doppelstrang-Nukleinsäure-Oligomers, wobei während der Oberflächen-Derivatisierung ein geeigneter monofunktionaler Linker zugesetzt wird, der neben dem Einzelstrang- oder Doppelstrang-Nukleinsäure-Oligomer ebenfalls an die Oberfläche gebunden wird (die Oberflächenanbindung wird durchgeführt wie unter a) - c) in diesem Abschnitt beschrieben). Erfindungsgemäß hat der monofunktionale Linker eine Kettenlänge, die der Kettenlänge des Spacers zwischen der Oberfläche und dem Nukleinsäure-Oligomer identisch ist oder um maximal vier Kettenatome abweicht. Bei der Verwendung von Doppelstrang-Nukleinsäure-Oligomer zur Oberflächen-Derivatisierung wird der Nukleinsäure-Oligomer-Doppelstrang nach der gemeinsamen Anbindung des Doppelstrang- Nukleinsäure-Oligomers und des Linkers an die Oberfläche thermisch dehybridisiert. Durch die gleichzeitige Anbindung eines Linkers an die Oberfläche wird der Abstand zwischen den ebenfalls an die Oberfläche gebundenen Einzel- oder Doppelstrang-Nukleinsäure-Oligomeren vergrößert. Im Falle der Verwendung von Doppelstrang-Nukleinsäure-Oligomer wird dieser Effekt durch die anschließende thermische Dehybridisierung noch verstärkt.
3.) Herstellung einer modifizierten Oberfläche durch Anbindung eines Einzelstrangoder Doppelstrang-Oligonukleotids, an das die redoxaktive Einheit bereits angebunden ist, wobei die redoxaktive Einheit einen Durchmesser von größer als 30 Å aufweist. Bei der Verwendung von Doppelstrang-Oligonukleotid wird der Oligonukleotid-Doppelstrang nach der Anbindung des Doppelstrang-Oligonukleotids an die Oberfläche thermisch dehybridisiert.

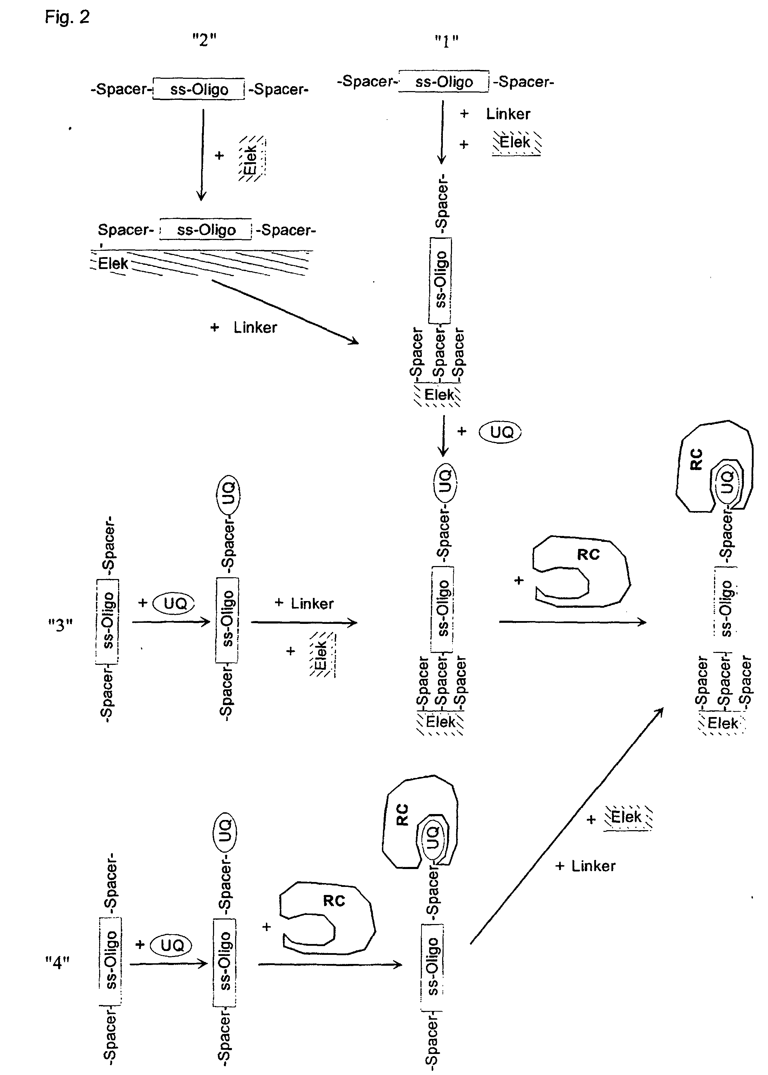

Im Bezug auf die einzelnen Schritte zur "Bindung einer redoxaktiven Einheit an ein Nukleinsäure-Oligomer" als auch zur "Bindung eines Oligonukleotids an die leitfähige Oberfläche" sei darauf verwiesen, daß im Abschnitt "Wege zur Ausführung der Erfindung" die verschiedenen Kombinationsmöglichkeiten der einzelnen Schritte, die zum selben Endergebnis führen, an einem Beispiel demonstriert sind (Figur 2).

### Verfahren zur elektrochemischen Detektion von Nukleinsäure-Oligomer-Hybriden

Vorteilhafterweise werden gemäß dem Verfahren zur elektrochemischen Detektion von Nukleinsäure-Oligomer-Hybriden mehrere Sonden-Nukleinsäure-Oligomere unterschiedlicher Sequenz, idealerweise alle nötigen Kombinationen des Nukleinsäure-Oligomers, auf einem Oligomer (DNA) -Chip aufgebracht, um die Sequenz eines beliebigen Target-Nukleinsäure-Oligomers oder einer (fragmentierten) Target-DNA zu detektieren bzw. um Mutationen im Target aufzuspüren und sequenzspezifisch nachzuweisen. Dazu werden auf einer leitfähigen Oberfläche die Oberflächenatome oder -moleküle eines definierten Bereichs (einer Test-Site) mit DNA-/RNA-/PNA-Nukleinsäure-Oligomeren bekannter, aber beliebiger Sequenz, wie oben beschrieben, verknüpft. In einer allgemeinsten Ausführungsform kann aber der DNA-Chip auch mit einem einzigen Sonden-Oligonukleotid derivatisiert werden. Als Sonden- Nukleinsäure-Oligomere werden Nukleinsäure-Oligomere (z. B. DNA-, RNA- oder PNA-Fragmente) der Basenlänge 3 bis 50, bevorzugt der Länge 5 bis 30, besonders bevorzugt der Länge 8 bis 25 verwendet. Erfindungsgemäß wird oder ist an die Sonden-Nukleinsäure-Oligomere, wie nachfolgend beschrieben, eine redoxaktive Einheit gebunden.

Die Modifikation der Sonden-Nukleinsäure-Oligomere mit einer redoxaktiven Einheit kann komplett oder in Bestandteilen der redoxaktiven Einheit entweder vor oder nach der Bindung des Sonden-Oligonukleotids an die leitfähige Oberfläche erfolgen. Die verschiedenen Kombinationsmöglichkeiten der einzelnen Schritte (Reaktionssequenzen), sind mit Hilfe der Figur 2 am Beispiel einer über ein Sonden-Oligonukleotid an eine Elektrode gebundenen redoxaktiven Einheit im Abschnitt "Wege zur Ausführung der Erfindung" demonstriert.

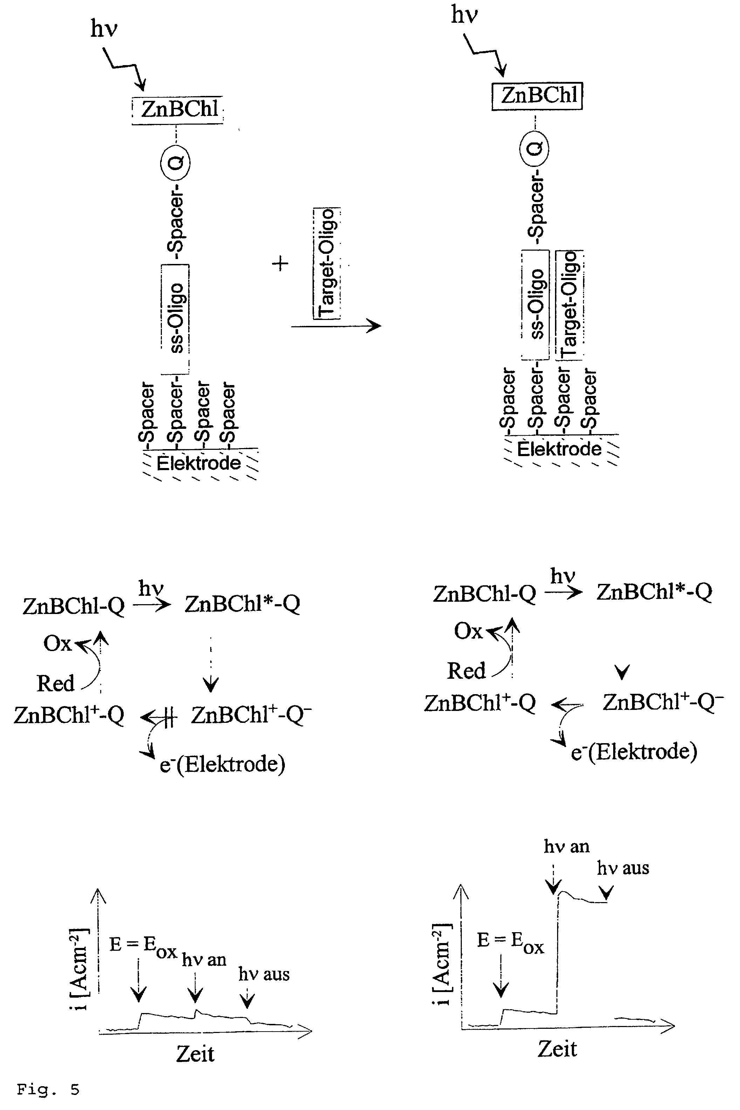

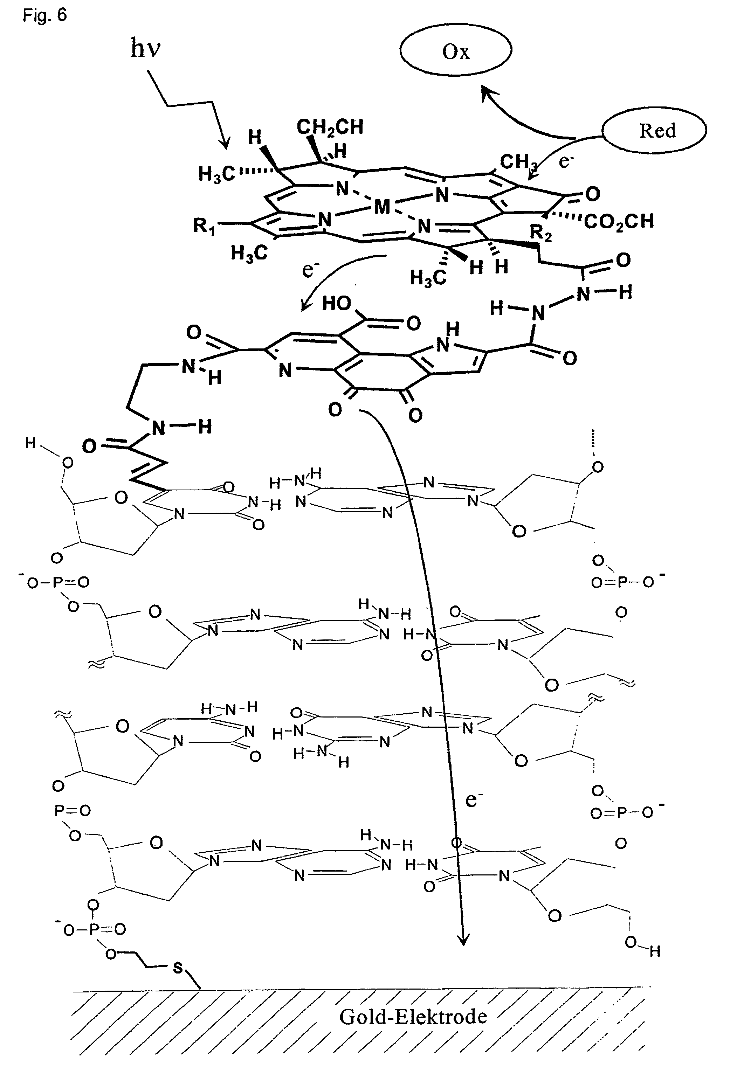

Unabhängig von der jeweiligen Reaktionssequenz entsteht ein Oberflächen-Hybrid der allgemeinen Struktur Elek-Spacer-ss-oligo-Spacer-Einheit, wobei "Einheit" repräsentativ für die photoinduzierbar redoxaktive Einheit bzw. chemisch induzierbar redoxaktive Einheit steht. Die Verbrückungen können natürlich auch ohne Spacer oder mit nur einem Spacer (Elek-ss-oligo-Spacer-Einheit bzw. Elek-Spacer-ss-oligo-Einheit) durchgeführt werden. Im Beispiel der Figur 2 ist die Einheit eine photoinduzierbar redoxaktive Einheit, nämlich das Reaktionszentrum (RC) der Photosynthese betreibenden Bakterien des Stammes *Rhodobacter sphaeroides,* ein photoinduzierbar redoxaktives Protein bestehend aus Apoprotein und Cofaktoren. Im Beispiel der Figur 2, 3 und 4 ist das RC über seinen Cofaktor Ubichinon-50 (UQ) in der sogenannten Q_{A}-Protein-Bindungstasche des RCs kovalent mit dem Nukleinsäure-Oligomer verbunden. Das RC bildet mit dem Cofaktor Ubichinon-50 in der Q_{A}-Bindungstasche einen 1:1 Komplex, wobei das Ubichinon-50 in der beschriebenen Weise kovalent an das Nukleinsäure-Oligomer gebunden ist. Im Beispiel der Figur 5 und 6 ist die Einheit ein photoinduzierbar redoxaktiver, verknüpfter, wenigstens bimolekularer Elektron-Donor-/Elektron-Akzeptor-Komplex, nämlich ein kovalent verknüpfter Zn-Bakteriochlorophyll-Chinon-Komplex, der über das Chinon, dem Elektron-Akzeptor-Molekül des Komplexes, kovalent (über einen Spacer) mit dem Nukleinsäure-Oligomer verbunden ist.

Die elektrochemische Kommunikation zwischen der (leitfähigen) Oberfläche und der über ein Einzelstrang-Oligonukleotid verbrückten redoxaktiven Einheit ("Einheit") in der allgemeinen Struktur Elek-Spacer-ss-oligo-Spacer-Einheit ist schwach oder gar nicht vorhanden.

In einem nächsten Schritt werden die Test-Sites mit der zu untersuchenden Nukleinsäure-Oligomer-Lösung (Target) in Kontakt gebracht. Dabei kommt es nur in dem Fall zur Hybridisierung, in dem die Lösung Nukleinsäure-Oligomer-Stränge enthält, die zu den an die leitfähige Oberfläche gebundenen Sonden-Nukleinsäure-Oligomeren komplementär, oder zumindest in weiten Bereichen komplementär sind. Im Falle der Hybridisierung zwischen Sonden- und Target-Nukleinsäure-Oligomer kommt es zu einer verstärkten Leitfähigkeit zwischen der Oberfläche und der redoxaktiven Einheit, da diese nunmehr über das aus einem Doppelstrang bestehende Nukleinsäure-Oligomer verbrückt ist. Figur 3 zeigt dies schematisch am Beispiel der Elek-Spacer-ss-oligo-Spacer-UQ(RC). In Figur 4 ist die Sequenz der Elektron-Transfer-Schritte in Elek-Spacer-ds-oligo-Spacer-UQ(RC) im Detail gezeigt, während Figur 5 das Beispiel Elek-Spacer-ss-oligo-Spacer-Q-ZnBChl schematisch zeigt und Figur 6 die Sequenz der Elektron-Transfer-Schritte in Elek-Spacer-ds-oligo-Spacer-Q-ZnBChl im Detail darstellt.

Aufgrund der Hybridisierung von Sonden-Nukleinsäure-Oligomer und dem dazu komplementären Nukleinsäure-Oligomer-Strang (Target) verändert sich die elektrische Kommunikation zwischen der (leitfähigen) Oberfläche und der photoinduzierbar redoxaktiven Einheit. Somit kann ein sequenzspezifisches Hybridisierungsereignis durch elektrochemische Verfahren wie z. B. Cyclovoltametrie, Amperometrie oder Leitfähigkeitsmessungen detektiert werden.

Bei der Cyclovoltametrie wird das Potential einer stationären Arbeitselektrode zeitabhängig linear verändert. Ausgehend von einem Potential bei dem keine Elektrooxidation oder - reduktion stattfindet, wird das Potential solange verändert bis die redoxaktive Substanz oxidiert oder reduziert wird (also Strom fließt). Nach Durchlaufen des Oxidations- bzw. Reduktionsvorgangs, der in der Strom/Spannungskurve einen zunächst ansteigenden Strom, dann einen Maximalstrom (Peak) und schließlich einen allmählich abfallenden Strom erzeugt, wird die Richtung des Potentialvorschubs umgekehrt. Im Rücklauf wird dann das Verhalten der Produkte der Elektrooxidation oder -reduktion aufgezeichnet.

Eine alternative elektrische Detektionsmethode, die Amperometrie, wird dadurch ermöglicht, daß die redoxaktive Einheit durch Anlegen eines geeigneten, konstant gehaltenen Elektrodenpotentials zwar elektrooxidiert (etektroreduziert) werden kann, die Rereduktion (Reoxidation) der redoxaktiven Einheit in den ursprünglichen Zustand aber nicht wie in der Cyclovoltametrie durch Änderung des Elektrodenpotentials erfolgt, sondern durch ein der Targetlösung zugesetztes geeignetes Reduktionsmittel (Oxidationsmittel), der "redoxaktiven Substanz", wodurch der Stromkreis des Gesamtsystems geschlossen wird. Solange solches Reduktionsmittel (Oxidationsmittel) vorhanden ist bzw. solange das verbrauchte Reduktionsmittel (Oxidationsmittel) an der Gegenelektrode rereduziert (reoxidiert) wird, fließt Strom, der amperometrisch detektiert werden kann und der proportional zur Zahl der Hybridisierungsereignisse ist.

Dieses Prinzip der amperometrischen Detektion soll stellvertretend für eine photoinduzierbar redoxaktive Einheit bzw. für eine redoxaktive Einheit am Beispiel der Glucoseoxidase näher erläutert werden. Die Glucoseoxidase ist ein aus Apoprotein und einem Cofaktor (Flavin-Adenin-Dinukleotid) bestehendes redoxaktives Enzym. Das mit einem Ende kovalent an die Elektrode angebundene Sonden-Oligonukleotid kann am anderen, noch freien Ende mit der vollständigen enzymatischen Einheit der Glucoseoxidase funktionalisiert werden, indem z. B. der Flavin-Adenin-Dinukleotid (FAD)-Cofaktor des Enzyms kovalent an das Sonden-Oligonukleotid angebunden wird und anschließend mit dem Glucoseoxidase-Apoprotein (GOx) rekonstituiert wird. Das entstandene Oberflächen-Hybrid der allgemeinen Struktur Elek-Spacer-ss-oligo-Spacer-FAD(GOx) weist zwischen Elektrode und FAD keine oder nur geringe Leitfähigkeit auf. Im Falle der Hybridisierung mit dem zu "ss-oligo" komplementären Target-Oligonukleotid wird die Leitfähigkeit deutlich erhöht wird. Bei Zusatz des Substrats Glucose zur Target-Oligonukleotid-Lösung wird das FAD der Gukoseoxidase (FAD(GOx)) zu FADH₂ der Glucoseoxidase (FADH₂(GOx)) reduziert, wobei Glucose zur Gluconsäure oxidiert wird. Liegt nun an der Elektrode ein geeignetes äußeres Potential an, so daß über das hybridisierte Oligonukleotid Elektronen von FADH₂(GOx) an die Elektrode abgegeben werden und somit FADH₂(GOx) zu FAD(GOx) reoxidiert wird (aber weder Glucose noch Gluconsäure bei diesem Potential elektrooxidiert oder -reduziert werden kann), fließt im System Elek-Spacer-ds-oligo-Spacer-FAD(GOx) solange Strom wie FAD(GOx) durch freie Glucose reduziert wird, d. h. bis die gesamte Glucose verbraucht ist bzw. für den Fall, daß an der Gegenelektrode ein Potential anliegt, bei dem Gluconsäure zu Glucose reduziert werden kann, solange wie Gluconsäure an der Gegenelektrode reduziert wird. Dieser Strom kann amperometrisch detektiert werden und ist proportional zur Zahl der Hybridisierungsereignisse.

Die für diese Erfindung relevanten photoinduzierbar redoxaktiven Einheiten bzw. chemisch induzierbar redoxaktiven Einheiten besitzen statt eines Elektron-Donors bzw. Elektron-Akzeptors jedoch mindestens einen Elektron-Donor und mindestens einen Elektron-Akzeptor.

Im Falle einer chemisch induzierbar redoxaktiven Einheit ist im Sinne der vorliegenden Erfindung mindestens ein Ladungstransferschritt zwischen Elektron-Donor(en) und Elektron-Akzeptor(en) zwischengeschaltet. Die freie redoxaktive Substanz, die D reduziert (bzw. A oxidiert) und somit einen Elektrontransfer von D⁻ zu A unter Bildung von A⁻ (bzw. den Elektrontransfer von D zu A⁺ unter Bildung von D⁺) initiiert, ermöglicht es, die Elektrode auf ein Potential zu setzten, bei dem A⁻ (bzw. D⁺), nicht jedoch A (bzw. D) oxidiert (bzw. reduziert) werden kann. Dies hat den Vorteil, daß die Elektrode ein Potential besitzt, bei dem die direkte Reaktion der freien, redoxaktiven Substanz mit der Elektrode deutlich unterdrückt werden kann und hauptsächlich Elektronenübertragungen zwischen redoxaktiver Einheit und Elektrode detektiert werden.

Handelt es sich bei der redoxaktiven Einheit um eine photoinduzierbar redoxaktive Einheit, so wird die Redoxaktivität der Einheit erst durch Licht bestimmter oder beliebiger Wellenlänge ausgelöst. Erfindungsgemäß wird diese Eigenschaft dadurch ausgenutzt, daß die elektrochemische Detektion erst durch Einstrahlen von Licht auf das Oberflächenhybrid der allgemeinen Struktur Elek-Spacer-ds-oligo-Spacer-Einheit (Oberflächenhybrid mit hybridisiertem Target) ausgelöst wird und maximal solange aufrechterhalten wird wie die Lichteinstrahlung andauert. Insbesondere bei der amperometrischen Detektion fließt somit bei Verwendung einer photoinduzierbar redoxaktiven Einheit, unter bestimmten äußeren Umständen, erst dann (längeranhaltend) Strom, wenn Licht auf das Oberfächenhybrid eingestrahlt wird. Solche äußere Umstände sind z. B. die Gegenwart eines geeigneten Reduktionsmittels (bzw. Oxidationsmittels), um einen durch Photoinduktion gebildeten, oxidierten Donor D⁺ (bzw. reduzierten Akzeptor A⁻) der photoinduzierbar redoxaktiven Einheit zu reduzieren (bzw. zu reduzieren) und das Anliegen eines Potentials an der Elektrode, bei dem zwar ein durch Photoinduktion gebildeter reduzierter Akzeptor A⁻ (bzw. oxidierter Donor D⁺) der photoinduzierbar redoxaktiven Einheit, nicht jedoch der nicht reduzierte Akzeptor A (bzw. der nicht oxidierte Donor D) oxidiert (bzw. reduziert) werden kann. Im Abschnitt "Wege zur Ausführung der Erfindung" wird dies anhand verschiedener Beispiele von Elek-Spacer-ss-oligo-Spacer-Einheit mit photoinduzierbar redoxaktiver Einheit näher erläutert. Somit kann die Detektion bei Verwendung einer photoinduzierbar redoxaktiven Einheit auf eine bestimmtes Test-Site oder eine bestimmte Test-Site-Gruppe des Oligomer-Chips räumlich beschränkt werden, indem das Licht auf dieses Test-Site oder auf diese Test-Site-Gruppe begrenzt wird. Erfindungsgemäß können also verschiedene Test-Sites (Nukleinsäure-Oligomer-Kombinationen) eines Oligomer-Chips auf eine gemeinsame, durchgängige, elektrisch leitende Oberfläche aufgebracht werden. Ein bestimmtes Test-Site oder eine bestimmte Test-Site-Gruppe kann einfach durch Anlegen eines geeigneten äußeren Potentials an die (gesamte) Oberfläche bei Lichteinstrahlung auf genau dieses Test-Site oder diese Test-Site Gruppe adressiert und amperometrisch detektiert werden. Die verschiedenen Test-Sites müssen also nicht auf einzelnen, elektrisch voneinander isolierten und zum Anlegen eines Potentials und Auslesen des Stroms einzeln ansteuerbaren (Mikro-)Elektroden aufgebracht werden. Darüberhinaus kann bei der Verwendung von Oberflächenhybriden der allgemeinen Struktur Elek-Spacer-ss-oligo-Spacer-Einheit mit photoinduzierbar redoxaktiver Einheit und amperometrischer Detektion der Ausleseprozess zur Detektion der einzelnen sequenzspezifischen Hybridisierungsereignisse auf dem Oligomer-Chip dadurch optimiert werden, daß die Test-Sites durch entsprechende Fokusierung des Lichts erst grobgerastert ausgelesen werden und dann in den Rastern mit Hybridisierungsereignissen des Auflösungsvermögen sukzessive erhöht wird, also z. B. bei einem Oktamer-Chip mit 65.536 Test-Sites zuerst in z. B. 64 Gruppen von je 1024 Test-Sites ausgelesen wird, dann die Test-Site-Gruppen, die anhand der amperometrischen Messungen Hybridisierungsereignisse aufweisen, z. B. in 32 Gruppen von je 32 Test-Sites durchgetestet werden und anschließend in den erneut Hybridisierungsereignisse aufweisenden Test-Site-Gruppen die Test-Sites einzeln ausgetestet werden. Die einzelnen Hybridisierungsereignisse können dadurch mit geringem experimentellen Aufwand schnell bestimmten Sonden-Oligomeren zugeordnet werden.

### Kurze Beschreibung der Zeichnungen

Die Erfindung soll nachfolgend anhand von Ausführungsbeispielen im Zusammenhang mit den Zeichnungen näher erläutert werden. Es zeigen
- Fig. 1: Schematische Darstellung der Oligonukleotid-Sequenzierung durch Hybridisierung auf einem Chip;
- Fig. 2: Verschiedene Reaktionssequenzen zur Herstellung des Oberflächenhybrids Elek-Spacer-ss-oligo-Spacer-UQ(RC). Die photoinduzierbar redoxaktive Einheit in diesem Oberflächenhybrid ist das Reaktionszentrum (RC) der Photosynthese betreibenden Bakterien *Rhodobacter sphaeroides.* Dieses photoinduzierbar redoxaktive Protein besteht aus Apoprotein und Cofaktoren. Das RC ist über seinen Cofaktor Ubichinon-50 (UQ) in der sogenannten Q_{A}-Protein-Bindungstasche kovalent über einen Spacer mit dem Oligonukleotid verbunden;

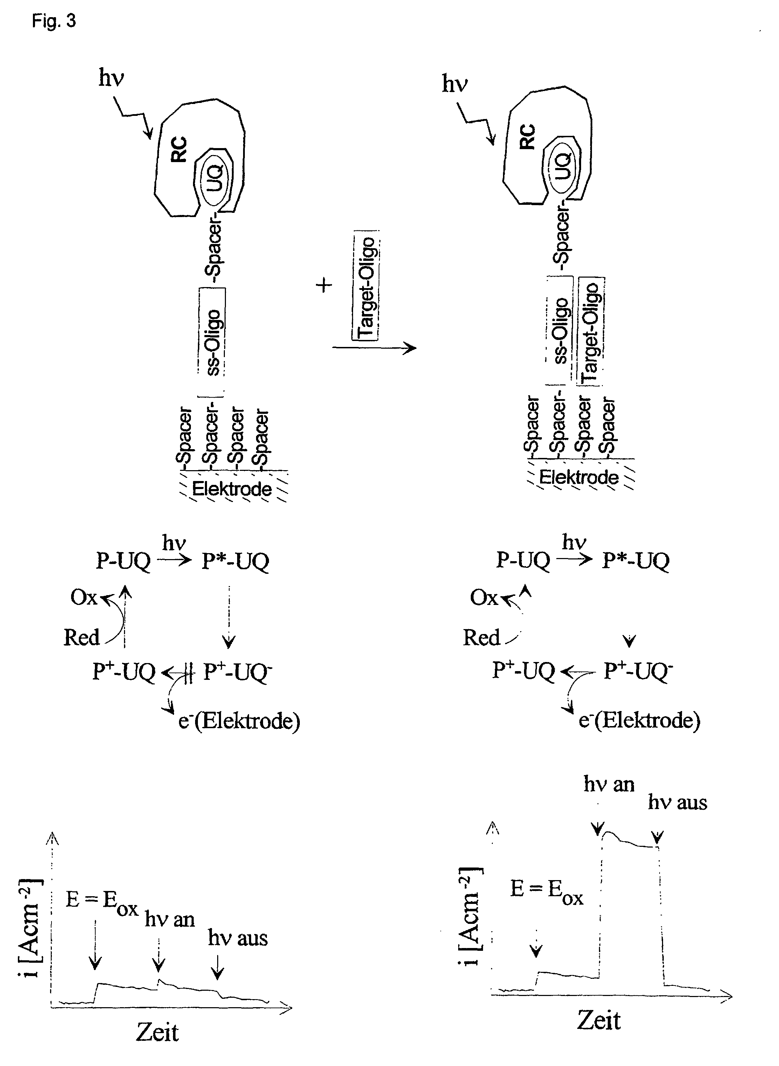

- Fig. 3: Schematische Darstellung der photoinduzierten amperometrischen Meßmethode am Beispiel des Oberflächen-Hybrids Elek-Spacer-ss-oligo-Spacer-UQ(RC) aus Figur 2 (hν: Einstrahlung von Licht, P: primärer Donor des RC, UQ: Ubichinon-50 Elektron Akzeptor in der Q_{A}-Protein-Bindungstasche des RC, Red/Ox: reduzierte bzw. oxidierte Form der freien, der Targetlösung zugesetzten redoxaktiven Substanz, z. B. cyt c₂²⁺, Natriumascorbat oder Fe(CN)₆²⁺, die die oxidierte Form P⁺ in den ursprünglich neutralen Zustand P rereduzieren können, E_{Ox}: Potential der Elektrode, bei dem UQ⁻ durch Elektronabgabe an die Elektode zu UQ oxidiert wird, "hν an": Beginn der Lichteinstrahlung, "hν aus": Ende der Lichteinstrahlung);

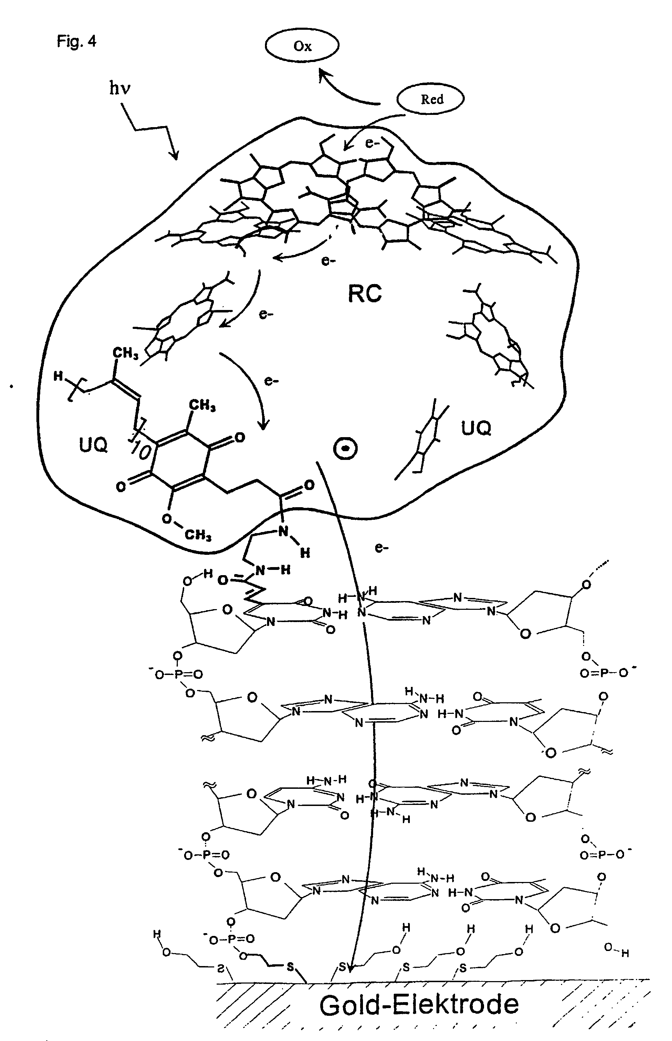

- Fig. 4: Detaillierte schematische Darstellung des Oberflächenhybrids Au-S(CH₂)₂-ds-oligo-Spacer-UQ(RC) der Figur 3 mit Gold als Oberflächenmaterial, Mercaptoethanol als Spacer (-S-CH₂CH₂- Spacer) zwischen Elektrode und Oligonukleotid und -CH₂-CH=CH-CO-NH-CH₂-CH₂-NH- als Spacer zwischen dem Elektron-Akzeptor UQ und Oligonukleotid sowie die Darstellung der Sequenz der photoinduzierten Elektron-Transfer-Schritte. Das Apoprotein des RCs ist nur als Hülle (durchgezogene Linie) angedeutet (vgl. Struktur 1). Das 12 Bp Sonden-Oligonukleotid der exemplarischen Sequenz 5'-TAGTCGGAAGCA-3' ist, als Ausschnitt, im hybridisierten Zustand gezeigt;
- Fig. 5: Schematische Darstellung der photoinduzierten amperometrischen Meßmethode am Beispiel des Oberflächen-Hybrids Elek-Spacer-ss-oligo-Spacer-Q-ZnBChl (hν: Einstrahlung von Licht, ZnBChl: das Elektron-Donor-Molekül Zn-Bakteriochlorophyll, Q: das Elektron-Akzeptor-Molekül Chinon, z. B. modifiziertes Anthrachinon oder PQQ, Red/Ox: reduzierte bzw. oxidierte Form der freien, der Targetlösung zugesetzten redoxaktiven Substanz, z. B. Fe(CN)₆²⁺, das die oxidierte Form des Elektron-Donors ZnBChl⁺ in den ursprünglich neutralen Zustand ZnBChl rereduzieren kann, E_{Ox}: Potential der Elektrode, bei dem Q⁻ durch Elektronabgabe an die Elektrode zu Q oxidiert wird, "hν an": Beginn der Lichteinstrahlung, "hν aus": Ende der Lichteinstrahlung);
- Fig. 6: Detaillierte schematische Darstellung des Oberflächenhybrids Au-S(CH₂)₂-ds-oligo-Spacer-Q-ZnBChl der Figur 5 mit Gold als Oberflächenmaterial, Mercaptoethanol als Spacer (-S-CH₂CH₂- Spacer) zwischen Elektrode und Oligonukleotid und -CH₂-CH=CH-CO-NH-CH₂-CH₂-NH- als Spacer zwischen dem Elektron-Akzeptor PQQ und Oligonukleotid sowie die Darstellung der Sequenz der photoinduzierten Elektron-Transfer-Schritte. Das 12 Bp Sonden-Oligonukleotid der exemplarischen Sequenz 5'-TAGTCGGAAGCA-3' ist, als Ausschnitt, im hybridisierten Zustand gezeigt.

### Wege zur Ausführung der Erfindung

Eine Bildungseinheit einer exemplarischen Test-Site mit hybridisiertem Target, AuS(CH₂)₂-ds-oligo-Spacer-UQ(RC) der allgemeinen Struktur Elek-Spacer-ds-oligo-Spacer-Einheit ist in Figur 4 dargestellt. Unter Bildungseinheit wird im Rahmen der vorliegenden Erfindung die kleinste sich wiederholende Einheit einer Test-Site verstanden. In dem Beispiel der Figur 4 ist die Oberfläche eine Gold-Elektrode. Die Verbindung zwischen Gold-Elektrode und Sonden-Oligonukleotid wurde mit dem Linker (HO-(CH₂)₂-S)₂ aufgebaut, der mit der endständigen Phosphatgruppe am 3' Ende zu P-O-(CH₂)₂-S-S-(CH₂)₂-OH verestert wurde und nach homolytischer Spaltung der S-S Bindung an der Gold-Oberfläche je eine Au-S Bindung bewirkte, womit 2-Hydroxy-mercaptoethanol und Mercaptoethanol-verbrücktes Oligonukleotid auf der Oberfläche koadsorbiert wurde. Die photoinduzierbar redoxaktive Einheit im Beispiel der Figur 4 ist das Reaktionszentrum (RC) der Photosynthese betreibenden Bakterien *Rhodobacter sphaeroides,* ein photoinduzierbar redoxaktives Protein bestehend aus Apoprotein und Cofaktoren. Im Anwendungsbeispiel ist das RC über seinen Cofaktor Ubichinon-50 (UQ) in der sogenannten Q_{A}-Bindungstasche des RCs kovalent mit dem Oligonukleotid verbunden, wobei zuerst freies UQ mit einer reaktiven Carbonsäuregruppe versehen wurde (siehe Beispiel 1), dann freies UQ über diese Carbonsäure-Gruppe kovalent an das Sonden-Oligonukleotid angebunden wurde (Amidbildung unter Wasserabspaltung mit der terminalen Aminofunktion des an die C-5-Position des 5'-Thymins angebundenen -CH=CH-CO-NH-CH₂-CH₂-NH₂ Linkers) und schließlich das restliche RC (Apoprotein mit allen Cofaktoren außer UQ) an UQ rekonstituiert wurde.

Wie weiter oben bereits erwähnt, kann die Modifikation der Sonden-Oligonukleotide mit der kompletten oder mit einem Bestandteil der redoxaktiven Einheit entweder vor oder nach der Bindung des Sonden-Oligonukleotids an die leitfähige Oberfläche erfolgen. Die verschiedenen Kombinationsmöglichkeiten der einzelnen Schritte, die prinzipiell zur selben Bildungseinheit einer Test-Site führen, sollen im folgenden mit Hilfe der Figur 2 am Beispiel des Oberflächenhybrids Au-S(CH₂)₂-ss-oligo-Spacer-UQ(RC) bzw. in seiner allgemeineren Form als Elek-Spacer-ss-oligo-Spacer-UQ(RC) dargestellt werden.

Das Reaktionszentrum kann durch einfache Manipulation von den beiden Ubichinon-Cofaktoren in der Q_{A}- bzw. Q_{B}-Bindungstasche befreit werden (Gunner, M.R., Robertson, D.E., Dutton, P.L.,1986, Journal of Physical Chemistry, Vol. 90, S. 3783-3795), so daß man Ubichinon getrennt vom restlichen RC (Apoprotein einschließlich aller Cofaktoren außer Ubichinon in der Q_{A}- bzw. Q_{B}-Bindungstasche) erhält. Das Sonden-Oligonukleotid ist in der Nähe der beiden Enden jeweils über einen (beliebigen) Spacer mit (gleichen oder verschiedenen) reaktiven Gruppe versehen. In einer Reaktionssequenz "1" kann das so modifizierte Sonden-Oligonukleotid in Gegenwart eines monofunktionalen Linkers (entsprechend den Punkten a) - c) und 2.) im Abschnitt "Bindung eines Oligonukleotids an die leitfähige Oberfläche") gemeinsam mit dem monofunktionalen Linker kovalent an die Elektrode angebunden werden, wobei darauf geachtet wird, daß genügend monofunktionaler Linker geeigneter Kettenlänge zugesetzt wird, um zwischen den einzelnen Sonden-Oligonukleotiden genügend Freiraum für eine Hybridisierung mit dem Target-Oligonukleotid und für die Anbindung der redoxaktiven Einheit zur Verfügung zu stellen. Danach wird an die freie, spacerverbrückte, reaktive Gruppe des Sonden-Oligonukleotids UQ, das vorher mit einer passenden reaktiven Kopplungsgruppe versehen wurde, angebunden. Die Anbindung erfolgt wie unter a) bzw. b) im Abschnitt "Bindung einer redoxaktiven Einheit an ein Nukleinsäure-Oligomer" beschrieben. Im letzten Schritt dieser Reaktionssequenz "1" wird dann das restliche RC (Apoprotein mit allen Cofaktoren außer UQ) an UQ rekonstituiert. In einer Variante dazu (Reaktionssequenz "2") kann das (mit Spacer und reaktiven Gruppen) modifizierte Sonden-Oligonukleotid zuerst ohne freien, monofunktionalen Linker (Spacer) kovalent an die Elektrode gebunden werden, wobei es zu einer flachen Anlagerung des Oligonukleotids kommt. Danach wird der freie, monofunktionale Linker (Spacer) kovalent an die Elektrode gebunden. Eine weitere Möglichkeit (Reaktionssequenz "3") besteht darin, das (mit Spacer und reaktiven Gruppen) modifizierte Sonden-Oligonukleotid zuerst mit UQ zu modifizieren, dann in Gegenwart von freiem, monofunktionalen Linker (Spacer) kovalent an die Elektrode anzubinden und anschließend mit dem restlichen RC zu rekonstituieren. Schließlich kann in einer Reaktionssequenz "4" das (mit Spacer und reaktiven Gruppen) modifizierte Sonden-Oligonukleotid zuerst mit UQ modifiziert werden, um es dann mit dem restlichen RC zu rekonstituieren und anschließend kovalent an die Elektrode zu binden. Falls, wie im Fall des RCs, die redoxaktive Einheit einen wesentlich größeren Durchmesser aufweist als das hybridisierte ds-Oligonukleotid (größer als 30 Å), kann auf die kovalente Anbindung eines geeigneten freien, monofunktionalen Linkers (Spacers) an die Elektrode verzichtet werden, anderenfalls geschieht die Anbindung der Struktur -Spacer-ss-oligo-Spacer-UQ(RC) an die Elektrode in Gegenwart eines geeigneten, freien monofunktionalen Linkers.

Im Beispiel der Figur 2 ist das RC über seinen Cofaktor Ubichinon-50 (UQ) in der sogenannten Q_{A}-Protein-Bindungstasche des RCs kovalent mit dem Oligonukleotid verbunden. Alternativ kann statt des UQ-Cofaktors in der Q_{A}-Bindungstasche auch ein anderer Cofaktor des RCs oder das Apoprotein kovalent an das Sonden-Oligonukleotid angebunden werden, es können beliebige Kombinationen der Reaktionssequenzen "1", "2", "3" oder "4" in Figur 2 angewandt werden, solange sie zum gleichen Endprodukt führen (vgl. Figur 2) und es kann in beliebigen Reaktionsschritten statt des Einzelstrang-Sonden-Oligonukleotids das mit komplementären, unmodifizierten (Target-)Oligonukleotid hybridisierte Sonden-Oligonukleotid verwendet werden. Das Sonden-Oligonukleotid kann auch direkt, also nicht über einen Spacer verbrückt, sowohl an die Elektrode als auch an die redoxaktive Einheit angebunden werden, wie unter c) im Abschnitt "Bindung eines Nukleinsäure-Oligomers an die leitfähige Oberfläche" bzw. a) im Abschnitt "Bindung einer redoxaktiven Einheit an ein Nukleinsäure-Oligomer" beschrieben.

Die elektrische Kommunikation zwischen der leitfähigen Oberfläche und der über ein Einzelstrang-Oligonukleotid verbrückten redoxaktiven Einheit in der allgemeinen Struktur Elek-Spacer-ss-oligo-Spacer-Einheit ist schwach oder gar nicht vorhanden. Durch Behandlung der Test-Site(s) mit einer zu untersuchenden Oligonukleotid-Lösung, kommt es, im Falle der Hybridisierung zwischen Sonde und Target, zu einer verstärkten Leitfähigkeit zwischen der Oberfläche und der über ein Doppelstrang-Oligonukleotid verbrückten redoxaktiven Einheit. Für die Bildungseinheit der exemplarische Test-Site Au-S(CH₂)₂-ds-oligo-Spacer-UQ(RC) (mit 12-Bp Sonden-Oligonukleotiden) ist dies schematisch in Figur 3 anhand amperometrischer Messungen gezeigt.

Durch Lichteinstrahlung geeigneter Wellenlänge auf das RC wird der Cofaktor P, der sogenannte primäre Donor, elektronisch angeregt und es kommt innerhalb der Cofaktoren des RCs zur photoinduzierten Ladungstrennung, wobei ein Elektron vom angeregten primären Donor P* auf das UQ in der Q_{A}-Bindungstasche übertragen wird. Liegt an der Elektrode ein geeignetes Potential an, um vom reduzierten Ubichinon (UQ⁻) ein Elektron auf die Elektrode zu übertragen, kommt es im Falle des nicht mit Target-Oligonukleotid hybridisierten Sonden-Oligonukleotids trotzdem zu keinem Stromfluß, da die Leitfähigkeit des ss-Oligonukleotids in Au-S(CH₂)₂-ss-oligo-Spacer-UQ(RC) sehr gering oder überhaupt nicht vorhanden ist. Im hybridisierten Zustand (Au-S(CH₂)₂-ds-oligo-Spacer-UQ(RC)) jedoch ist die Leitfähigkeit hoch, ein Elektron kann von UQ⁻ zur Elektrode übertragen werden (unter Bildung von UQ) und bei Anwesenheit einer geeigneten redoxaktiven Substanz, die P⁺ zu P zu reduziert, wird der Stromkreis geschlossen und weitere Lichtabsorption durch das RC startet den Zyklus erneut. Dies äußert sich amperometrisch in einem deutlichen Stromfluß zwischen Elektrode und photoinduzierbar redoxaktiver Einheit (Figur 3). Damit ist es möglich, die sequenzspezifische Hybridisierung des Targets mit den Sonden-Oligonukleotiden durch Amperometrie lichtinduziert zu detektieren. Die einzelnen Elektron Transfer Schritte, die im Oberflächenhybrid Au-S(CH₂)₂-ds-oligo-Spacer-UQ(RC) durch Lichteinstrahlung und bei Anwesenheit einer geeigneten redoxaktiven Substanz zur Reduktion von P⁺ zu P ausgelöst werden, sind in Figur 4 dargestellt. Natürlich kann das Oberflächenhybrid Au-S(CH₂)₂-ds-oligo-Spacer-UQ(RC) unter geeigneten äußeren Umständen und bei geeigneter Anbindung (z. B. Anbindung des RCs an das Sonden-Oligonukleotid in der Nähe des primären Donors), auch umgekehrt geschaltet werden, so daß nach Lichteinstrahlung P⁺ von der Elektrode reduziert und Q⁻ von einem geeigneten Oxidationsmittel oxidiert wird.

Eine weiteres Test-Site, Au-S(CH₂)₂-ss-oligo-Spacer-Q-ZnBChl, der allgemeinen Struktur Elek-Spacer-ss-oligo-Spacer-Einheit ist in Figur 5 dargestellt. Durch Lichteinstrahlung geeigneter Wellenlänge auf ZnBChl wird ZnBChl elektronisch angeregt und es kommt zur photoinduzierten Ladungstrennung, wobei ein Elektron vom angeregten ZnBChl* auf das Chinon Q übertragen wird. Liegt an der Elektrode ein geeignetes Potential an, um vom so reduzierten Chinon (Q⁻) ein Elektron auf die Elektrode zu übertragen, kommt es im Falle des nicht mit Target-Oligonukleotid hybridisierten Sonden-Oligonukleotids trotzdem zu keinem Stromfluß, da die Leitfähigkeit des ss-Oligonukleotids in Au-S(CH₂)₂-ss-oligo-Spacer-Q-ZnBChl sehr gering oder überhaupt nicht vorhanden ist. Im hybridisierten Zustand Au-S(CH₂)₂-dsoligo-Spacer-Q-ZnBChl jedoch ist die Leitfähigkeit hoch, ein Elektron kann von Q⁻ zur Elektrode übertragen werden (unter Bildung von Q) und bei Anwesenheit einer geeigneten redoxaktiven Substanz, die ZnBChl⁺ zu ZnBChl reduziert, wird der Stromkreis geschlossen und weitere Lichtabsorption durch ZnBChl startet den Zyklus erneut. Dies äußert sich amperometrisch in einem deutlichen Stromfluß zwischen Elektrode und photoinduzierbar redoxaktiver Einheit (Figur 5). Damit ist es möglich, die sequenzspezifische Hybridisierung des Targets mit den Sonden-Oligonukleotiden durch Amperometrie lichtinduziert zu detektieren. Natürlich kann das Oberflächenhybrid Au-S(CH₂)₂-ds-oligo-Spacer-Q-ZnBChI unter geeigneten äußeren Umständen und geeigneter Anbindung (z. B. Au-S(CH₂)₂-ds-oligo-Spacer-ZnBChl-Q) auch umgekehrt geschaltet werden, so daß nach Lichteinstrahlung ZnBChl⁺ von der Elektrode reduziert und Q⁻ von einem geeigneten Oxidationsmittel oxidiert wird.

Da die Redoxaktivität der photoinduzierbar redoxaktiven Einheit - auch bei passendem Elektrodenpotential - erst durch Lichteinstrahlung geeigneter Wellenlänge ausgelöst und maximal solange aufrechterhalten wird, wie die Lichteinstrahlung andauert, kann dies erfindungsgemäß dadurch ausgenutzt werden, daß ein bestimmtes Test-Site oder eine bestimmte Test-Site-Gruppe eines Oligomer-Chips räumlich aufgelöst wird, indem das Licht auf dieses Test-Site oder auf diese Test-Site-Gruppe begrenzt wird. Dies birgt den erfindungsgemäßen Vorteil, daß die verschiedenen Test-Sites (Nukleinsäure-Oligomer-Kombinationen) eines Oligomer-Chips auf eine gemeinsame, durchgängige, elektrisch leitende Oberfläche aufgebracht werden können und ein bestimmtes Test-Site oder eine bestimmte Test-Site-Gruppen einfach durch Anlegen eines geeigneten äußeren Potentials an die (gesamte) Oberfläche bei Lichteinstrahlung nur auf genau dieses Test-Site oder diese Test-Site Gruppe adressiert und amperometrisch detektiert werden kann. Die verschiedenen Test-Sites müssen also nicht auf einzelnen, elektrisch voneinander isolierten und zum Anlegen eines Potentials und Auslesen des Stroms einzeln ansteuerbaren (Mikro-) Elektroden aufgebracht werden.

Daneben können fehlerhafte Basenpaarungen (Basenpaar Mismatches) durch eine geänderte cyclovoltammetrische Charakteristik erkannt werden. Ein Mismatch äußert sich in einem größeren Potentialabstand zwischen den Strommaxima der Elektroreduktion und der Elektroreoxidation (Umkehrung der Elektroreduktion bei umgekehrter Potentialvorschubrichtung) bzw. der Elektrooxidation und Elektrorereduktion in einem cyclovoltammetrisch reversiblen Elektronen-Transfer zwischen der elektrisch leitenden Oberfläche und der photoinduzierbar redoxaktiven Einheit. Dieser Umstand wirkt sich vor allem in der amperometrischen Detektion günstig aus, da dort der Strom bei einem Potential getestet werden kann, bei dem zwar das perfekt hybridisierende Oligonukleotid-Target signifikant Strom liefert, nicht aber das fehlerhaft gepaarte Oligonukleotid-Target.

***Beispiel 1:*** *Modifikation des Ubichinon-50 mit einer Spacer-verbrückten reaktiven Carbonsäure-Gruppe.* Die 2-Methoxy-Gruppe des Ubichinon-50 (UQ-50) wird durch Etherspaltung mit HBr, einer Standardmethode, zur 2-Hydroxygruppe modifiziert (alternativ kann 2-OH-UQ-50 nach dem Verfahren von Moore, H. W. and Folkers, K. Journal of the American Chemical Society, 1966, 88, 564-570 oder von Daves, G. et al., Journal of the American Chemical Society, 1968, 90, 5587-5593 hergestellt werden). Anschließend wird 2-OH-UQ-50 in einem Standardverfahren mit einer äquimolaren Menge an Cl-CH₂-CH₂-CO₂H zum 2-(CH₂-CH₂-CO₂H)-UQ-50 umgesetzt und chromatographisch aufgereinigt. Alternativ können 5-OH-6-alkyl-1,4-Benzochinon-Analoga des UQ-50 (Darstellung gemäß Catlin et al., Journal of the American Chemical Society, 1968, 90, 3572-3574) in einem Standardverfahren mit einer äquimolaren Menge an Cl-CH₂-CH₂-CO₂H zu 5-(CH₂-CH₂-CO₂H)-UQ-50-Analoga modifiziert werden.

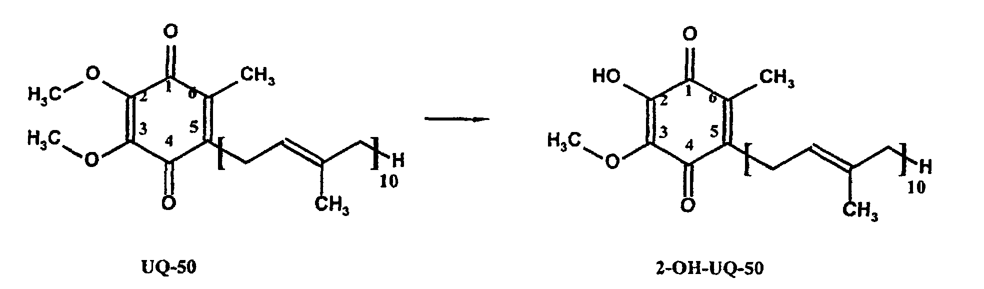

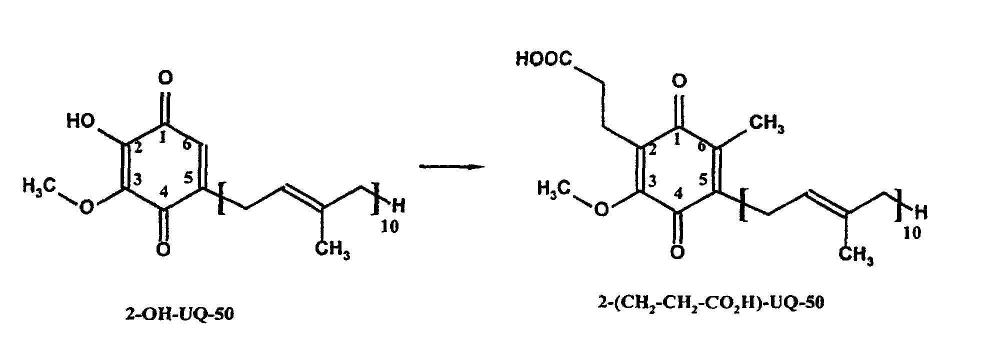

***Beispiel 2:*** *Herstellung der Oligonukleotid-Elektrode Au-S(CH*_{*2*}*)*_{*2*}*-ss-oligo SpacerUQ(RC).* Die Herstellung von Au-S(CH₂)₂-ss-oligo-Spacer-UQ(RC) gliedert sich in 4 Teilabschnitte, nämlich der Darstellung der leitfähigen Oberfläche, der Derivatisierung der Oberfläche mit dem Sonden-Oligonukleotid in Gegenwart eines geeigneten monofunktionalen Linkers (Inkubationsschritt), der kovalenten Anbindung des modifizierten Ubichinon-50 (Redoxschritt) und der Rekonstitution des restlichen RCs (Rekonstitutionsschritt).

Das Trägermaterial für die kovalente Anbindung der Doppelstrang-Oligonukleotide bildet ein ca. 100 nm dünner Gold-Film auf Mica (Muskovit Plättchen). Dazu wurde in einer elektrischen Entladungskammer frisch gespaltenes Mica mit einem Argonlonenplasma gereinigt und durch elektrische Entladung Gold (99.99%) in einer Schichtdicke von ca. 100nm aufgebracht. Anschließend wurde der Gold-Film mit 30 % H₂O₂, / 70 % H₂SO₄ von Oberflächenverunreinigungen befreit (Oxidation organischer Ablagerungen) und für ca. 20 Minuten in Ethanol getaucht, um an der Oberfläche adsorbierten Sauerstoff zu verdrängen. Nach Abspülen der Oberfläche mit bidestilliertem Wasser wird auf die horizontal gelagerte Oberfläche eine vorher bereitete 1x10⁻⁴ molare Lösung des (modifizierten) Doppelstrang-Oligonukleotids aufgetragen, so daß die komplette Gold-Oberfläche benetzt wird (Inkubationsschritt, siehe auch unten).

Zur Inkubation wurde ein doppelt modifiziertes 12 Bp Einzelstrang-Oligonukleotid der Sequenz 5'-TAGTCGGAAGCA-3' verwendet, das an der Phosphatgruppe des 3' Endes mit (HO-(CH₂)₂-S)₂ zum P-O-(CH₂)₂-S-S-(CH₂)₂-OH verestert ist. Am 5'-Ende ist die endständige Base Thymin des Oligonukleotids am C-5 Kohlenstoff mit -CH=CH-CO-NH-CH₂-CH₂-NH₂ modifiziert. Zu einer 2x10⁻⁴ molaren Lösung dieses Oligonukleotids in HEPES-Puffer (0,1 molar in Wasser, pH 7.5 mit 0.7 molarem Zusatz von TEATFB, siehe Abkürzungen) wurde ca. 10⁻⁴ bis 10⁻¹ molar 2-Hydroxy-mercaptoethanol gegeben (oder ein anderer Thiol- oder Disulfid-Linker geeigneter Kettenlänge) und die Gold-Oberfläche eines Test-Sites komplett benetzt und 2-24h inkubiert. Während dieser Reaktionszeit wird der Disulfidspacer P-O-(CH₂)₂-S-S-(CH₂)₂-OH des Oligonukleotids homolytisch gespalten. Dabei bildet der Spacer mit Au-Atomen der Oberfläche eine kovalente Au-S Bindung aus, wodurch es zu einer 1:1 Koadsorption des ss-Oligonukleotids und des abgespaltenen 2-Hydroxy-mercaptoethanols kommt. Das in der Inkubationslösung gleichzeitig anwesende, freie 2-Hydroxy-mercaptoethanol wird ebenfalls durch Ausbildung einer Au-S Bindung koadsorbiert (Inkubationsschritt).

Die so mit einer Monolayer aus ss-Oligonukleotid und 2-Hydroxy-mercaptoethanol modifizierte Goldelektrode wurde mit bidestilliertem Wasser gewaschen und anschließend mit einer Lösung von 3x10⁻³ molarem Chinon 2-(CH₂-CH₂-CO₂H)-UQ-50, 10⁻² molarem EDC und 10⁻² molarem sulfo-NHS in HEPES-Puffer (0,1 molar (in Wasser, pH = 7.5), benetzt. Nach einer Reaktionszeit von ca. 1 - 4 h bilden der - CH=CH-CO-NH-CH₂-CH₂-NH₂ Spacer und das 2-(CH₂-CH₂-CO₂H)-UQ-50 eine kovalente Bindung aus (Amidbildung zwischen der Aminogruppe des Spacers und der C-2-Säurefunktion des 2-(CH₂-CH₂-CO₂H)-UQ-50, Redoxschritt).

Anschließend wurde die so modifizierte Goldelektrode mit bidestilliertem Wasser gewaschen und mit einer Lösung von ca. 5x10⁻⁵ molarem Ubichinon-50-freien RCs in 10 mM Tris, pH = 8, mit 0.7 molarem Zusatz von TEATFB bei ca. 4 °C für ca. 12 h inkubiert, um das restliche RC an das Oligonukleotid-gebundene UQ-50 zu rekonstituieren (Rekonstitutionsschritt).

Alternativ zur kovalenten Anbindung von 2-(CH₂-CH₂-CO₂H)-UQ-50 an das Sonden-Oligonukleotid kann, unter gleichen Bedingungen, auch ein 5-(CH₂-CH₂-CO₂H)-UQ-50-Analogon (Beispiel 1) oder ein anderes, mit einer reaktiven Carbonsäure versehenes Chinon der Formel 1 - 8 verwendet werden, da auch an diese Ubichinon-50-freies RC rekonstituiert werden kann.

***Beispiel 3:*** *Herstellung der Oligonukleotid-Elektrode Au-S(CH*_{*2*}*)*_{*2*}*-ss-oligo-Spacer-Q ZnBChl.* Die Herstellung von Au-S(CH₂)₂-ss-oligo-Spacer-Q-ZnBChl gliedert sich in 5 Teilabschnitte, nämlich der Darstellung der leitfähigen Oberfläche, der Derivatisierung der Oberfläche mit dem (mit Komplementärstrang hybridisierten) Sonden-Oligonukleotid in Gegenwart eines geeigneten monofunktionalen Linkers (Inkubationsschritt), der kovalenten Anbindung des Elektron-Akzeptors (Akzeptorschritt), der kovalenten Anbindung des Elektron-Donors (Donorschritt) und der thermischen Dehybridisierung des Doppelstrang-Oligonukleotids (Dehybridisierungsschritt).

Das Trägermaterial für die kovalente Anbindung der Doppelstrang-Oligonukleotide, ein ca. 100 nm dünner Gold-Film auf Mica (Muskovit Plättchen), wurde wie in Beispiel 1 beschrieben, hergestellt.

Zur Inkubation wurde ein doppelt modifiziertes 12 Bp Einzelstrang-Oligonukleotid der Sequenz 5'-TAGTCGGAAGCA-3' verwendet, das an der Phosphatgruppe des 3' Endes mit (HO-(CH₂)₂-S)₂ zum P-O-(CH₂)₂-S-S-(CH₂)₂-OH verestert ist. Am 5'-Ende ist die endständige Base Thymin des Oligonukleotids am C-5 Kohlenstoff mit -CH=CH-CO-NH-CH₂-CH₂-NH₂ modifiziert. Eine 2x10⁻⁴ molare Lösung dieses Oligonukleotids im Hybridisierungspuffer (10 mM Tris, 1 mM EDTA, pH 7.5 mit 0.7 molarem Zusatz von TEATFB, siehe Abkürzungen) wurde mit einer 2x10⁻⁴ molaren Lösung des (unmodifizierten) komplementären Strangs im Hybridisierungspuffer bei Raumtemperatur für ca. 2h hybridisiert (Hybridisierungsschritt). Nach der Hybridisierung wurde der nun 1x10⁻⁴ molaren Doppelstrang-Oligonukleotid-Lösung ca. 10⁻⁴ bis 10⁻¹ molar 2-Hydroxy-mercaptoethanol (oder ein anderer Thiol- oder Disulfid-Linkers geeigneter Kettenlänge) zugesetzt, die Gold-Oberfläche eines Test-Sites komplett benetzt und 2-24h inkubiert. Während dieser Reaktionszeit wird der Disulfidspacer P-O-(CH₂)₂-S-S-(CH₂)₂-OH des Oligonukleotids homolytisch gespalten. Dabei bildet der Spacer mit Au-Atomen der Oberfläche eine kovalente Au-S Bindung aus, wodurch es zu einer 1:1 Koadsorption des ds-Oligonukleotids und des abgespaltenen 2-Hydroxy-mercaptoethanols kommt. Das in der Inkubationslösung gleichzeitig anwesende, freie 2-Hydroxy-mercaptoethanol wird ebenfalls durch Ausbildung einer Au-S Bindung koadsorbiert (Inkubationsschritt).

Die so mit einer Monolayer aus ds-Oligonukleotid und 2-Hydroxy-mercaptoethanol modifizierte Goldelektrode wurde mit bidestilliertem Wasser gewaschen und anschließend mit einer Lösung von 3x10⁻³ molarem Chinon PQQ, 10⁻² molarem EDC und 10⁻² molarem sulfo-NHS in HEPES Puffer benetzt. Nach einer Reaktionszeit von ca. 1 - 4 h bilden der -CH=CH-CO-NH-CH₂-CH₂-NH₂ Spacer und das PQQ eine kovalente Bindung (Amidbildung zwischen der Aminogruppe des Spacers und der C-7-Carbonsäurefunktion des PQQ, Akzeptorschritt).

Anschließend wurde die so modifizierte Goldelektrode mit bidestilliertem Wasser gewaschen und mit einer wässerigen Lösung aus 3x10⁻³ molarem Donor ZnBChl (freie Säure), 1,5x10⁻¹ molarem EDC, 2,5x10⁻³ molarem Hydrazin-Monohydrat (NH₂-NH₂xH₂O) und 1x10⁻¹ molarem Imidazol benetzt. Nach einer Reaktionszeit von ca. 16 h bei 23 °C bindet die C-1-Carbonsäurefunktion des an das Oligonukleotid gebundenen PQQ über Hydrazin an die freie Carbonsäure-Gruppe des ZnBChl (Amidbildung zwischen den Aminogruppen des Hydrazins und der C-1-Carbonsäure-Gruppe des PQQ bzw. der freien Carbonsäure-Gruppe des ZnBChl, Donorschritt). Anschließend wurden die Doppelstränge bei Temperaturen von T > 40 °C thermisch dehybridisiert und erneut mit bidestilliertem Wasser abgespült (Dehybridisierungsschritt). Das ZnBChl (freie Säure) wird aus Zn-BChl (Darstellung gemäß Hartwich et al., Journal of the American Chemical Society, 1998, 120, 3684-3693) durch Inkubation mit Trifluoressigsäure hergestellt.

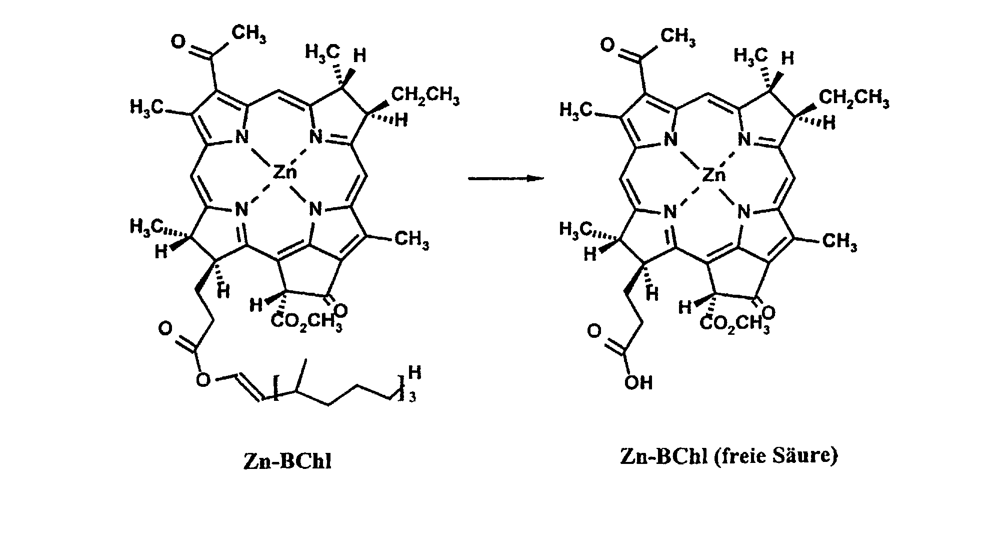

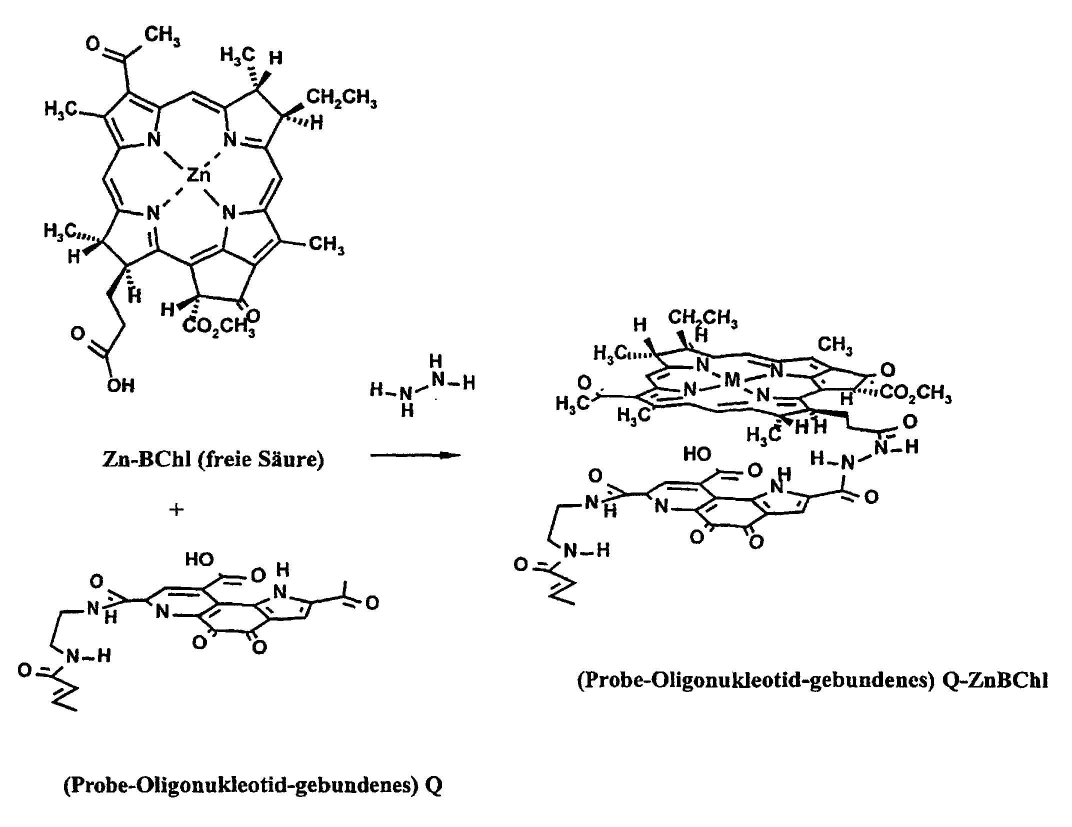

Alternativ kann z. B. ZnBChl (freie Säure) über Esterbildung nach Standardverfahren auch an die 3-OH-Gruppe des 5'-terminalen Zuckers des Sonden-Oligonukleotids gebunden werden oder der vorher kovalent verbundene Elektron-Donor/Elektron-Akzeptor-Komplex wird, wie im Donorschritt beschrieben, über eine freie Carbonsäure-Gruppe z. B. des Donors an das Sonden-Oligonukleotid angebunden. Statt PQQ kann unter den gleichen Reaktionsbedingungen auch Anthrachinon-2,6-Disulfonsäure Dinatriumsalz im Akzeptorschritt verwendet werden. Bei Verwendung von PNA-Oligonukleotid mit z. B. -NH-(CH₂)₂-N(COCH₂-Base)-CH₂CO- als Oligonukleotid-Baustein besteht eine alternative Anbindungsmöglichkeit der ZnBChl-PQQ-Einheit an das Nukleinsäure-Oligomer (PNA-Oligonukleotid) entsprechend d) im Abschnitt "Bindung einer photoinduzierbar redoxaktiven Einheit an ein Nukleinsäure-Oligomer". Dabei wird während der PNA-Oligonukleotid-Synthese statt der N-terminale Base in der Standard-PNA-Synthese-Reaktion PQQ über seinen Pyrrol-Stickstoff angebunden. Anschließend wird Zn-BChl, ähnlich wie im Donorschritt beschrieben, durch Inkubation des mit PQQ modifizierten PNA-Oligonukleotids mit 3x10⁻³ molarem ZnBChl (freie Säure), 1,5x10⁻¹ molarem EDC 10⁻² und 2x10⁻¹ molarem sulfo-NHS in HEPES Puffer an das Amino-Ende des Peptid-Rückgrats gebunden (Amidbildung zwischen der Aminogruppe des Rückgrats und der Carbonsäure-Gruppe des Zn-BChl (freie Säure)).

## Patentansprüche

1. Durch kovalente Anbindung einer redoxaktiven Einheit modifiziertes Nukleinsäure-Oligomer, **dadurch gekennzeichnet, dass** die redoxaktive Einheit wenigstens ein Elektron-Donor-Molekül und wenigstens ein Elektron-Akzeptor-Molekül enthält, wobei Elektron-Donor-Molekül und Elektron-Akzeptor-Molekül nicht durch Nukleinsäure-Oligomere miteinander verbunden sind.

2. Modifiziertes Nukleinsäure-Oligomer nach Anspruch 1, **dadurch gekennzeichnet, dass** die redoxaktive Einheit wenigstens einen redoxaktiven, verknüpften, wenigstens bimolekularen Elekton-Donor-/Elektron-Akzeptor-Komplex enthält, wobei wenigstens ein Elektron-Donor-Molekül der redoxaktiven Einheit und wenigstens ein Elektron-Akzeptor-Molekül der redoxaktiven Einheit durch eine oder mehrere Bindungen miteinander verbunden sind.

3. Modifiziertes Nukleinsäure-Oligomer nach Anspruch 2, **dadurch gekennzeichnet, dass** es sich bei den Bindungen um kovalente Bindungen handelt.

4. Modifiziertes Nukleinsäure-Oligomer nach Anspruch 1, **dadurch gekennzeichnet, dass** die redoxaktive Einheit wenigstens einen redoxaktiven, verknüpften, wenigstens bimolekularen Etekton-Donor-/Elektron-Akzeptor-Komplex enthält, wobei wenigstens ein Elektron-Donor-Molekül der redoxaktiven Einheit und wenigstens ein Elektron-Akzeptor-Molekül der redoxaktiven Einheit durch einen oder mehrere verzweigte oder unverzweigte Molekülteile beliebiger Zusammensetzung und Kettenlänge kovalent verbunden sind.

5. Modifiziertes Nukleinsäure-Oligomer nach Anspruch 4, **dadurch gekennzeichnet, dass** die verzweigten oder unverzweigten Molekülteile eine Kettenlänge von 1 - 20 Atomen aufweisen.

6. Modifiziertes Nukleinsäure-Oligomer nach Anspruch 5, **dadurch gekennzeichnet, dass** die verzweigten oder unverzweigten Molekülteile eine Kettenlänge von 1 - 14 Atomen, aufweisen.

7. Modifiziertes Nukleinsäure-Oligomer nach einem der vorhergehenden Ansprüche, **dadurch gekennzeichnet, dass** die redoxaktive Einheit zusätzlich ein oder mehrere Makromoleküle umfaßt.

8. Modifiziertes Nukleinsäure-Oligomer nach einem der vorhergehenden Ansprüche, wobei die redoxaktive Einheit das native oder modifizierte Reaktionszentrum von Photosynthese betreibenden Organismen ist.

9. Modifiziertes Nukleinsäure-Oligomer nach Anspruch 8, wobei die redoxaktive Einheit das native oder modifizierte Reaktionszentrum von Photosynthese betreibenden Bakterien ist.

10. Modifiziertes Nukleinsäure Oligomer nach einem der Ansprüche 1 bis 7, **dadurch gekennzeichnet, dass** wenigstens eines der Elektron-Donor-Moleküle und Elektron-Akzeptor-Moleküle ein Farbstoff ist.

11. Modifiziertes Nukleinsäure-Oligomer nach Anspruch 10, **dadurch gekennzeichnet, dass** es sich bei dem Farbstoff um ein Flavin, ein (Metallo-)Porphyrin, ein (Metallo-)Chlorophyll, ein (Metallo-)Bakteriochlorophyll oder ein Derivat dieser Farbstoffe handelt.

12. Modifiziertes Nukleinsäure-Oligomer nach einem der Ansprüche 1 bis 7, **dadurch gekennzeichnet, dass** wenigstens eines der Elektron-Donor-Moleküle und Elektron-Akzeptor-Moleküle ein-Nikotinsäureamid oder ein Chinon ist.

13. Modifiziertes Nukleinsäure-Oligomer nach Anspruch 12, **dadurch gekennzeichnet, dass** es sich bei dem Chinon um ein Pyrrolo-Chinolin-Chinon (PQQ), ein 1,2-Benzochinon, ein 1,4-Benzochinon, ein 1,2-Naphtochinon, ein 1,4-Naphtochinon, ein 9,10-Anthrachinon oder eines derer Derivate handelt.

14. Modifiziertes Nukleinsäure-Oligomer nach einem der Ansprüche 1 bis 7, **dadurch gekennzeichnet, dass** wenigstens eines der Elektron-Donor-Moleküle und Elektron-Akzeptor-Moleküle ein Charge-Transfer-Komplex ist.

15. Modifiziertes Nukleinsäure-Oligomer nach Anspruch 14, **dadurch gekennzeichnet, dass** der Charge-Transfer-Komplex ein Übergangsmetall-Komplex ist.

16. Modifiziertes Nukleinsäure-Oligomer nach Anspruch 15, **dadurch gekennzeichnet, dass** der Charge-Transfer-Komplex ein Ru(II)-, ein Cr(III)-, ein Fe(II)-, ein Os(II)- oder ein Co(II)-Komplex ist.

17. Modifiziertes Nukleinsäure-Oligomer nach einem der vorhergehenden Ansprüche, **dadurch gekennzeichnet, dass** das modifizierte Nukleinsäure-Oligomer sequenzspezifisch Einzelstrang-DNA, RNA und/oder PNA binden kann.

18. Modifiziertes Nukleinsäure-Oligomer nach Anspruch 17, **dadurch gekennzeichnet, dass** das modifizierte Nukleinsäure-Oligomer ein Desoxyribonukleinsäure-, ein Ribonukleinsäure- oder ein Peptidnukleinsäure-Oligomer ist.

19. Modifiziertes Nukleinsäure-Oligomer nach einem der vorhergehenden Ansprüche, **dadurch gekennzeichnet, dass** die redoxaktive Einheit kovalent an eine der Phosphorsäure-Gruppen, an eine der Carbonsäure-Gruppen, an eine der Amin-Gruppen oder an einen Zucker des Nukleinsäure-Oligomer-Rückgrats gebunden ist.

20. Modifiziertes Nukleinsäure-Oligomer nach einem der vorhergehenden Ansprüche, **dadurch gekennzeichnet, dass** die redoxaktive Einheit kovalent an an eine Zucker-Hydroxy-Gruppe des Nukleinsäure-Oligomer-Rückgrats gebunden ist.

21. Modifiziertes Nukleinsäure-Oligomer nach einem der Ansprüche 1 bis 18, **dadurch gekennzeichnet, dass** die redoxaktive Einheit kovalent an eine Thiol-Gruppe, eine Hydroxy-Gruppe, eine Carbonsäure-Gruppe oder eine Amin-Gruppe einer modifizierten Base des Nukleinsäure-Oligomers angebunden ist.

22. Modifiziertes Nukleinsäure-Oligomer nach Anspruch 21, **dadurch gekennzeichnet, dass** die reaktive Thiol-, Hydroxy-, Carbonsäure- oder Arnin-Gruppe der Base kovalent über einen verzweigten oder unverzweigten Molekülteil beliebiger Zusammensetzung und Kettenlänge an die Base gebunden ist, wobei die kürzeste durchgehende Verbindung zwischen der Thiol-, Hydroxy-, Carbonsäure- oder Amin-Gruppe und der Base ein verzweigtes oder unverzweigtes Molekülteil mit einer Kettenlänge von 1 - 20 Atomen ist.

23. Modifiziertes Nukleinsäure-Oligomer nach Anspruch 22, **dadurch gekennzeichnet, dass** die kürzeste durchgehende Verbindung zwischen der Thiol-, Hydroxy-, Carbonsäure- oder Amin-Gruppe und der Base ein verzweigtes oder unverzweigtes Molekülteil mit einer Kettenlänge von 1 - 14 Atomen ist.

24. Modifiziertes Nukleinsäure-Oligomer nach einem der Ansprüche 19 bis 22, **dadurch gekennzeichnet, dass** die redoxaktive Einheit an ein Ende des Nukleinsäure-Oligomer-Rückgrats oder an eine endständige, modifizierte Base angebunden ist.

25. Modifiziertes Nukleinsäure-Oligomer nach einem der vorhergehenden Ansprüche, **dadurch gekennzeichnet, dass** die redoxaktive Einheit eine photoinduzierbar redoxaktive Einheit ist.

26. Modifiziertes Nukleinsäure-Oligomer nach einem der Ansprüche 1 bis 24, **dadurch gekennzeichnet, dass** die redoxaktive Einheit eine chemisch induzierbar redoxaktive Einheit ist.

27. Modifiziertes Nukleinsäure-Oligomer nach einem der vorhergehenden Ansprüche, **dadurch gekennzeichnet, dass** mehrere redoxaktive Einheiten an das Nukleinsäure-Oligomer angebunden sind.

28. Verfahren zur Herstellung eines modifizierten Nukleinsäure-Oligomers wie in einem der vorhergehenden Ansprüche definiert, **dadurch gekennzeichnet, dass** eine redoxaktive Einheit kovalent an ein Nukleinsäure-Oligomer angebunden wird.

29. Verfahren zur Herstellung eines modifizierten Nukleinsäure-Oligomers nach Anspruch 28, **dadurch gekennzeichnet, dass** die redoxaktive Einheit durch kovalente Anbindung von wenigstens einem Elektron-Donor-Motekül an ein Nukleinsäure-Oligomer angebunden wird.

30. Verfahren zur Herstellung eines modifizierten Nukleinsäure-Oligomers nach Anspruch 28, **dadurch gekennzeichnet, dass** die redoxaktive Einheit durch kovalente Anbindung von wenigstens einem Elektron-Akzeptor-Molekül an ein Nukleinsäure-Oligomer angebunden wird.

31. Verfahren zur Herstellung eines modifizierten Nukleinsäure-Oligomers nach Anspruch 28, **dadurch gekennzeichnet, dass** die redoxaktive Einheit durch kovalente Anbindung wenigstens eines Makromoleküls oder durch kovalente Anbindung wenigstens eines Proteins an ein Nukleinsäure-Oligomer angebunden wird.

32. Verfahren zur Herstellung eines modifizierten Nukleinsäure-Oligomers nach den Ansprüchen 29 bis 31, **dadurch gekennzeichnet, dass** die redoxaktive Einheit durch Zugabe von wenigstens einem Bestandteil ausgewählt aus der Gruppe bestehend aus Elektron-Akzeptor-Moleküle, Elektron-Donor-Moleküle, Makromoleküle und Proteine vervollständigt wird.

33. Verfahren zur Herstellung eines modifizierten Nukleinsäure-Oligomers nach einem der Ansprüche 28 bis 32, **dadurch gekennzeichnet, dass** das Nukleinsäure-Oligomer durch eine oder mehrere Amidbildungen mit Amin- oder mit Säure-Gruppen der redoxaktiven Einheit, durch eine oder mehrere Esterbildungen mit Alkohol- oder mit Säure-Gruppen der redoxaktiven Einheit, durch Thioesterbildung mit Thio-Alkohol- oder mit Säure-Gruppen der redoxaktiven Einheit oder durch Kondensation einer oder mehrerer Amin-Gruppen des Nukleinsäure-Oligomers mit Aldehyd-Gruppen der redoxaktiven Einheit und anschließender Reduktion der entstandenen Kohlenstoff-Stickstoff-Doppelbindung an die redoxaktive Einheit gebunden wird.

34. Verfahren zur Herstellung eines modifizierten Nukleinsäure-Oligomers nach einem der Ansprüche 28 bis 33, **dadurch gekennzeichnet, dass** wenigstens ein verzweigter oder unverzweigter Molekülteil beliebiger Zusammensetzung und Kettenlänge kovalent an die redoxaktive Einheit angebunden ist und die verzweigten oder unverzweigten Molekülteile eine reaktive Amin-, Hydroxy-, Thiol-, Säure- oder Aldehyd-Gruppe zur kovalenten Anbindung an ein Nukleinsäure-Oligomer aufweisen.

35. Verfahren zur Herstellung eines modifizierten Nukleinsäure-Oligomers nach Anspruch 34, **dadurch gekennzeichnet, dass** die kürzeste durchgehende Verbindung zwischen dem Nukleinsäure-Oligomer und der redoxaktiven Einheit ein verzweigtes oder unverzweigtes Molekülteil mit einer Kettenlänge von 1 - 20 Atomen ist.

36. Verfahren zur Herstellung eines modifizierten Nukleinsäure-Oligomers nach Anspruch 35, **dadurch gekennzeichnet, dass** die kürzeste durchgehende Verbindung zwischen dem Nukleinsäure-Oligomer und der redoxaktiven Einheit ein verzweigtes oder unverzweigtes Molekülteil mit einer Kettenlänge von 1 - 14 Atomen ist.

37. Modifizierte leitfähige Oberfläche, **dadurch gekennzeichnet, dass** wenigstens eine Art von modifiziertem Nukleinsäure-Oligomer gemäß einem der Ansprüche 1 bis 27 an eine leitfähige Oberfläche angebunden ist.

38. Modifizierte leitfähige Oberfläche nach Anspruch 37, **dadurch gekennzeichnet, dass** die Oberfläche aus einem Metall oder einer Metallegierung besteht.

39. Modifizierte leitfähige Oberfläche nach Anspruch 38, **dadurch gekennzeichnet, dass** die Oberfläche aus einem Metall ausgewählt aus der Gruppe Platin, Palladium, Gold, Cadmium, Quecksilber, Nickel, Zink, Kohlenstoff, Silber, Kupfer, Eisen, Blei, Aluminium und Mangan besteht.

40. Modifizierte leitfähige Oberfläche nach Anspruch 37, **dadurch gekennzeichnet, dass** die Oberfläche aus einem Halbleiter besteht.

41. Modifizierte leitfähige Oberfläche nach Anspruch 38, **dadurch gekennzeichnet, dass** die Oberfläche aus einem Halbleiter ausgewählt aus der Gruppe Kohlenstoff, Silizium, Germanium und Zinn besteht.

42. Modifizierte leitfähige Oberfläche nach Anspruch 37, **dadurch gekennzeichnet, dass** die Oberfläche aus einer binären Verbindung der Elemente der Gruppen 14 und 16, einer binären Verbindung der Elemente der Gruppen 13 und 15, einer binären Verbindung der Elemente der Gruppen 15 und 16, oder einer binären Verbindung der Elemente der Gruppen 11 und 17 besteht.

43. Modifizierte leitfähige Oberfläche nach Anspruch 42, **dadurch gekennzeichnet, dass** die Oberfläche aus einem Cu(I)-Halogenid oder einem Ag(I)-Halogenid besteht.

44. Modifizierte leitfähige Oberfläche nach Anspruch 37, **dadurch gekennzeichnet, dass** die Oberfläche aus einer temären Verbindung der Elemente der Gruppen 11, 13 und 16 oder einer temären Verbindung Elemente der Gruppen 12, 13 und 16 besteht.

45. Modifizierte leitfähige Oberfläche nach den Ansprüchen 37 bis 44, **dadurch gekennzeichnet, dass** die Anbindung der modifizierten Nukleinsäure-Oligomere an die leitfähige Oberfläche kovalent oder durch Chemi- oder Physisorption erfolgt.

46. Modifizierte leitfähige Oberfläche nach den Ansprüchen 37 bis 45, **dadurch gekennzeichnet, dass** eine der Phosphorsäure-, Carbonsäure-, Amin- oder eine Zucker-Gruppe des Nukleinsäure-Oligomer-Rückgrats kovalent oder durch Chemi- oder Physisorption an die leitfähige Oberfläche angebunden ist.

47. Modifizierte leitfähige Oberfläche nach Anspruch 46, **dadurch gekennzeichnet, dass** eine Zucker-Hydroxy-Gruppe des Nukleinsäure-Oligomer-Rückgrats kovalent oder durch Chemi- oder Physisorption an die leitfähige Oberfläche angebunden ist.

48. Modifizierte leitfähige Oberfläche nach den Ansprüchen 37 bis 45, **dadurch gekennzeichnet, dass** eine Thiol-Gruppe, eine Hydroxy-Gruppe, eine Carbonsäure-Gruppe oder eine Amin-Gruppe einer modifizierten Base des Nukleinsäure-Oligomers kovalent oder durch Chemi- oder Physisorption an die leitfähige Oberfläche angebunden ist.

49. Modifizierte leitfähige Oberfläche nach den Ansprüchen 46 bis 48, **dadurch gekennzeichnet, dass** das modifizierte Nukleinsäure-Oligomer über eine Gruppe am Ende des Nukleinsäure-Oligomer-Rückgrats oder über eine Gruppe einer endständigen, modifizierten Base an die leitfähige Oberfläche gebunden ist.

50. Modifizierte leitfähige Oberfläche nach den Ansprüchen 37 bis 49, **dadurch gekennzeichnet, dass** an die leitfähige Oberfläche verzweigte oder unverzweigte Molekülteile beliebiger Zusammensetzung und Kettenlänge kovalent oder durch Chemi- oder Physisorption angebunden sind und die modifizierten Nukleinsäure-Oligomere kovalent an diese Molekülteile angebunden sind.

51. Modifizierte leitfähige Oberfläche nach Anspruch 50, **dadurch gekennzeichnet. dass** die kürzeste durchgehende Verbindung zwischen der leitfähigen Oberfläche und dem Nukleinsäure-Oligomer ein verzweigtes oder unverzweigtes Molekülteil mit einer Kettenlänge von 1 - 20 Atomen ist.

52. Modifizierte leitfähige Oberfläche nach Anspruch 51, **dadurch gekennzeichnet, dass** die kürzeste durchgehende Verbindung zwischen der leitfähigen Oberfläche und dem Nukleinsäure-Oligomer ein verzweigtes oder unverzweigtes Molekülteil mit einer Kettenlänge von 1 - 12 Atomen, ist.

53. Modifizierte leitfähige Oberfläche nach den Ansprüchen 50 bis 52, **dadurch gekennzeichnet, dass** der verzweigte oder unverzweigte Molekülteil an eine Phosphorsäure-Gruppe, eine Carbonsäure-Gruppe, eine Amin-Gruppe oder eine Zucker-Gruppe des Nukleinsäure-Oligomer-Rückgrats oder eine Thiol-, Hydroxy-, Carbonsäure- oder Amin-Gruppe einer modifizierten Base des Nukleinsäure-Oligomers angebunden ist.

54. Modifizierte leitfähige Oberfläche nach Anspruch 53, **dadurch gekennzeichnet, dass** der verzweigte oder unverzweigte Molekülteil an eine Zucker-Hydroxy-Gruppe des Nukleinsäure-Oligomer-Rückgrats angebunden ist.

55. Modifizierte leitfähige Oberfläche nach Anspruch 53 oder 54, **dadurch gekennzeichnet, dass** der verzweigte oder unverzweigte Molekülteil an eine Phosphorsäure-, Zucker-Hydroxy-, Carbonsäure- oder Amin-Gruppe am Ende des Nukleinsäure Oligomer-Rückgrats oder an eine Thiol-, Hydroxy-, Carbonsäure- oder Amin-Gruppe einer endständigen, modifizierten Base gebunden ist.

56. Modifizierte leitfähige Oberfläche nach einem der Ansprüche 37 bis 55, **dadurch gekennzeichnet, dass** jeweils überwiegend eine Art von modifizierten Nukleinsäure-Oligomeren in einem räumlich begrenzten Bereich der leitfähigen Oberfläche angebunden ist.

57. Modifizierte leitfähige Oberfläche nach Anspruch 56, **dadurch gekennzeichnet, dass** jeweils ausschließlich eine Art von modifizierten Nukleinsäure-Oligomeren in einem räumlich begrenzten Bereich der leitfähigen Oberfläche angebunden ist.

58. Verfahren zur Herstellung einer modifizierten leitfähigen Oberfläche wie in den Ansprüchen 37 bis 57 definiert, **dadurch gekennzeichnet, dass** wenigstens eine Art von modifizierten Nukleinsäure-Oligomeren auf eine leitfähige Oberfläche aufgebracht wird.

59. Verfahren zur Herstellung einer modifizierten leitfähigen Oberfläche wie in den Ansprüchen 37 bis 57 definiert, **dadurch gekennzeichnet, dass** wenigstens eine Art von Nukleinsäure-Oligomeren auf eine leitfähige Oberfläche aufgebracht wird und anschließend eine Modifikation der Nukleinsäure-Oligomere durch ein Verfahren gemäß den Ansprüchen 28 bis 36 durchgeführt wird.

60. Verfahren zur Herstellung einer modifizierten leitfähigen Oberfläche nach Anspruch 58 oder 59, **dadurch gekennzeichnet, dass** die Nukleinsäure-Oligomere oder die modifizierten Nukleinsäure-Oligomere mit dem dazu jeweils komplementären Nukleinsäure-Oligomerstrang hybridisiert werden und in Form des Doppelstranghybrids auf die leitfähige Oberfläche aufgebracht werden.

61. Verfahren zur Herstellung einer modifizierten leitfähigen Oberfläche nach den Ansprüchen 58 oder 59, **dadurch gekennzeichnet, dass** das Nukleinsäure-Oligomer oder das modifizierte Nukleinsäure-Oligomer in Gegenwart von weiteren chemischen Verbindungen, die ebenfalls an die leitfähige Oberfläche angebunden werden, auf die leitfähige Oberfläche aufgebracht wird.

62. Verfahren zur elektrochemischen Detektion von Nukleinsäure-Oligomer-Hybridisierungsereignissen, **dadurch gekennzeichnet, dass** wenigstens eine modifizierte leitfähige Oberfläche, wie in den Ansprüchen 37 bis 57 definiert, mit Nukleinsäure-Oligomeren in Kontakt gebracht wird und anschließend eine Detektion der elektrischen Kommunikation zwischen der redoxaktiven Einheit und der leitfähigen Oberfläche erfolgt.

63. Verfahren nach Anspruch 62, **dadurch gekennzeichnet, dass** die Detektion cyclovoltametrisch, amperometrisch oder durch Leitfähigkeitsmessung erfolgt.

64. Verfahren nach den Ansprüchen 62 oder 63, **dadurch gekennzeichnet, dass** die elektrochemische Detektion durch photoinduzierte Ladungstrennung in der über ein Nukleinsäure-Oligomer an die leitfähige Oberfläche angebundenen photoinduzierbar redoxaktiven Einheit gestartet wird.

65. Verfahren nach Anspruch 64, **dadurch gekennzeichnet, dass** die Lichteinstrahlung zur photoinduzierten Ladungstrennung in der über ein Nukleinsäure-Oligomer an die leitfähige Oberfläche angebundenen photoinduzierbar redoxaktiven Einheit auf einen Bereich der leitfähigen Oberfläche mit wenigstens einer Art von modifizierten Nukleinsäure-Oligomeren begrenzt wird.

66. Verfahren nach den Ansprüchen 64 oder 65, **dadurch gekennzeichnet, dass** das nach Einstrahlen von Licht bestimmter oder beliebiger Wellenlänge entstandene oxidierte Elektron-Donor-Molekül der photoinduzierbar redoxaktiven Einheit durch eine geeignete, freie, nicht an das Nukleinsäure-Oligomer gebundene, aber mit dem Nukleinsäure-Oligomer in Kontakt stehende, redoxaktive Substanz, re-reduziert, also in seinen ursprünglichen vor der Lichteinstrahlung vorhandenen Zustand, zurückversetzt wird.

67. Verfahren nach den Ansprüchen 64 oder 65, **dadurch gekennzeichnet, dass** das nach Einstrahlen von Licht bestimmter oder beliebiger Wellenlänge entstandene reduzierte Elektron-Akzeptor-Molekül der photoinduzierbar redoxaktiven Einheit durch eine geeignete, freie, nicht an das Nukleinsäure-Oligomer gebundene, aber mit dem Nukleinsäure-Oligomer in Kontakt stehende, redoxaktive Substanz, re-oxidiert, also in seinen ursprünglichen vor der Lichteinstrahlung vorhandenen Zustand, zurückversetzt wird.

68. Verfahren zur elektrochemischen Detektion nach den Ansprüchen 62 oder 63, **dadurch gekennzeichnet, dass** die elektrochemische Detektion durch eine freie redoxaktive Substanz, durch die ein thermischer Ladungstransfer auf die redoxaktive Einheit stattfindet, ermöglicht wird.

69. Verfahren nach den Ansprüchen 67 oder 68, **dadurch gekennzeichnet, dass** die freie, nicht an ein Nukleinsäure-Oligomer gebundene, aber mit dem Nukleinsäure-Oligomer in Kontakt stehende, redoxaktive Substanz bei einem Potential ϕ selektiv oxidierbar und reduzierbar ist, wobei ϕ der Bedingung 2,0 V ≥ ϕ ≥ - 2 V, gemessen gegen Normalwasserstoffelektrode, genügt.

70. Verfahren nach den Ansprüchen 66 bis 69, **dadurch gekennzeichnet, dass** die freie, nicht an ein Nukleinsäure-Oligomer gebundene, aber mit dem Nukleinsäure-Oligomer in Kontakt stehende, redoxaktive Substanz ein freies Chinon, ein freier Hexacyanoferrat(II)-Komplex, ein freies Natriumascorbat, ein freier Ru(II)hexamin-Komplex oder ein freies redoxaktives Protein ist.

71. Verfahren nach Anspruch 70, **dadurch gekennzeichnet, dass** die freie, nicht an ein Nukleinsäure-Oligomer gebundene, aber mit dem Nukleinsäure-Oligomer in Kontakt stehende, redoxaktive Substanz ein freies Cytochrom ist.

## Claims

1. A nucleic acid oligomer modified by covalently attaching a redox-active moiety, **characterized in that** the redox-active moiety comprises at least one electron-donor molecule and at least one electron-acceptor molecule, the electron-donor molecule and electron-acceptor molecule not being joined with one another by nucleic acid oligomers.

2. The modified nucleic acid oligomer according to claim 1, **characterized in that** the redox-active moiety comprises at least one redox-active, linked, at least bimolecular electron-donor/electron-acceptor complex, at least one electron-donor molecule of the redox-active moiety and at least one electron-acceptor molecule of the redox-active moiety being joined with one another via one or more bonds.

3. The modified nucleic acid oligomer according to claim 2, **characterized in that** the bonds are covalent bonds.

4. The modified nucleic acid oligomer according to claim 1, **characterized in that** the redox-active moiety comprises at least one redox-active, linked, at least bimolecular electron-donor/electron-acceptor complex, at least one electron-donor molecule of the redox-active moiety and at least one electron-acceptor molecule of the redox-active moiety being covalently joined via one or more branched or linear molecular moieties of any composition and chain length.

5. The modified nucleic acid oligomer according to claim 4, **characterized in that** the branched or linear molecular moieties have a chain length of 1 - 20 atoms.

6. The modified nucleic acid oligomer according to claim 5, **characterized in that** the branched or linear molecular moieties have a chain length of 1 - 14 atoms.

7. The modified nucleic acid oligomer according to one of the preceding claims, **characterized in that** the redox-active moiety additionally comprises one or more macromolecules.

8. The modified nucleic acid oligomer according to one of the preceding claims, wherein the redox-active moiety is the native or modified reaction center of photosynthesizing organisms.

9. The modified nucleic acid oligomer according to claim 8, wherein the redox-active moiety is the native or modified reaction center of photosynthesizing bacteria.

10. The modified nucleic acid oligomer according to one of claims 1 through 7, **characterized in that** at least one of the electron-donor molecules and electron-acceptor molecules is a pigment.

11. The modified nucleic acid oligomer according to claim 10, **characterized in that** the pigment is a flavin, a (metallo)porphyrin, a (metallo)chlorophyll, a (metallo)bacteriochlorophyll, or a derivative of these pigments.

12. The modified nucleic acid oligomer according to one of claims 1 through 7, **characterized in that** at least one of the electron donor molecules and electron acceptor molecules is a nicotinamide or a quinone.

13. The modified nucleic acid oligomer according to claim 12, **characterized in that** the quinone is a pyrroloquinoline quinone (PQQ), a 1,2-benzoquinone, a 1,4-benzoquinone, a 1,2-naphthoquinone, a 1,4-naphthoquinone, a 9,10-anthraquinone, or one of their derivatives.

14. The modified nucleic acid oligomer according to one of claims 1 through 7, **characterized in that** at least one of the electron-donor molecules and electron-acceptor molecules is a charge transfer complex.

15. The modified nucleic acid oligomer according to claim 14, **characterized in that** the charge transfer complex is a transition metal complex.

16. The modified nucleic acid oligomer according to claim 15, **characterized in that** the charge transfer complex is a Ru(II), Cr(III), Fe(II), Os(II), or Co(II) complex.

17. The modified nucleic acid oligomer according to one of the preceding claims, **characterized in that** the modified nucleic acid oligomer can sequence-specifically bind single-strand DNA, RNA, and/or PNA.

18. The modified nucleic acid oligomer according to claim 17, **characterized in that** the modified nucleic acid oligomer is a deoxyribonucleic acid oligomer, a ribonucleic acid oligomer, or a peptide nucleic acid oligomer.

19. The modified nucleic acid oligomer according to one of the preceding claims, **characterized in that** the redox-active moiety is covalently bound to one of the phosphoric-acid groups, to one of the carboxylic-acid groups, to one of the amine groups, or to a sugar of the nucleic acid oligomer backbone.

20. The modified nucleic acid oligomer according to one of the preceding claims, **characterized in that** the redox-active moiety is covalently bound to a sugar-hydroxy group of the nucleic acid oligomer backbone.

21. The modified nucleic acid oligomer according to one of claims 1 through 18, **characterized in that** the redox-active moiety is covalently attached to a thiol group, a hydroxyl group, a carboxylic-acid group, or an amine group of a modified base of the nucleic acid oligomer.

22. The modified nucleic acid oligomer according to claim 21, **characterized in that** the reactive thiol, hydroxyl, carboxylic-acid, or amine group of the base is covalently bound to the base via a branched or linear molecular moiety of any composition and chain length, the shortest continuous link between the thiol, hydroxyl, carboxylic-acid, or amine group and the base being a branched or linear molecular moiety having a chain length of 1 - 20 atoms.

23. The modified nucleic acid oligomer according to claim 22, **characterized in that** the shortest continuous link between the thiol, hydroxyl, carboxylic-acid, or amine group and the base is a branched or linear molecular moiety having a chain length of 1 - 14 atoms.

24. The modified nucleic acid oligomer according to one of claims 19 through 22, **characterized in that** the redox-active moiety is attached to an end of the nucleic acid oligomer backbone or to a terminal modified base.

25. The modified nucleic acid oligomer according to one of the preceding claims, **characterized in that** the redox-active moiety is a photoinducibly redox-active moiety.

26. The modified nucleic acid oligomer according to one of claims 1 through 24, **characterized in that** the redox-active moiety is a chemically-inducibly redox-active moiety.

27. The modified nucleic acid oligomer according to one of the preceding claims, **characterized in that** multiple redox-active moieties are attached to the nucleic acid oligomer.

28. A method of producing a modified nucleic acid oligomer as defined in one of the preceding claims, **characterized in that** a redox-active moiety is covalently attached to a nucleic acid oligomer.

29. The method of producing a modified nucleic acid oligomer according to claim 28, **characterized in that** the redox-active moiety is attached to a nucleic acid oligomer by covalently attaching at least one electron-donor molecule.

30. The method of producing a modified nucleic acid oligomer according to claim 28, **characterized in that** the redox-active moiety is attached to a nucleic acid oligomer by covalently attaching at least one electron-acceptor molecule.

31. The method of producing a modified nucleic acid oligomer according to claim 28, **characterized in that** the redox-active moiety is attached to a nucleic acid oligomer by covalently attaching at least one macromolecule or by covalently attaching at least one protein.

32. The method of producing a modified nucleic acid oligomer according to claims 29 through 31, **characterized in that** the redox-active moiety is completed by adding at least one component selected from the group consisting of electron-acceptor molecules, electron-donor molecules, macromolecules, and proteins.

33. The method of producing a modified nucleic acid oligomer according to one of claims 28 through 32, **characterized in that** the nucleic acid oligomer is bound to the redox-active moiety by one or more amidations with amine or acid groups of the redox-active moiety, by one or more esterifications with alcohol or acid groups of the redox-active moiety, by thioester formation with thioalcohol or acid groups of the redox-active moiety, or by condensation of one or more amine groups of the nucleic acid oligomer with aldehyde groups of the redox-active moiety and subsequent reduction of the resultant carbon-nitrogen double bond.

34. The method of producing a modified nucleic acid oligomer according to one of claims 28 through 33, **characterized in that** at least one branched or linear molecular moiety of any composition and chain length is covalently attached to the redox-active moiety and the branched or linear molecular moiety has a reactive amine, hydroxyl, thiol, acid, or aldehyde group for covalent attachment to a nucleic acid oligomer.

35. The method of producing a modified nucleic acid oligomer according to claim 34, **characterized in that** the shortest continuous link between the nucleic acid oligomer and the redox-active moiety is a branched or linear molecular moiety having a chain length of 1 - 20 atoms.

36. The method of producing a modified nucleic acid oligomer according to claim 35, **characterized in that** the shortest continuous link between the nucleic acid oligomer and the redox-active moiety is a branched or linear molecular moiety having a chain length of 1 - 14 atoms.

37. A modified conductive surface, **characterized in that** at least one type of modified nucleic acid oligomer according to one of claims 1 through 27 is attached to a conductive surface.

38. The modified conductive surface according to claim 37, **characterized in that** the surface consists of a metal or a metal alloy.

39. The modified conductive surface according to claim 38, **characterized in that** the surface consists of a metal selected from the group: platinum, palladium, gold, cadmium, mercury, nickel, zinc, carbon, silver, copper, iron, lead, aluminum, and manganese.

40. The modified conductive surface according to claim 37, **characterized in that** the surface consists of a semiconductor.

41. The modified conductive surface according to claim 38, **characterized in that** the surface consists of a semiconductor selected from the group: carbon, silicon, germanium, and tin.

42. The modified conductive surface according to claim 37, **characterized in that** the surface consists of a binary compound of the elements of groups 14 and 16, a binary compound of the elements of groups 13 and 15, a binary compound of the elements of groups 15 and 16, or a binary compound of the elements of groups 11 and 17.

43. The modified conductive surface according to claim 42, **characterized in that** the surface consists of a Cu(I) halide or an Ag(I) halide.

44. The modified conductive surface according to claim 37, **characterized in that** the surface consists of a ternary compound of the elements of groups 11, 13, and 16, or a ternary compound of the elements of groups 12, 13, and 16.

45. The modified conductive surface according to claims 37 through 44, **characterized in that** the attachment of the modified nucleic acid oligomers to the conductive surface occurs covalently or by chemisorption or physisorption.

46. The modified conductive surface according to claims 37 through 45, **characterized in that** one of the phosphoric-acid, carboxylic-acid or amine groups or a sugar group of the nucleic acid oligomer backbone is attached, covalently or by chemisorption or physisorption, to the conductive surface.

47. The modified conductive surface according to claim 46, **characterized in that** a sugar-hydroxyl group of the nucleic acid oligomer backbone is attached, covalently or by chemisorption or physisorption, to the conductive surface.

48. The modified conductive surface according to claims 37 through 45, **characterized in that** a thiol group, a hydroxyl group, a carboxylic-acid group, or an amine group of a modified base of the nucleic acid oligomer is attached, covalently or by chemisorption or physisorption, to the conductive surface.

49. The modified conductive surface according to claims 46 through 48, **characterized in that** the modified nucleic acid oligomer is bound to the conductive surface via a group at the end of the nucleic acid oligomer backbone or via a group of a terminal, modified base.

50. The modified conductive surface according to claims 37 through 49, **characterized in that** branched or linear molecular moieties of any composition and chain length are attached, covalently or by chemisorption or physisorption, to the conductive surface and the modified nucleic acid oligomers are covalently attached to these molecular moieties.

51. The modified conductive surface according to claim 50, **characterized in that** the shortest continuous link between the conductive surface and the nucleic acid oligomer is a branched or linear molecular moiety having a chain length of 1 - 20 atoms.

52. The modified conductive surface according to claim 51, **characterized in that** the shortest continuous link between the conductive surface and the nucleic acid oligomer is a branched or linear molecular moiety having a chain length of 1 - 12 atoms.

53. The modified conductive surface according to claims 50 through 52, **characterized in that** the branched or linear molecular moiety is attached to a phosphoric-acid group, a carboxylic-acid group, an amine group, or a sugar group of the nucleic acid oligomer backbone or a thiol, hydroxyl, carboxylic-acid, or amine group of a modified base of the nucleic acid oligomer.

54. The modified nucleic acid oligomer according to claim 53, **characterized in that** the branched or linear molecular moiety is attached to a sugar-hydroxy group of the nucleic acid oligomer backbone.

55. The modified conductive surface according to claim 53 or 54, **characterized in that** the branched or linear molecular moiety is bound to a phosphoric-acid, sugar-hydroxy, carboxylic-acid, or amine group at the end of the nucleic acid oligomer backbone or to a thiol, hydroxyl, carboxylic acid, or amine group of a terminal, modified base.

56. The modified conductive surface according to one of claims 37 through 55, **characterized in that** predominantly one type of modified nucleic acid oligomer each is attached in a spatially delimited area of the conductive surface.

57. The modified conductive surface according to claim 56, **characterized in that** only one type of modified nucleic acid oligomer each is attached in a spatially delimited area of the conductive surface.

58. A method of producing a modified conductive surface as defined in claims 37 through 57, **characterized in that** at least one type of modified nucleic acid oligomer is applied to a conductive surface.

59. The method of producing a modified conductive surface as defined in claims 37 through 57, **characterized in that** at least one type of nucleic acid oligomer is applied to a conductive surface and, subsequently, a modification of the nucleic acid oligomers is carried out using a method according to claims 28 through 36.

60. The method of producing a modified conductive surface according to claim 58 or 59, **characterized in that** the nucleic acid oligomers or the modified nucleic acid oligomers are hybridized with the respective complementary nucleic acid oligomer strand and applied to the conductive surface in the form of the double-strand hybrid.

61. The method of producing a modified conductive surface according to claim 58 or 59, **characterized in that** the nucleic acid oligomer or the modified nucleic acid oligomer is applied to the conductive surface in the presence of further chemical compounds that are likewise attached to the conductive surface.

62. A method of electrochemically detecting oligomer hybridization events, **characterized in that** at least one modified conductive surface as defined in claims 37 through 57 is brought into contact with nucleic acid oligomers and, subsequently, detection of the electrical communication between the redox-active moiety and the conductive surface takes place.

63. The method according to claim 62, **characterized in that** detection takes place by cyclic voltammetry, amperometry, or conductivity measurement.

64. The method according to claim 62 or 63, **characterized in that** electrochemical detection is initiated by photoinduced charge separation in the photoinducibly redox-active moiety attached to the conductive surface via a nucleic acid oligomer.

65. The method according to claim 64, **characterized in that** the light irradiation for photoinduced charge separation in the photoinducibly redox-active moiety attached to the conductive surface via a nucleic acid oligomer is limited to an area of the conductive surface having at least one type of modified nucleic acid oligomer.

66. The method according to claim 64 or 65, **characterized in that** the photoinducibly redox-active moiety's oxidized electron-donor molecule resulting from irradiation with light of a specific or any given wavelength is rereduced by a suitable free redox-active substance that is not bound to but in contact with the nucleic acid oligomer, i.e. it is restored to the state it was originally in prior to light irradiation.

67. The method according to claim 64 or 65, **characterized in that** the photoinducibly redox-active moiety's reduced electron-acceptor molecule resulting from irradiation with light of a specific or any given wavelength is reoxidized by a suitable free redox-active substance that is not bound to but in contact with the nucleic acid oligomer, i.e. it is restored to the state it was originally in prior to light irradiation.

68. The method of electrochemical detection according to claim 62 or 63, **characterized in that** the electrochemical detection is facilitated by a free redox-active substance that effectuates a thermal charge transfer to the redox-active moiety.

69. The method according to claim 67 or 68, **characterized in that** the free redox-active substance that is not bound to but in contact with the nucleic acid oligomer is selectively oxidizable and reducible at a potential ϕ, where ϕ satisfies the condition 2.0 V ≥ ϕ ≥ - 2.0 V, measured against normal hydrogen electrode.

70. The method according to claims 66 through 69, **characterized in that** the free redox-active substance that is not bound to but in contact with the nucleic acid oligomer is a free quinone, a free hexacyanoferrate(II) complex, a free sodium ascorbate, a free Ru(II)hexamine complex, or a free redox-active protein.

71. The method according to claim 70, **characterized in that** the free redox-active substance that is not bound to but in contact with the nucleic acid oligomer is a free cytochrome.

## Revendications

1. Oligomère d'acide nucléique modifié par liaison covalente d'une partie ayant une activité rédox, **caractérisé en ce que** la partie ayant une activité rédox comprend au moins une molécule électrodonneuse et au moins une molécule électroacceptrice, la molécule électrodonneuse et la molécule électroacceptrice n'étant pas liées l'une à l'autre par des oligomères d'acide nucléique.

2. Oligomère d'acide nucléique modifié suivant la revendication 1, **caractérisé en ce que** la partie ayant une activité rédox comprend au moins un complexe au moins bimoléculaire électrodonneur-électroaccepteur, relié, ayant une activité rédox, au moins une molécule électrodonneuse de la partie ayant une activité rédox et au moins une molécule électroacceptrice de la partie ayant une activité rédox étant liées l'une à l'autre par l'intermédiaire d'une ou de plusieurs liaisons.

3. Oligomère d'acide nucléique modifié suivant la revendication 2, **caractérisé en ce que** les liaisons sont des liaisons covalentes.

4. Oligomère d'acide nucléique modifié suivant la revendication 1, **caractérisé en ce que** la partie ayant une activité rédox comprend au moins un complexe au moins bimoléculaire électrodonneur-électroaccepteur, relié, ayant une activité rédox, au moins une molécule électrodonneuse de la partie ayant une activité rédox et au moins une molécule électroacceptrice de la partie ayant une activité rédox étant liées de façon covalente l'une à l'autre par l'intermédiaire d'une ou de plusieurs parties moléculaires linéaires ou ramifiées de composition et longueur de chaîne quelconque.

5. Oligomère d'acide nucléique modifié suivant la revendication 4, **caractérisé en ce que** les parties moléculaires linéaires ou ramifiées ont une longueur de chaîne de 1 à 20 atomes.

6. Oligomère d'acide nucléique modifié suivant la revendication 5, **caractérisé en ce que** les parties moléculaires linéaires ou ramifiées ont une longueur de chaîne de 1 à 14 atomes.

7. Oligomère d'acide nucléique modifié suivant l'une quelconque des revendications précédentes, **caractérisé en ce que** la partie ayant une activité rédox comprend de plus une ou plusieurs macromolécules.

8. Oligomère d'acide nucléique modifié suivant l'une quelconque des revendications précédentes, **caractérisé en ce que** la partie ayant une activité rédox est le centre de réaction naturel ou modifié d'organismes ayant une activité de photosynthèse.

9. Oligomère d'acide nucléique modifié suivant la revendication 8, **caractérisé en ce que** la partie ayant une activité rédox est le centre de réaction naturel ou modifié de bactéries ayant une activité de photosynthèse.

10. Oligomère d'acide nucléique modifié suivant l'une quelconque des revendications 1 à 7, **caractérisé en ce qu'**au moins l'une des molécules électrodonneuses et électroacceptrices est un pigment.

11. Oligomère d'acide nucléique modifié suivant la revendication 10, **caractérisé en ce que** le pigment est une flavine, une (métallo)-porphyrine, une (métallo)-chlorophylle, une (métallo)-bactériochlorophylle, ou un dérivé de ces pigments.

12. Oligomère d'acide nucléique modifié suivant l'une quelconque des revendications 1 à 7, **caractérisé en ce qu'**au moins l'une des molécules électrodonneuses et électroacceptrices est un nicotinamide ou une quinone.

13. Oligomère d'acide nucléique modifié suivant la revendication 12, **caractérisé en ce que** la quinone est une pyrroloquinoléine quinone (PQQ), une 1,2-benzoquinone, une 1,4-benzoquinone, une 1,2-naphtoquinone, une 1,4-naphtoquinone, une 9,10-anthraquinone, ou l'un de leurs dérivés.

14. Oligomère d'acide nucléique modifié suivant l'une quelconque des revendications 1 à 7, **caractérisé en ce qu'**au moins l'une des molécules électrodonneuses et électroacceptrices est un complexe de transfert de charge.

15. Oligomère d'acide nucléique modifié suivant la revendication 14, **caractérisé en ce que** le complexe de transfert de charge est un complexe de métal de transition.

16. Oligomère d'acide nucléique modifié suivant la revendication 15, **caractérisé en ce que** le complexe de transfert de charge est un complexe de Ru(II), Cr(III), Fe(II), Os(II) ou Co(II).

17. Oligomère d'acide nucléique modifié suivant l'une quelconque des revendications précédentes, **caractérisé en ce que** l'oligomère d'acide nucléique modifié peut fixer de façon spécifique d'une séquence un ADN, ARN et/ou ANP monocaténaire.

18. Oligomère d'acide nucléique modifié suivant la revendication 17, **caractérisé en ce que** l'oligomère d'acide nucléique modifié est un oligomère d'acide désoxyribonucléique, un oligomère d'acide ribonucléique, ou un oligomère d'acide nucléique peptidique.

19. Oligomère d'acide nucléique modifié suivant l'une quelconque des revendications précédentes, **caractérisé en ce que** la partie ayant une activité rédox est liée de façon covalente à l'un des groupes acide phosphorique, à l'un des groupes acide carboxylique, à l'un des groupes amine, ou à un sucre du squelette d'oligomère d'acide nucléique.

20. Oligomère d'acide nucléique modifié suivant l'une quelconque des revendications précédentes, **caractérisé en ce que** la partie ayant une activité rédox est liée de façon covalente à un groupe hydroxy de sucre du squelette d'oligomère d'acide nucléique.

21. Oligomère d'acide nucléique modifié suivant l'une quelconque des revendications 1 à 18, **caractérisé en ce que** la partie ayant une activité rédox est liée par covalence à un groupe thiol, un groupe hydroxy, un groupe acide carboxylique, ou un groupe amine d'une base modifiée de l'oligomère d'acide nucléique.

22. Oligomère d'acide nucléique modifié suivant la revendication 21, **caractérisé en ce que** le groupe réactif thiol, hydroxy, acide carboxylique ou amine de la base est lié de façon covalente à la base par une partie moléculaire ramifiée ou linéaire de composition et longueur de chaîne quelconque, la liaison continue la plus courte entre le groupe thiol, hydroxy, acide carboxylique ou amine et la base étant une partie moléculaire linéaire ou ramifiée ayant une longueur de chaîne de 1 à 20 atomes.

23. Oligomère d'acide nucléique modifié suivant la revendication 22, **caractérisé en ce que** la liaison continue la plus courte entre le groupe thiol, hydroxy, acide carboxylique ou amine et la base est une partie moléculaire linéaire ou ramifiée ayant une longueur de chaîne de 1 à 14 atomes.

24. Oligomère d'acide nucléique modifié suivant l'une quelconque des revendications 19 à 22, **caractérisé en ce que** la partie ayant une activité rédox est attachée à une extrémité du squelette de l'oligomère d'acide nucléique ou à une base modifiée terminale.

25. Oligomère d'acide nucléique modifié suivant l'une quelconque des revendications précédentes, **caractérisé en ce que** la partie ayant une activité rédox est une partie ayant une activité rédox photoinductible.

26. Oligomère d'acide nucléique modifié suivant l'une quelconque des revendications 1 à 24, **caractérisé en ce que** la partie ayant une activité rédox est une partie ayant une activité rédox inductible par voie chimique.

27. Oligomère d'acide nucléique modifié suivant l'une quelconque des revendications précédentes, **caractérisé en ce que** de multiples parties ayant une activité rédox sont fixées à l'oligomère d'acide nucléique.

28. Procédé pour la production d'un oligomère d'acide nucléique modifié suivant l'une quelconque des revendications précédentes, **caractérisé en ce qu'**une partie ayant une activité rédox est liée de façon covalente à un oligomère d'acide nucléique.

29. Procédé pour la production d'un oligomère d'acide nucléique modifié suivant la revendication 28, **caractérisé en ce que** la partie ayant une activité rédox est liée à un oligomère d'acide nucléique par liaison covalente à au moins une molécule électrodonneuse.

30. Procédé pour la production d'un oligomère d'acide nucléique modifié suivant la revendication 28, **caractérisé en ce que** la partie ayant une activité rédox est liée à un oligomère d'acide nucléique par liaison covalente à au moins une molécule électroacceptrice.

31. Procédé pour la production d'un oligomère d'acide nucléique modifié suivant la revendication 28, **caractérisé en ce que** la partie ayant une activité rédox est liée à un oligomère d'acide nucléique par liaison covalente à au moins une macromolécule ou par liaison covalente à au moins une protéine.

32. Procédé pour la production d'un oligomère d'acide nucléique modifié suivant les revendications 29 à 31, **caractérisé en ce que** la partie ayant une activité rédox est complétée par l'addition d'au moins un composant choisi dans le groupe consistant en molécules électroacceptrices, molécules électrodonneuses, macromolécules et protéines.

33. Procédé pour la production d'un oligomère d'acide nucléique modifié suivant l'une quelconque des revendications 28 à 32, **caractérisé en ce que** l'oligomère d'acide nucléique est lié à la partie ayant une activité rédox par une ou plusieurs amidations avec des groupes amine ou acide de la partie ayant une activité rédox, par une ou plusieurs estérifications avec des groupes alcool ou acide de la partie ayant une activité rédox, par formation d'un thioester avec des groupes thioalcool ou acide de la partie ayant une activité rédox, ou par condensation d'un ou de plusieurs groupes amine de l'oligomère d'acide nucléique avec des groupes aldéhyde de la partie ayant une activité rédox et réduction consécutive de la double liaison carbone-azote résultante.

34. Procédé pour la production d'un oligomère d'acide nucléique modifié suivant l'une quelconque des revendications 28 à 33, **caractérisé en ce qu'**au moins une partie moléculaire linéaire ou ramifiée de composition et longueur de chaîne quelconque est liée de façon covalente à la partie ayant une activité rédox, et **en ce que** la partie moléculaire linéaire ou ramifiée possède un groupe réactif amine, hydroxy, thiol, acide ou aldéhyde pour la fixation par covalence à un oligomère d'acide nucléique.

35. Procédé pour la production d'un oligomère d'acide nucléique modifié suivant la revendication 34, **caractérisé en ce que** la liaison continue la plus courte entre l'oligomère d'acide nucléique et la partie ayant une activité rédox est une partie moléculaire linéaire ou ramifiée ayant une longueur de chaîne de 1 à 20 atomes.

36. Procédé pour la production d'un oligomère d'acide nucléique modifié suivant la revendication 35, **caractérisé en ce que** la liaison continue la plus courte entre l'oligomère d'acide nucléique et la partie ayant une activité rédox est une partie moléculaire linéaire ou ramifiée ayant une longueur de chaîne de 1 à 14 atomes.

37. Surface conductrice modifiée, **caractérisée en ce qu'**au moins un type d'oligomère d'acide nucléique modifié suivant l'une quelconque des revendications 1 à 27, est attaché à une surface conductrice.

38. Surface conductrice modifiée suivant la revendication 37, **caractérisée en ce que** la surface consiste en un métal ou en un alliage métallique.

39. Surface conductrice modifiée suivant la revendication 38, **caractérisée en ce que** la surface consiste en un métal choisi dans le groupe consistant en platine, palladium, or, cadmium, mercure, nickel, zinc, carbone, argent, cuivre, fer, plomb, aluminium et manganèse.

40. Surface conductrice modifiée suivant la revendication 37, **caractérisée en ce que** la surface consiste en un semiconducteur.

41. Surface conductrice modifiée suivant la revendication 38, **caractérisée en ce que** la surface consiste en un semiconducteur choisi dans le groupe consistant en carbone, silicium, germanium et étain.

42. Surface conductrice modifiée suivant la revendication 37, **caractérisée en ce que** la surface consiste en un composé binaire des éléments des groupes 14 et 16, un composé binaire des éléments des groupes 13 à 15, un composé binaire des éléments des groupes 15 et 16 ou un composé binaire des éléments des groupes 11 et 17.

43. Surface conductrice modifiée suivant la revendication 42, **caractérisée en ce que** la surface consiste en un halogénure de Cu(I) ou un halogénure d'Ag(I).

44. Surface conductrice modifiée suivant la revendication 37, **caractérisée en ce que** la surface consiste en un composé ternaire des éléments des groupes 11, 13 et 16 ou un composé ternaire des éléments des groupes 12, 13 et 16.

45. Surface conductrice modifiée suivant les revendications 37 à 44, **caractérisée en ce que** la liaison des oligomères d'acide nucléique modifiés à la surface conductrice a lieu par covalence ou par chimisorption ou physisorption.

46. Surface conductrice modifiée suivant les revendications 37 à 45, **caractérisée en ce que** l'un des groupes acide phosphorique, acide carboxylique ou amine ou un groupe sucre du squelette d'oligomère d'acide nucléique est fixé, par covalence ou par chimisorption ou physisorption à la surface conductrice.

47. Surface conductrice modifiée suivant la revendication 46, **caractérisée en ce qu'**un groupe hydroxy de sucre du squelette d'oligomère d'acide nucléique est fixé, par covalence ou par chimisorption ou physisorption, à la surface conductrice.

48. Surface conductrice modifiée suivant les revendications 37 à 45, **caractérisée en ce qu'**un groupe thiol, un groupe hydroxy, un groupe acide carboxylique, ou un groupe amine d'une base modifiée de l'oligomère d'acide nucléique est fixé, par covalence ou par chimisorption ou physisorption, à la surface conductrice.

49. Surface conductrice modifiée suivant les revendications 46 à 48, **caractérisée en ce que** l'oligomère d'acide nucléique modifié est lié à la surface conductrice par l'intermédiaire d'un groupe situé à l'extrémité du squelette d'oligomère d'acide nucléique ou par l'intermédiaire d'un groupe d'une base modifiée terminale.

50. Surface conductrice modifiée suivant les revendications 37 à 49, **caractérisée en ce que** des parties moléculaires linéaires ou ramifiées de composition et longueur de chaîne quelconque sont fixées par covalence, ou par chimisorption ou physisorption, à la surface conductrice et **en ce que** les oligomères d'acide nucléique modifiés sont liés par covalence à ces parties moléculaires.

51. Surface conductrice modifiée suivant la revendication 50, **caractérisée en ce que** la liaison continue la plus courte entre la surface conductrice et l'oligomère d'acide nucléique est une partie moléculaire linéaire ou ramifiée ayant une longueur de chaîne de 1 à 20 atomes.

52. Surface conductrice modifiée suivant la revendication 51, **caractérisée en ce que** la liaison continue la plus courte entre la surface conductrice et l'oligomère d'acide nucléique est une partie moléculaire linéaire ou ramifiée ayant une longueur de chaîne de 1 à 12 atomes.

53. Surface conductrice modifiée suivant les revendications 50 à 52, **caractérisée en ce que** la partie moléculaire linéaire ou ramifiée est fixée à un groupe acide phosphorique, un groupe acide carboxylique, un groupe amine ou un groupe sucre du squelette de l'oligomère d'acide nucléique ou à un groupe thiol, hydroxy, acide carboxylique ou amine d'une base modifiée de l'oligomère d'acide nucléique.

54. Oligomère d'acide nucléique modifié suivant la revendication 53, **caractérisé en ce que** la partie moléculaire linéaire ou ramifiée est fixée à un groupe hydroxy de sucre du squelette d'oligomère d'acide nucléique.

55. Surface conductrice modifiée suivant les revendications 53 ou 54, **caractérisée en ce que** la partie moléculaire linéaire ou ramifiée est liée à un groupe phosphorique, hydroxy de sucre, acide carboxylique ou amine situé à l'extrémité du squelette d'oligomère d'acide nucléique ou à un groupe thiol, hydroxy, acide carboxylique ou amine d'une base modifiée terminale.

56. Surface conductrice modifiée suivant l'une quelconque des revendications 37 à 55, **caractérisée en ce que** principalement chaque type d'oligomère d'acide nucléique modifié est fixé dans une zone délimitée dans l'espace de la surface conductrice.

57. Surface conductrice modifiée suivant la revendication 56, **caractérisée en ce que** seulement un type d'oligomère d'acide nucléique modifié est fixé dans une zone délimitée dans l'espace de la surface conductrice.

58. Procédé pour la production d'une surface conductrice modifiée suivant les revendications 37 à 57, **caractérisé en ce qu'**au moins un type d'oligomère d'acide nucléique modifié est appliqué sur une surface conductrice.

59. Procédé pour la production d'une surface conductrice modifiée suivant les revendications 37 à 57, **caractérisé en ce qu'**au moins un type d'oligomère d'acide nucléique est appliqué à une surface conductrice et qu'ensuite, une modification des oligomères d'acide nucléique est effectuée selon un procédé suivant les revendications 28 à 36.

60. Procédé pour la production d'une surface conductrice modifiée suivant les revendications 58 ou 59, **caractérisé en ce que** les oligomères d'acide nucléique ou les oligomères d'acide nucléique modifiés sont hybridés avec le brin d'oligomère d'acide nucléique complémentaire respectif et appliqués sur la surface conductrice sous la forme de l'hybride bicaténaire.

61. Procédé pour la production d'une surface conductrice modifiée suivant les revendications 58 ou 59, **caractérisé en ce que** l'oligomère d'acide nucléique ou l'oligomère d'acide nucléique modifié est appliqué sur la surface conductrice en présence d'autres composés chimiques qui sont de même fixés à la surface conductrice.

62. Procédé pour la détection électrochimique d'événements d'hybridation d'oligomère, **caractérisé en ce qu'**au moins une surface conductrice modifiée suivant les revendications 37 à 57 est mise en contact avec des oligomères d'acide nucléique et **en ce que**, ensuite, a lieu la détection de la communication électrique entre la partie ayant une activité rédox et la surface conductrice.

63. Procédé suivant la revendication 62, **caractérisé en ce que** la détection a lieu par voltammétrie cyclique, ampérométrie ou mesure de conductivité.

64. Procédé suivant les revendications 62 ou 63, **caractérisé en ce que** la détection électrochimique est amorcée par séparation de charge photoinduite dans la partie ayant une activité rédox photoinductible fixée à la surface conductrice par un oligomère d'acide nucléique.

65. Procédé suivant la revendication 64, **caractérisé en ce que** l'irradiation par une lumière pour la séparation de charge photoinduite dans la partie ayant une activité rédox photoinductible fixée à la surface conductrice par un oligomère d'acide nucléique est limitée à une zone de la surface conductrice ayant au moins un type d'oligomère d'acide nucléique modifié.

66. Procédé suivant les revendications 64 ou 65, **caractérisé en ce que** la molécule électrodonneuse oxydée de la partie ayant une activité rédox photoinductible résultant d'une irradiation avec une lumière d'une longueur d'onde spécifique ou donnée quelconque est reréduite par une substance ayant une activité rédox libre convenable qui n'est pas liée mais est en contact avec l'oligomère d'acide nucléique, c'est-à-dire qu'elle est restaurée à l'état qu'elle avait à l'origine avant l'irradiation par la lumière.

67. Procédé suivant les revendications 64 ou 65, **caractérisé en ce que** la molécule électroaccepteuse réduite de la partie ayant une activité rédox photoinductible résultant d'une irradiation avec une lumière d'une longueur d'onde spécifique ou donnée quelconque est reoxydée par une substance ayant une activité rédox libre convenable qui n'est pas liée mais est en contact avec l'oligomère d'acide nucléique, c'est-à-dire qu'elle est restaurée à l'état qu'elle avait à l'origine avant l'irradiation par la lumière.

68. Procédé de détection électrochimique suivant les revendications 62 et 63, **caractérisé en ce que** la détection électrochimique est facilitée par une substance ayant une activité rédox libre qui effectue un transfert de charge thermique vers la partie ayant une activité rédox.

69. Procédé suivant les revendications 67 ou 68, **caractérisé en ce que** la molécule ayant une activité rédox libre qui n'est pas liée mais est en contact avec l'oligomère d'acide nucléique peut être oxydée et réduite de façon sélective à un potentiel Φ, où Φ satisfait à la condition
2,0 V≥Φ≥-2,0V
mesuré contre une électrode à hydrogène normale.

70. Procédé suivant les revendications 66 à 69, **caractérisé en ce que** la molécule ayant une activité rédox libre qui n'est pas liée mais est en contact avec l'oligomère d'acide nucléique est une quinone libre, un complexe d'hexacyanoferrate (II) libre, un ascorbate de sodium libre, un complexe de Ru(II)hexamine libre ou une protéine ayant une activité rédox libre.

71. Procédé suivant la revendication 70, **caractérisé en ce que** la substance ayant une activité rédox libre qui n'est pas liée mais est en contact avec l'oligomère d'acide nucléique est un cytochrome libre.
